(19) Europäisches Patentamt / European Patent Office / Office européen des brevets

(11) **EP 2 785 358 B1**

(12) **EUROPEAN PATENT SPECIFICATION**

(45) Date of publication and mention
of the grant of the patent:
**01.06.2022 Bulletin 2022/22**

(21) Application number: **12853488.0**

(22) Date of filing: **29.11.2012**

(51) International Patent Classification (IPC):
*A61K 33/24* $^{(2019.01)}$    *A61K 35/32* $^{(2015.01)}$
*A61K 38/17* $^{(2006.01)}$    *A61K 38/18* $^{(2006.01)}$
*A61K 38/20* $^{(2006.01)}$    *A61K 38/29* $^{(2006.01)}$
*A61K 33/30* $^{(2006.01)}$    *A61K 33/32* $^{(2006.01)}$
*A61L 27/54* $^{(2006.01)}$    *A61K 9/00* $^{(2006.01)}$
*A61K 45/06* $^{(2006.01)}$

(52) Cooperative Patent Classification (CPC):
(C-Sets available)
A61K 33/24; A61K 9/0024; A61K 33/04;
A61K 33/30; A61K 33/32; A61K 35/32;
A61K 38/179; A61K 38/1793; A61K 38/1808;
A61K 38/1825; A61K 38/1841; A61K 38/1858;
A61K 38/1866; A61K 38/1875; A61K 38/2026;
(Cont.)

(86) International application number:
**PCT/US2012/067087**

(87) International publication number:
**WO 2013/082295 (06.06.2013 Gazette 2013/23)**

(54) **INSULIN-MIMETICS AS THERAPEUTIC ADJUNCTS FOR BONE REGENERATION**

INSULINMIMETIKA ALS PHARMAZEUTISCHE ZUSÄTZE ZUR KNOCHENREGENERATION

AGENTS MIMÉTIQUES DE L'INSULINE EN TANT QUE TRAITEMENT D'APPOINT THÉRAPEUTIQUE POUR UNE RÉGÉNÉRATION OSSEUSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.11.2011 US 201161564822 P**
**09.12.2011 PCT/US2011/064240**
**25.10.2012 US 201261718646 P**

(43) Date of publication of application:
**08.10.2014 Bulletin 2014/41**

(73) Proprietor: **Rutgers, the State University of New Jersey**
**New Brunswick, NJ 08909 (US)**

(72) Inventors:
• **LIN, Sheldon, Suton**
**Chatham, NJ 07928 (US)**
• **PAGLIA, David, Naisby**
**Suffern, NY 10901 (US)**
• **O'CONNOR, James, Patrick**
**Fanwood, NY 07023 (US)**
• **BENEVENIA, Joseph**
**Montclair, NJ 07101 (US)**
• **WEY, Aaron**
**East Brunswick, NJ 08816 (US)**
• **SUBRAMANIAN, Sangeeta**
**Jacksonville, FL 32246 (US)**
• **CHIRICHELLA, Paul**
**Fair Lawn, NJ 07410 (US)**
• **VIVES, Michael, J.**
**Mendham, NJ 07945 (US)**
• **KOERNER, John D.**
**Pennsylvania 19102 (US)**

(74) Representative: **Zacco GmbH**
**Bayerstrasse 83**
**80335 München (DE)**

(56) References cited:
**WO-A1-2010/114827    WO-A1-2011/088318**

WO-A2-2005/032466    US-A- 5 716 415
US-A1- 2004 019 132    US-A1- 2006 093 646
US-A1- 2007 027 543    US-A1- 2009 068 285
US-A1- 2009 220 566    US-A1- 2009 226 534
US-A1- 2011 004 307

- **KAZUYUKI YUSA ET AL: "In vitro prominent bone regeneration by release zinc ion from Zn-modified implant", BIOCHEMICAL AND BIOPHYSICAL RESEARCH COMMUNICATIONS, vol. 412, no. 2, 28 July 2011 (2011-07-28) , pages 273-278, XP028273864, ISSN: 0006-291X, DOI: 10.1016/J.BBRC.2011.07.082 [retrieved on 2011-07-28]**
- **WANG X ET AL: "Zinc-containing apatite layers on external fixation rods promoting cell activity", ACTA BIOMATERIALIA, ELSEVIER, AMSTERDAM, NL, vol. 6, no. 3, 1 March 2010 (2010-03-01), pages 962-968, XP026879138, ISSN: 1742-7061 [retrieved on 2009-09-01]**
- **None**

Remarks:

The file contains technical information submitted after the application was filed and not included in this specification

(52) Cooperative Patent Classification (CPC): (Cont.)
**A61K 38/2066; A61K 38/2086; A61K 38/29; A61K 45/06; A61L 27/54; A61P 19/00; A61P 19/02; A61P 19/08; A61P 43/00;**
A61L 2430/38

C-Sets
**A61K 33/04;
A61K 33/24;
A61K 33/30;
A61K 33/32;
A61K 35/32;
A61K 38/179;
A61K 38/1793;
A61K 38/1808;
A61K 38/1825;
A61K 38/1841;
A61K 38/1858;
A61K 38/1866;
A61K 38/1875;
A61K 38/2026;
A61K 38/2066;
A61K 38/2086;
A61K 38/29**

**Description**

FIELD OF THE INVENTION

The present invention relates *inter alia* to a bone regeneration material for bone fusion or void filling consisting of an osteoconductive carrier and an insulin-mimetic zinc compound, and a bone injury treatment kit comprising a therapeutically effective amount of said bone regeneration material and an allograft bone tissue material and/or ceramic bone-graft substitute . The present invention is defined by the appended claims.

BACKGROUND OF THE INVENTION

About six million bone fractures, including about 600,000 non-union cases, occur annually in the United States, among which approximately 10% do not heal. Fracture healing is a complex process that involves the sequential recruitment of cells and the specific temporal expression of factors essential for bone repair. The fracture healing process begins with the initial formation of a blood clot at the fracture site. Platelets and inflammatory cells within the clot release several factors that are important for chemotaxis, proliferation, angiogenesis and differentiation of mesenchymal cells into osteoblasts or chondroblasts.

In the orthopedic procedures conducted, about one million performed annually require allograft or autograft. One solution to enhancement of bone healing is through tissue engineering, in which cells, such as osteoblast, fibroblast, chondroblasts, are treated with bioactive signaling molecules, e.g., insulin or insulin mimetics, with or without a carrier such as β-TCP ($CaPO_4$) and collagen under an appropriate environment. Current methods of treatment of bone fractures include (a) electro-stimulation devices (such as PEMF, Exogen) and (b) biologics, such as bone morphogenic proteins (BMPs), e.g., rhBMP-2/ACS (INFUSE® Bone Graft). The latter has been approved by FDA as an autograft replacement in spine fusion (ALIF) with specific interbody cages (2002), as an adjuvant for repair of tibia fractures with IM nail (2004), and for craniofacial maxillary surgery (2006), but this method is expensive, costing about $5,000 per application. (Lieberman, J.R., et al., J. Bone Joint Surg. Am., 2002, 84: 1032-1044; Trippel, S.B., et al., J. Bone Joint Surg. Am., 1996, 78: 1272-86.)

The fracture healing process subsequent to the initial hematoma formation can be classified as primary or secondary fracture healing. Primary fracture healing occurs in the presence of rigid internal fixation with little to no interfragmentary strain resulting in direct bone formation across the fracture gap. Secondary fracture healing occurs in response to interfragmentary strain due to an absence of fixation or non-rigid fixation resulting in bone formation through intramembranous and endochondral ossification characterized by responses from the periosteum and external soft tissue.

Intramembranous bone formation originates in the periosteum. Osteoblasts located within this area produce bone matrix and synthesize growth factors, which recruit additional cells to the site. Soon after the initiation of intramembranous ossification, the granulation tissue directly adjacent to the fracture site is replaced by cartilage leading to endochondral bone formation. The cartilage temporarily bridging the fracture gap is produced by differentiation of mesenchymal cells into chondrocytes. The cartilaginous callus begins with proliferative chondrocytes and eventually becomes dominated by hypertrophic chondrocytes. Hypertrophic chondrocytes initiate angiogenesis and the resulting vasculature provides a conduit for the recruitment of osteoblastic progenitors as well as chondroclasts and osteoclasts to resorb the calcified tissue. The osteoblastic progenitors differentiate into osteoblasts and produce woven bone, thereby forming a united fracture. The final stages of fracture healing are characterized by remodeling of woven bone to form a structure, which resembles the original tissue and has the mechanical integrity of unfractured bone.

However, the processes of bone metabolism are vastly different from bone repair. Bone metabolism is the interplay between bone formation and bone resorption. Bone repair, as described previously, is a complex process that involves the sequential recruitment and the differentiation of mesenchymal cells towards the appropriate osteoblastic/chondrogenic lineage to repair the fracture/defect site.

Spinal fusion is a common procedure performed for a variety of conditions including spondylosis, disk disorders, and spinal stenosis. The rates of pseudoarthrosis after single level spinal fusion have been reported up to 35%. The process of osteogenesis after spinal arthrodesis is similar to that which occurs during fracture healing and heterotopic ossification, and agents that increase the rate of fusion have an important role in decreasing pseudoarthrosis following spinal fusions. To our knowledge, prior to this invention, no *in vivo* evaluation of therapy on spinal fusion by local administration of an insulin-mimetic agent, such as a zinc or vanadium compound, has been performed.

There is a clear need to develop new methods for repairing bone fractures by enhancing bone regeneration as well as new methods to enhance spinal fusion.

SUMMARY OF THE INVENTION

The present invention provides a unique strategy for bone regeneration through local administration of insulin pathway-stimulating zinc compounds.

In one aspect the present invention provides a bone regeneration material for bone fusion or void filling, consisting of an osteoconductive carrier and an insulin-mimetic zinc agent, wherein said osteoconductive carrier is selected from the group consisting of autograft material, allograft material, and xenograft materials, and wherein the insulin-mimetic zinc compound is an inorganic zinc compound selected from the group consisting of zinc chloride, zinc sulfate, zinc phosphate, and zinc carbonate, or an organic acid zinc salt selected from the group consisting of zinc acetate, zinc formate, zinc propionate, zinc gluconate, bis(maltolato)zinc, zinc acexamate, zinc aspartate, bis(maltolato)zinc(II) [Zn(ma)2], bis(2-hydroxypyridine-N-oxido)zinc(II) [Zn(hpo)2], bis(allixinato)Zn(II) [Zn(alx)2], bis(6-methylpicolinato)Zn(II) [Zn(6mpa)2], bis(aspirinato)zinc(II), bis(pyrrole-2-carboxylato)zinc [Zn(pc)2], bis(alpha-furonic acidato)zinc [Zn(fa)2], bis(thiophene-2-carboxylato)zinc [Zn(tc)2], bis(thiophene-2-acetato)zinc [Zn(ta)2], (N-acetyl-L-cysteinato)Zn(II) [Zn(nac)], zinc(II)/poly(γ-glutamic acid) [Zn(γ-pga)], bis(pyrrolidine-N-dithiocarbamate)zinc(II) [Zn(pdc)2], zinc(II) L-lactate [Zn(lac)2], zinc(II) D-(2)-quinic acid [Zn(qui)2], bis(1,6-dimethyl-3-hydroxy-5-methoxy-2-pentyl-1,4-dihydropyridine-4-thionato)zinc(II) [Zn(tanm)2], and β-alanyl-L-histidinato zinc(II) (AHZ). In one embodiment, the bone regeneration material contains autograft bone tissue. In another embodiment, the bone regeneration material contains allograft bone tissue. In another embodiment, the bone regeneration material contains xenograft bone tissue.

In another aspect the present invention provides a bone injury treatment kit comprising a therapeutically effective amount of a bone regeneration material as defined above and an allograft bone tissue material and/or ceramic bone-graft substitute.

In another aspect the present invention provides a bone regeneration material for bone fusion or void filling an insulin-mimetic zinc compound and an osteoconductive carrier, wherein the osteoconductive carrier is calcium sulfate, and wherein the insulin-mimetic zinc compound is an inorganic zinc compound selected from the group consisting of zinc chloride, zinc sulfate, zinc phosphate, and zinc carbonate, or an organic acid zinc salt selected from the group consisting of zinc acetate, zinc formate, zinc propionate, zinc gluconate, bis(maltolato)zinc, zinc acexamate, zinc aspartate, bis(maltolato)zinc(II) [Zn(ma)2], bis(2-hydroxypyridine-N-oxido)zinc(II) [Zn(hpo)2], bis(allixinato)Zn(II) [Zn(alx)2], bis(6-methylpicolinato)Zn(II) [Zn(6mpa)2], bis(aspirinato)zinc(II), bis(pyrrole-2-carboxylato)zinc [Zn(pc)2], bis(alpha-furonic acidato)zinc [Zn(fa)2], bis(thiophene-2-carboxylato)zinc [Zn(tc)2], bis(thiophene-2-acetato)zinc [Zn(ta)2], (N-acetyl-L-cysteinato)Zn(II) [Zn(nac)], zinc(II)/poly(γ-glutamic acid) [Zn(γ-pga)], bis(pyrrolidine-N-dithiocarbamate)zinc(II) [Zn(pdc)2], zinc(II) L-lactate [Zn(lac)2], zinc(II) D-(2)-quinic acid [Zn(qui)2], bis(1,6-dimethyl-3-hydroxy-5-methoxy-2-pentyl-1,4-dihydropyridine-4-thionato)zinc(II) [Zn(tanm)2], and β-alanyl-L-histidinato zinc(II) (AHZ).

In another aspect the present invention provides an orthopedic or spinal implant, comprising at least one bone-contacting surface coated with a composition consisting of an insulin-mimetic zinc compound and an osteoconductive carrier selected from the group consisting of autograft materials, allograft materials, and xenograft materials, wherein the orthopedic implant is selected from the group consisting of screws, plates, rods, k-wires, pins, hooks, anchors, intramedullary devices, pedicle screws, pedicle hooks, spinal fusion cages, spinal fusion plates, prostheses, and said implant is made from a metal selected from the group consisting of titanium, alloys thereof, tantalum, alloys thereof, cobalt chrome alloys, steel alloys, and combinations thereof or from a polymeric material comprising a polymer selected from polyglycolic acid (PGA), poly(lactic-co-glycolic acid) (PLGA), polylactic acid (PLA), polycaprolactone (PCL), polyether ether ketone (PEEK), polyethylene terephthalate (PET), polypropylene (PP), polycarbonates (PC), poly(ortho esters) (POEs), and combinations thereof, and wherein the insulin-mimetic zinc compound is an inorganic zinc compound selected from the group consisting of zinc chloride, zinc sulfate, zinc phosphate, and zinc carbonate, or an organic acid zinc salt selected from the group consisting of zinc acetate, zinc formate, zinc propionate, zinc gluconate, bis(maltolato)zinc, zinc acexamate, zinc aspartate, bis(maltolato)zinc(II) [Zn(ma)2], bis(2-hydroxypyridine-N-oxido)zinc(II) [Zn(hpo)2], bis(allixinato)Zn(II) [Zn(alx)2], bis(6-methylpicolinato)Zn(II) [Zn(6mpa)2], bis(aspirinato)zinc(II), bis(pyrrole-2-carboxylato)zinc [Zn(pc)2], bis(alpha-furonic acidato)zinc [Zn(fa)2], bis(thiophene-2-carboxylato)zinc [Zn(tc)2], bis(thiophene-2-acetato)zinc [Zn(ta)2], (N-acetyl-L-cysteinato)Zn(II) [Zn(nac)], zinc(II)/poly(γ-glutamic acid) [Zn(γ-pga)], bis(pyrrolidine-N-dithiocarbamate)zinc(II) [Zn(pdc)2], zinc(II) L-lactate [Zn(lac)2], zinc(II) D-(2)-quinic acid [Zn(qui)2], bis(1,6-dimethyl-3-hydroxy-5-methoxy-2-pentyl-1,4-dihydropyridine-4-thionato)zinc(II) [Zn(tanm)2], and β-alanyl-L-histidinato zinc(II) (AHZ).

In another aspect, the present invention provides a bone tissue kit for use in a spinal fusion surgical procedure to facilitate fusion of vertebrae, comprising a composition formulated for facile application in said spinal fusion procedure consisting of an insulin-mimetic zinc compound, an allograft bone tissue material and/or ceramic bone-graft substitute and a pharmaceutically acceptable carrier, and wherein the insulin-mimetic zinc compound is an inorganic zinc compound selected from the group consisting of zinc chloride, zinc sulfate, zinc phosphate, and zinc carbonate, or an organic acid zinc salt selected from the group consisting of zinc acetate, zinc formate, zinc propionate, zinc gluconate, bis(maltolato)zinc, zinc acexamate, zinc aspartate, bis(maltolato)zinc(II) [Zn(ma)2], bis(2-hydroxypyridine-N-oxido)zinc(II) [Zn(hpo)2], bis(allixinato)Zn(II) [Zn(alx)2], bis(6-methylpicolinato)Zn(II) [Zn(6mpa)2], bis(aspirinato)zinc(II), bis(pyrrole-2-carboxylato)zinc [Zn(pc)2], bis(alpha-furonic acidato)zinc [Zn(fa)2], bis(thiophene-2-carboxylato)zinc [Zn(tc)2], bis(thiophene-2-acetato)zinc [Zn(ta)2], (N-acetyl-L-cysteinato)Zn(II) [Zn(nac)], zinc(II)/poly(γ-glutamic acid) [Zn(γ-pga)],

bis(pyrrolidine-N-dithiocarbamate)zinc(II) [Zn(pdc)2], zinc(II) L-lactate [Zn(lac)2], zinc(II) D-(2)-quinic acid [Zn(qui)2], bis(1,6-dimethyl-3-hydroxy-5-methoxy-2-pentyl-1,4-dihydropyridine-4-thionato)zinc(II) [Zn(tanm)2], and β-alanyl-L-histidinato zinc(II) (AHZ). In an embodiment the kit also contains allograft bone tissue material. In one embodiment the insulin-mimetic zinc compound and the allograft bone tissue material are provided in a mixture. In another embodiment, the insulin-mimetic zinc compound and allograft bone tissue material are provided for subsequent mixing. In another aspect the present invention provides a composition for use in a spinal fusion surgical procedure consisting of an insulin-mimetic zinc compound and an osteoconductive carrier selected from the group consisting of autograft materials, allograft materials, and xenograft materials, wherein said composition enhances spinal fusion, and wherein the insulin-mimetic zinc compound is an inorganic zinc compound selected from the group consisting of zinc chloride, zinc sulfate, zinc phosphate, and zinc carbonate, or an organic acid zinc salt selected from the group consisting of zinc acetate, zinc formate, zinc propionate, zinc gluconate, bis(maltolato)zinc, zinc acexamate, zinc aspartate, bis(maltolato)zinc(II) [Zn(ma)2], bis(2-hydroxypyridine-N-oxido)zinc(II) [Zn(hpo)2], bis(allixinato)Zn(II) [Zn(alx)2], bis(6-methylpicolinato)Zn(II) [Zn(6mpa)2], bis(aspirinato)zinc(II), bis(pyrrole-2-carboxylato)zinc [Zn(pc)2], bis(alpha-furonic acidato)zinc [Zn(fa)2], bis(thiophene-2-carboxylato)zinc [Zn(tc)2], bis(thiophene-2-acetato)zinc [Zn(ta)2], (N-acetyl-L-cysteinato)Zn(II) [Zn(nac)], zinc(II)/poly(y-glutamic acid) [Zn(y-pga)], bis(pyrrolidine-N-dithiocarbamate)zinc(II) [Zn(pdc)2], zinc(II) L-lactate [Zn(lac)2], zinc(II) D-(2)-quinic acid [Zn(qui)2], bis(1,6-dimethyl-3-hydroxy-5-methoxy-2-pentyl-1,4-dihydropyridine-4-thionato)zinc(II) [Zn(tanm)2], and β-alanyl-L-histidinato zinc(II) (AHZ). In one embodiment, the composition contains allograft bone material

In another aspect, the present invention provides an implantable device for enhancing spinal fusion, comprising a prosthetic implant configured to stabilize and promote the fusion of two adjacent vertebrae, wherein the bone tissue contacting surfaces of the prosthetic implant are coated with a composition consisting of an insulin-mimetic zinc compound and a pharmaceutically acceptable carrier, and wherein the insulin-mimetic zinc compound is an inorganic zinc compound selected from the group consisting of zinc chloride, zinc sulfate, zinc phosphate, and zinc carbonate, or an organic acid zinc salt selected from the group consisting of zinc acetate, zinc formate, zinc propionate, zinc gluconate, bis(maltolato)zinc, zinc acexamate, zinc aspartate, bis(maltolato)zinc(II) [Zn(ma)2], bis(2-hydroxypyridine-N-oxido)zinc(II) [Zn(hpo)2], bis(allixinato)Zn(II) [Zn(alx)2], bis(6-methylpicolinato)Zn(II) [Zn(6mpa)2], bis(aspirinato)zinc(II), bis(pyrrole-2-carboxylato)zinc [Zn(pc)2], bis(alpha-furonic acidato)zinc [Zn(fa)2], bis(thiophene-2-carboxylato)zinc [Zn(tc)2], bis(thiophene-2-acetato)zinc [Zn(ta)2], (N-acetyl-L-cysteinato)Zn(II) [Zn(nac)], zinc(II)/poly(y-glutamic acid) [Zn(y-pga)], bis(pyrrolidine-N-dithiocarbamate)zinc(II) [Zn(pdc)2], zinc(II) L-lactate [Zn(lac)2], zinc(II) D-(2)-quinic acid [Zn(qui)2], bis(1,6-dimethyl-3-hydroxy-5-methoxy-2-pentyl-1,4-dihydropyridine-4-thionato)zinc(II) [Zn(tanm)2], and β-alanyl-L-histidinato zinc(II) (AHZ).

## BRIEF DESCRIPTION OF THE DRAWINGS

Figure 1 depicts post-operative X-rays. Representative x-rays taken immediately post-operative: (A) Einhorn model, (B) model used in this work. (Note in (B) the Kirschner wire is going through the trochanter, which helps to stabilize the fracture site and prevent the migration of the Kirschner wire.)

Figure 2 depicts Mechanical Testing Setup: Intact femur before embedded in ¾ inch square nut with Field's Metal, where (A) ZINC 10 (3.0 mg/kg ZnCl2) and (B) ZINC 8 (1.0 mg/kg $ZnCl_2$) represent two sets of Zinc treated femurs harvested 4 weeks post-surgery, showing spiral fracture indicative of healing, compared to (C) ZINC 3 (control) showing non-spiral fracture indicative of non-union (Left: Intact Femur, Right: Fractured Femur).

Figure 3 illustrates 4-week radiographs (AP and Medial-Lateral views) of representative samples of fracture femur bones treated with local $ZnCl_2$ (1.0 and 3.0 mg/Kg) in comparison with saline control.

Figure 4 illustrates histomorphometry of $ZnCl_2$ treated fractures in comparison with saline control.

Figure 5 illustrates 4-week radiographs (AP and Medial-Lateral views) of representative sample for each group of fractured femur bones treated with 1.0 mg/Kg ZnCh + $CaSO_4$ carrier in comparison with $CaSO_4$ control.

Figure 6 illustrates comparison of use of $ZnCl_2$ with the existing therapy (BMP2): (1) a single intramedullary dose (1 mg/kg) of $ZnCl_2$ with the calcium sulfate ($CaSO_4$) vehicle (purple); (2) a single intramedullary dose (3 mg/kg) of $ZnCl_2$ without a vehicle (green); (3) BMP-2 study used a single percutaneous dose of BMP-2 (80 μg) with buffer vehicle (red); and (4) Exogen study used daily exposure periods of ultrasound treatment (20 min/day). The average value (duration of 25 days) is shown in blue.

Figure 7 illustrates 4-week post-fracture radiographs of local manganese chloride ($MnCl_2$) treatment group vs. saline control.

Figure 8 illustrates quantification of local VAC levels. Femur bone vanadium concentrations (μg vanadium/gram of bone mass) at one, four, seven, and fourteen days after surgery for fractured and contralateral (intact) femora.

Figure 9 illustrates histological comparison between VAC and saline control treated rats: Representative sections of saline control, 1.5 mg/kg VAC, and 3 mg/kg VAC groups show progression of healing from 10-21 days at 1.67 X as visualized under stereomicroscope.

Figure 10 illustrates 4-week radiographs of three representative samples for each group of fractured femur bones: (A) saline control, (B) 1.5 mg/kg VAC, (C) 3.0 mg/kg VAC.

Figure 11 illustrates 4-week mechanical testing of treatment with VAC with or without sterilization (normalized to intact femora). The data represents average values $\pm$ standard deviation. * Represent values statistically higher than control, $p < 0.05$ versus saline control.

Figure 12 illustrates the effect of local vanadium therapy on long-term healing of femur fractures in normal (non-diabetic) rats, measured by radiographic analysis.

Figure 13 illustrates comparison of local VAC treatment with current BMP2 and Exogen therapies.

Figure 14 illustrates that the transverse processes of L4-L5 were cleaned of soft tissue, and decorticated with a high-speed burr.

Figure 15 illustrates that the crushed autograft was then spread over and between the transverse processes at the appropriate level (L4-L5). An equivalent amount of implant or blank was incorporated into the autograft bed.

Figure 16 illustrates radiographs of the vanadium-treated spines in the rat model in comparison with those in the control group.

DETAILED DESCRIPTION OF THE INVENTION

The present invention is based on the discovery that insulin-mimetics can be used to accelerate bone regeneration by stimulating insulin pathway signaling at a fracture site. In particular, the present invention is based on the discovery that the biological impact of insulin-mimetic zinc compounds as defined in the claims on bone can be exploited. to play a critical role in bone healing. Insulin pathway-stimulating zinc compounds delivered locally with or without a carrier, can improve the torsional strength and bone mineral density of regenerated bone. Development of a zinc salt therapy to accelerate bone regeneration would be beneficial therapeutically and obviate the need for developing specialized methods to deliver complex molecules, such as protein growth factors like insulin, eliminate specialized storage, enable ease of use, and be cost-effective.

The present invention thus uses an insulin-mimetic zinc compound as defined in the claims to treat various bone conditions, such as bone fractures, and to enhance spinal fusion, for example, in treating spinal arthrodesis. For example, we used $ZnCl_2$ alone or as part of a formulation with an orthopedic carrier ($CaSO_4$, for example) and showed accelerated fracture healing when applied directly to the site of fracture post surgery.

Preferably, the patient in need of bone healing is afflicted with a bone condition selected from bone fracture, bone trauma, arthrodesis, including spinal arthrodesis, extremity arthrodesis and the like, and a bone deficit condition associated with post-traumatic bone surgery, post-prosthetic joint surgery, post-plastic bone surgery, post-dental surgery, bone chemotherapy treatment, congenital bone defect, post traumatic bone loss, post surgical bone loss, post infectious bone loss, allograft incorporation or bone radiotherapy treatment.

In another embodiment of this aspect, the bone condition is selected from bone fractures, osseous defects, and delayed unions and non-unions.

Thus, in one aspect, the present invention provides a bone regeneration material for bone fusion or void filling consisting of an osteoconductive carrier and an insulin-mimetic zinc compound, wherein said osteoconductive carrier is selected from the group consisting of autograft material, allograft material, and xenograft materials, and wherein the insulin-mimetic zinc compound is an inorganic zinc compound selected from the group consisting of zinc chloride, zinc sulfate, zinc phosphate, and zinc carbonate, or an organic acid zinc salt selected from the group consisting of zinc acetate, zinc formate, zinc propionate, zinc gluconate, bis(maltolato)zinc, zinc acexamate, zinc aspartate, bis(maltolato)zinc(II) [Zn(ma)2], bis(2-hydroxypyridine-N-oxido)zinc(II) [Zn(hpo)2], bis(allixinato)Zn(II) [Zn(alx)2], bis(6-methylpicolinato)Zn(II) [Zn(6mpa)2], bis(aspirinato)zinc(II), bis(pyrrole-2-carboxylato)zinc [Zn(pc)2], bis(alpha-furonic acidato)zinc [Zn(fa)2], bis(thiophene-2-carboxylato)zinc [Zn(tc)2], bis(thiophene-2-acetato)zinc [Zn(ta)2], (N-acetyl-L-cysteinato)Zn(II) [Zn(nac)], zinc(II)/poly(y-glutamic acid) [Zn(y-pga)], bis(pyrrolidine-N-dithiocarbamate)zinc(II) [Zn(pdc)2], zinc(II) L-lactate [Zn(lac)2], zinc(II) D-(2)-quinic acid [Zn(qui)2], bis(1,6-dimethyl-3-hydroxy-5-methoxy-2-pentyl-1,4-dihydropyridine-4-thionato)zinc(II) [Zn(tanm)2], and β-alanyl-L-histidinato zinc(II) (AHZ).

In another aspect, the present invention provides orthopedic and spinal implants, comprising at least one bone-contacting surface coated with a composition consisting of an insulin-mimetic zinc compound and an osteoconductive carrier selected from the group consisting of autograft materials, allograft materials, and xenograft materials, wherein the orthopedic implant is selected from the group consisting of screws, plates, rods, k-wires, pins, hooks, anchors, intramedullary devices, pedicle screws, pedicle hooks, spinal fusion cages, spinal fusion plates, prostheses, and said implant is made from a metal selected from the group consisting of titanium, alloys thereof, tantalum, alloys thereof, cobalt chrome alloys, steel alloys, and combinations thereof or from a polymeric material comprising a polymer selected from polyglycolic acid (PGA), poly(lactic-co-glycolic acid) (PLGA), polylactic acid (PLA), polycaprolactone (PCL), polyether ether ketone (PEEK), polyethylene terephthalate (PET), polypropylene (PP), polycarbonates (PC), poly(ortho esters) (POEs), and combinations thereof, and wherein the insulin-mimetic zinc compound is an inorganic zinc compound selected from the group consisting of zinc chloride, zinc sulfate, zinc phosphate, and zinc carbonate, or an organic acid zinc salt selected from the group consisting of zinc acetate, zinc formate, zinc propionate, zinc gluconate, bis(maltolato)zinc, zinc acexamate, zinc aspartate, bis(maltolato)zinc(II) [Zn(ma)2], bis(2-hydroxypyridine-N-oxido)zinc(II) [Zn(hpo)2], bis(allixinato)Zn(II) [Zn(alx)2], bis(6-methylpicolinato)Zn(II) [Zn(6mpa)2], bis(aspirinato)zinc(II), bis(pyrrole-2-carboxylato)zinc [Zn(pc)2], bis(alpha-furonic acidato)zinc [Zn(fa)2], bis(thiophene-2-carboxylato)zinc [Zn(tc)2], bis(thiophene-2-acetato)zinc [Zn(ta)2], (N-acetyl-L-cysteinato)Zn(II) [Zn(nac)], zinc(II)/poly(y-glutamic acid) [Zn(y-pga)], bis(pyrrolidine-N-dithiocarbamate)zinc(II) [Zn(pdc)2], zinc(II) L-lactate [Zn(lac)2], zinc(II) D-(2)-quinic acid [Zn(qui)2], bis(1,6-dimethyl-3-hydroxy-5-methoxy-2-pentyl-1,4-dihydropyridine-4-thionato)zinc(II) [Zn(tanm)2], and β-alanyl-L-histidinato zinc(II) (AHZ).

The insulin mimetic may be coated on the bone-contacting surface of the implant by conventional means. In the alternative the implant may be formulated and fabricated so that the insulin-mimetic is incorporated into the bone-contacting surface of the implant. Means by which this can be accomplished are readily apparent to those of ordinary skill in the art.

In another aspect, the present invention provides a bone injury treatment kit comprising a therapeutically effective amount of a bone regeneration material of the present invention and an allograft bone tissue material and/or ceramic bone-graft substitute. Such kits may also include a device for local administration, such as a hypodermic syringe.

In another aspect, the present invention provides composition for use in a spinal fusion procedure consisting of an insulin-mimetic zinc compound and an osteoconductive carrier selected from the group consisting of autograft materials, allograft materials, and xenograft materials, wherein said composition enhances spinal fusion, and wherein the insulin-mimetic zinc compound is an inorganic zinc compound selected from the group consisting of zinc chloride, zinc sulfate, zinc phosphate, and zinc carbonate, or an organic acid zinc salt selected from the group consisting of zinc acetate, zinc formate, zinc propionate, zinc gluconate, bis(maltolato)zinc, zinc acexamate, zinc aspartate, bis(maltolato)zinc(II) [Zn(ma)2], bis(2-hydroxypyridine-N-oxido)zinc(II) [Zn(hpo)2], bis(allixinato)Zn(II) [Zn(alx)2], bis(6-methylpicolinato)Zn(II) [Zn(6mpa)2], bis(aspirinato)zinc(II), bis(pyrrole-2-carboxylato)zinc [Zn(pc)2], bis(alpha-furonic acidato)zinc [Zn(fa)2], bis(thiophene-2-carboxylato)zinc [Zn(tc)2], bis(thiophene-2-acetato)zinc [Zn(ta)2], (N-acetyl-L-cysteinato)Zn(II) [Zn(nac)], zinc(II)/poly(y-glutamic acid) [Zn(y-pga)], bis(pyrrolidine-N-dithiocarbamate)zinc(II) [Zn(pdc)2], zinc(II) L-lactate [Zn(lac)2], zinc(II) D-(2)-quinic acid [Zn(qui)2], bis(1,6-dimethyl-3-hydroxy-5-methoxy-2-pentyl-1,4-dihydropyridine-4-thionato)zinc(II) [Zn(tanm)2], and β-alanyl-L-histidinato zinc(II) (AHZ). The insulin-mimetic zinc compounds suitable for the present invention include inorganic zinc compounds selected from the group consisting of zinc chloride, zinc sulfate, zinc phosphate, and zinc carbonate.

The insulin-mimetic zinc compounds can also be zinc salts of organic acids selected from the group consisting of zinc acetate, zinc formate, zinc propionate, zinc gluconate, bis(maltolato)zinc, zinc acexamate, zinc aspartate, bis(maltolato)zinc(II) [Zn(ma)2], bis(2-hydroxypyridine- N-oxido)zinc(II) [Zn(hpo)2], bis(allixinato)Zn(II) [Zn(alx)2], bis(6-methylpicolinato)Zn(II) [Zn(6mpa)2], bis(aspirinato)zinc(II), bis(pyrrole-2-carboxylato)zinc [Zn(pc)2], bis(alpha-furonic acidato)zinc [Zn(fa)2], bis(thiophene-2-carboxylato)zinc [Zn(tc)2], bis(thiophene-2-acetato)zinc [Zn(ta)2], (N-acetyl-L-cysteinato)Zn(II) [Zn(nac)], zinc(II)/poly(y-glutamic acid) [Zn(y-pga)], bis(pyrrolidine-N-dithiocarbamate)zinc(II) [Zn(pdc)$_2$], zinc(II) L-lactate [Zn(lac)$_2$], zinc(II) D-(2)-quinic acid [Zn(qui)$_2$], bis(1,6-dimethyl-3-hydroxy-5-methoxy-2-pentyl-1,4-dihydropyridine-4-thionato)zinc(II) [Zn(tanm)2], and β-alanyl-L-histidinato zinc(II) (AHZ In another embodiment, the organic acid of zinc salt is a naturally occurring fatty acid.

Advantages of small molecules (such as zinc)insulin-mimetic agents include, but are not limited to: (a) development of a small molecule insulin mimetic can be of great significance to bone fracture patients; (b) insulin composite which requires a carrier may be difficult to meet FDA requirements as a dual agent product; and (c) small molecule insulin mimetics may have longer half life and avoid the storage issues commonly seen with proteins.

Exemplary healing mechanisms include, but are not limited to: (a) retaining mineralized components in bone, (b) inhibiting release of mineralized components from bone, (c) stimulating osteoblast activity, (d) reducing osteoclast activity, or (e) stimulating bone remodeling.

The term "therapeutically effective amount," as used herein, means an amount at which the administration of an agent is physiologically significant. The administration of an agent is physiologically significant if its presence results in a detectable change in the bone healing process of the patient.

The term "bone injury," "injured bone," or the like, as used herein, refers to a bone condition selected from the group consisting of bone fracture, bone trauma, arthrodesis, and a bone deficit condition associated with post-traumatic bone surgery, post-prosthetic joint surgery, post-plastic bone surgery, post-dental surgery, bone chemotherapy treatment, congenital bone loss, post traumatic bone loss, post surgical bone loss, post infectious bone loss, allograft incorporation or bone radiotherapy treatment.

Insulin-mimetic compositions within the meaning of the present invention are particularly useful adjuncts for spinal fusion procedures. The compositions may be used to promote vertebral fusion and spinal stablization and also to improve function of spinal stabilization devices.

In another aspect, the present invention provides a bone tissue kit for use in a spinal fusion surgical procedure to facilitate fusion of vertebrae, including a composition formulated for facile application in said spinal fusion procedure consisting of an insulin-mimetic zinc compound, an allograft bone tissue material and/or ceramic bone-graft substitute and a pharmaceutically acceptable carrier; wherein the insulin-mimetic zinc compound is an inorganic zinc compound selected from the group consisting of zinc chloride, zinc sulfate, zinc phosphate, and zinc carbonate, or an organic acid zinc salt selected from the group consisting of zinc acetate, zinc formate, zinc propionate, zinc gluconate, bis(maltolato)zinc, zinc acexamate, zinc aspartate, bis(maltolato)zinc(II) [Zn(ma)2], bis(2-hydroxypyridine-N-oxido)zinc(II) [Zn(hpo)2], bis(allixinato)Zn(II) [Zn(alx)2], bis(6-methylpicolinato)Zn(II) [Zn(6mpa)2], bis(aspirinato)zinc(II), bis(pyrrole-2-carboxylato)zinc [Zn(pc)2], bis(alpha-furonic acidato)zinc [Zn(fa)2], bis(thiophene-2-carboxylato)zinc [Zn(tc)2], bis(thiophene-2-acetato)zinc [Zn(ta)2], (N-acetyl-L-cysteinato)Zn(II) [Zn(nac)], zinc(II)/poly(y-glutamic acid) [Zn(y-pga)], bis(pyrrolidine-N-dithiocarbamate)zinc(II) [Zn(pdc)2], zinc(II) L-lactate [Zn(lac)2], zinc(II) D-(2)-quinic acid [Zn(qui)2], bis(1,6-dimethyl-3-hydroxy-5-methoxy-2-pentyl-1,4-dihydropyridine-4-thionato)zinc(II) [Zn(tanm)2], and β-alanyl-L-histidinato zinc(II) (AHZ). In one embodiment the insulin-mimetic zinc compound and the allograft bone tissue material, xenograft bone tissue material, and/or ceramic bone-graft substitute are provided in a mixture. In another embodiment, the insulin-mimetic zinc compound and allograft bone tissue material, xenograft bone tissue material, or ceramic bone-graft substitute are provided for subsequent mixing.

Examples of diseases or conditions that make a patient in need of spinal fusion include, but are not limited to, arthrodesis, degenerative disc disease, spinal disc herniation, discogenic pain, spinal tumor, vertebral fracture, scoliosis, kyphosis (i.e., Scheuermann's disease), spondylolisthesis, spondylosis, Posterior Rami Syndrome, other degenerative spinal conditions, and any other conditions that cause instability of the spine.

It will be appreciated that actual preferred amounts of a pharmaceutical composition used in a given therapy will vary depending upon the particular form being utilized, the particular compositions formulated, the mode of application, and the particular site of administration, and other such factors that are recognized by those skilled in the art including the attendant physician or veterinarian. Optimal administration rates for a given protocol of administration can be readily determined by those skilled in the art using conventional dosage determination tests.

Dosages of an insulin-mimetic suitable for the present invention may vary depending on the particular use envisioned. The determination of the appropriate dosage or route of administration is well within the skill of an ordinary physician. The dosage regimen for the insulin-mimetic agents of the present invention will vary depending upon known factors, such as the pharmacodynamic characteristics of the particular agent, and its mode and route of administration; the species, age, sex, health, medical condition, and weight of the recipient; the nature and extent of the symptoms; etc. For exmple, the local dosage of a particular zinc compound may depend more on the bone condition than on the weight of a patient. A dosage of local administration may significantly differ from a dosage of systemic administration, and a dosage of administration in a solution form may differ from a dosage when it is administered through the surface coating on an implantable device. Without being bound by any particular theory, the dosage of an insulin-mimetic agent according to the present invention should be at a level so that the insulin pathway in a patient is stimulated in order to accelerate the bone healing or regeneration process.

By way of general guidance, the dosage of each active ingredient, when used for the indicated effects, will range between about 0.001 to about 200 mg/Kg based on a patient's weight, preferably between about 0.01 to about 100 mg/Kg, and most preferably between about 0.1 to about 50 mg/Kg. The doses can be repeated whenever needed, or considered to be beneficial to the bone healing and regeneration processes as determined by a physician, for example, once daily, once weekly, once every other week, once monthly, or any other time period that may provide most benefits to a particular patient.

The route of administration of "local zinc" via "insulin mimetic delivery system" is in accordance with known methods, e.g. via immediate-release, controlled-release, sustained-release, and extended-release means. Preferred modes of administration for the insulin-mimetic delivery system include injection directly into afflicted bone or a fusion site and areas adjacent and/or contiguous to these sites, or surgical implantation of insulin-mimetic agent(s) directly into the fusion sites and area adjacent and/or contiguous to these sites. This type of system will allow temporal control of release as well as location of release as stated above.

The formulations used herein may also contain more than one active compound as necessary for the particular indication being treated, preferably those with complementary activities that do not adversely affect each other. Alternatively, or in addition, the formulation may comprise a cytotoxic agent, cytokine or growth inhibitory agent. Such molecules are present in combinations and amounts that are effective for the intended purpose.

As an illustrated example, an insulin-mimetic may be continuously administered locally to a site via a delivery pump. In one embodiment, the pump is worn externally (in a pocket or on the belt) and attached to the body with a long, thin, and flexible plastic tubing that has a needle or soft cannula (thin plastic tube), and the cannula or needle is inserted and then left in place beneath the skin. The needle or cannula and tubing can be changed, for example, every 48 to 72 hours. The pump would store the insulin-mimetic in a cartridge and release it based on the optimal delivery rate. Optionally, the pump is programmed to give a small dose of a drug continuously through the day and night, which in certain circumstances may be preferred.

Various applications of the present invention are listed in Table 1.

**Table 1.** Applications of local administration of insulin-mimetics.

| Application | Method | Examples |
| --- | --- | --- |
| Fracture Healing | Local delivery to closed or open fractures | Bone fractures treated by closed reduction or surgical reduction |
| Fracture Non-unions | Local delivery to non-healing fractures | Treatment of fractures that have experienced delayed or failed healing |
| Arthrodesis/fusion (e.g., spine, fusion of joints such as foot and ankles, wrist) | Injection to increase osteogenesis in certain joints | Treatment of spine fusion |
| Allograft incorporation | Injection within and around allograft used to fill defect | Treatment of intercalary defect after traumatic injuries, tumor resection, failed arthroplasty, etc. |

The compounds and compositions for use according to the present invention are effective insulin mimetics without the issues associated with biologics such as insulin. They have various notable advantages over biologics, for example, their high tolerance to manufacturing process and conditions (e.g., elevated temperatures). In the case of $ZnCl_2$, it is a known, highly stable compound commonly used in medical products, has a long shelf life, and has no storage and contamination/sterilization related issues.

The compounds and compositions for use according to the present invention are also versatile - they can be used directly or as part of a formulation with a carrier applied to the site of fracture to accelerate fracture healing. No special techniques need to be developed in order to use the inventions described. For example, zinc compounds can be applied directly to the fracture site as part of the surgery or intramedullary.

When an implantable device coated by a composite surface coating comprising an insulin-mimetic zinc compound in accordance with the present invention is used, the coating can be formed by any methods known in the relevant art, for example, without limitation, those disclosed in Petrova, R. and Suwattananont, N., J. Electr. Mat., 34(5):8 (2005)). For example, suitable methods include chemical vapor deposition (CVD), physical vapor deposition (PVD), thermochemical treatment, oxidation, and plasma spraying (Fischer, R.C., Met. Progr. (1986); Habig, K.H., Tribol. Int., 22:65 (1989)). A suitable coating of the present invention may also comprise combinations of multiple, preferably two or three, layers obtained by forming first boron diffusion coating followed by CVD (Z. Zakhariev, Z., et al., Surf. Coating Technol., 31:265 (1987)). Thermochemical treatment techniques have been well investigated and used widely in the industry. This is a method by which nonmetals or metals are penetrated by thermodiffusion followed by chemical reaction into the surface. By thermochemical treatment, the surface layer changes its composition, structure, and properties.

Other suitable coating techniques may include, but are not limited to, carburizing, nitriding, carbonitriding, chromizing, and aluminizing. Among these coating techniques, boronizing, being a thermochemical process, is used to produce hard and wear-resistant surfaces. The present invention also finds wide application in veterinary medicines to treat a variety of factures or enhance spinal fusion in a mammalian animal, including but not limited to, horses, dogs, cats, or any other domestic or wild mammalian animals. A particular useful application may be found, for example, in treating an injured racehorse.

The following non-limiting examples illustrate certain aspects of the invention.

EXAMPLES

EXAMPLE 1

**Use of Zinc Compounds to Accelerate Bone Fracture Healing**

MATERIALS AND METHODS

**The BB Wistar Rat Model**

*Animal Source and Origin*

Diabetic Resistance (DR) BB Wistar rats used in the study were obtained from a breeding colony at UMDNJ-New Jersey Medical School (NJMS). The rats were housed under controlled environmental conditions and fed *ad libitum.* All research protocols were approved by the Institutional Animal Care and Use Committee at University of Medicine and Dentistry of New Jersey- New Jersey Medical School.

*Diabetic Resistant BB Wistar Rats*

A total of 24 DR BB Wistar rats were utilized in the study. Due to unstable fixation during mechanical testing, three samples were removed. Another sample was removed due to complications associated with a post-operative infection. The remaining 17 animals were used for mechanical testing and were distributed between the control saline (n=6), 0.1 mg/kg zinc chloride (n=2), 1.0 mg/kg zinc chloride (n=3), 3.0 mg/kg zinc chloride (n=3), 6.0 mg/kg zinc chloride (n=4) and 10.0 mg/kg zinc chloride (n=3) groups.

*Closed Femoral Fracture Model*

Surgery was performed in DR animals between ages 93 and 99 days using a closed mid-diaphyseal fracture model, on the right femur as described previously.

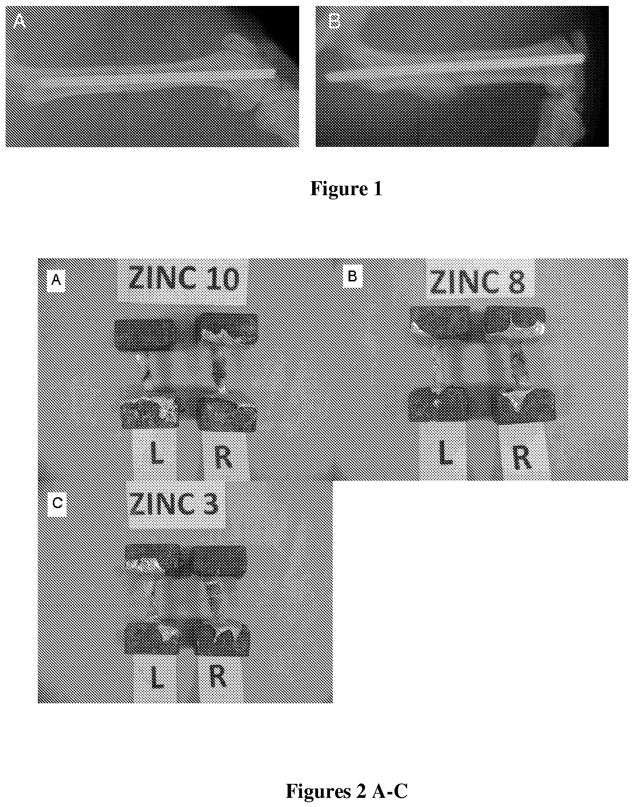

General anesthesia was administrated by intraperitoneal (IP) injection of ketamine (60 mg/kg) and xylazine (8 mg/kg). The right leg of each rat was shaved and the incision site was cleansed with Betadine and 70% alcohol. An approximately 1 cm medial, parapatellar skin incision was made over the patella. The patella was dislocated laterally and the interchondylar notch of the distal femur was exposed. An entry hole was made with an 18 gauge needle and the femur was reamed with the 18 gauge needle. A Kirschner wire (316LVM stainless steel, 0.04 inch diameter, Small Parts, Inc., Miami Lakes, FL) was inserted the length of the medullary canal, and drilled through the trochanter of the femur. The kirschner wire was cut flush with the femoral condyles. After irrigation, the wound was closed with 4-0 vicryl resorbable suture. A closed midshaft fracture was then created unilaterally with the use of a three-point bending fracture machine. X-rays were taken to determine whether the fracture was of acceptable configuration. An appropriate fracture is an approximately mid-diaphyseal, low energy, transverse fracture (Figure 1). The rats were allowed to ambulate freely immediately post-fracture. This closed fracture model is commonly used to evaluate the efficacy of osseous wound healing devices and drugs.

*Local Zinc Delivery*

Zinc Chloride [(ZnCl$_2$), Sigma Aldrich, St. Louis, MO] mixed with a buffer was injected into the intramedullary canal prior to fracture. The buffer consisted of sodium acetate, sodium chloride methyl hydroxybenzoate, and zinc chloride. Doses of 1.0 mg/kg and 3.0 mg/kg zinc chloride were tested and administered at a volume of 0.1mL.

*Mechanical Testing*

Fractured and contralateral femora were resected at three and four weeks post-fracture. Femora were cleaned of soft tissue and the intramedullary rod was removed. Samples were wrapped in saline (0.9% NaCl) soaked gauze and stored at -20 °C. Prior to testing, all femora were removed from the freezer and allowed to thaw to room temperature for three to four hours. The proximal and distal ends of the fractured and contralateral femora were embedded in ¾ inch square nuts with Field's Metal, leaving an approximate gauge length of 18 mm (Figure 2). After measuring callus, gauge length and femur dimensions, torsional testing was conducted using a servohydraulics machine (MTS Systems Corp., Eden Prairie, MN) with a 20 Nmm reaction torque cell (Interface, Scottsdale, AZ) and tested to failure at a rate of 2.0 deg/sec. The maximum torque to failure and angle to failure were determined from the force to angular displacement data.

Maximum torque to failure, maximum torsional rigidity, shear modulus, and maximum shear stress were calculated through standard equations (Ekeland, A., et al., Acta Orthop. Scand., 1981, 52(6):605-13; Engesaeter, L.B., et al., Acta Orthop. Scand., 1978, 49(6):512-8). Maximum torque to failure and maximum torsional rigidity are considered extrinsic properties while shear modulus and maximum shear stress are considered intrinsic properties. Maximum torque to failure was defined as the point where an increase in angular displacement failed to produce any further increase in torque. Maximum torsional rigidity is a function of the maximum torque to failure, gauge length (distance of the exposed femur between the embedded proximal and distal end) and angular displacement. Maximum shear stress is a function of the maximum torque to failure, maximum radius within the mid-diaphyseal region and the polar moment of inertia. The polar moment of inertia was calculated by modeling the femur as a hollow ellipse. Engesaeter et al. (1978) demonstrated that the calculated polar moment of inertia using the hollow ellipse model differed from the measured polar moment of inertia by only two percent (Engesaeter, L.B., et al., Acta Orthop. Scand., 1978, 49(6):512-8).

In order to compare the biomechanical parameters between different treatment groups, the data was normalized by dividing each fractured femur value by its corresponding intact, contralateral femur value (Figure 2). Normalization was used to minimize biological variability due to differences in age and weight among rats.

In addition to the biomechanical parameters determined through torsional testing, the mode of failure can also provide substantial information. The mode of torsional failure as determined by gross inspection provided an indication as to the extent of healing. A spiral failure in the mid-diaphyseal region indicated a complete union while a transverse failure through the fracture site indicated a nonunion. A combination spiral/transverse failure indicated a partial union (Figure 2).

*Data and Statistical Analysis*

Analysis of variance (ANOVA) was performed followed by Holm-Sidak post-hoc tests to determine differences between the treated $ZnCl_2$ groups with a group size larger than two. A Student's t-test was performed to identify differences between the two treated groups in the $ZnCl_2$ study (SigmaStat 3.0, SPSS Inc., Chicago, Illinois). A P value less than 0.05 was considered statistically significant.

*General Description of Animal Surgery*

A closed mid-diaphyseal fracture surgery was performed on the right femur of each rat as described previously. (Beam, H.A., et al., J. Orthop. Res. 2002, 20(6):1210-1216; Gandhi, A., et al., Bone 2006, 38(4):540-546.) General anesthesia was administered by intraperitoneal injection of ketamine (60 mg/kg) and xylazine (8 mg/kg). A closed, midshaft fracture was then created using a three-point bending fracture instrument (BBC Specialty Automotive, Linden NJ) and confirmed with X-rays immediately post-fracture.

*Preparation of $ZnCl_2$ solution*

Zinc chloride ($ZnCl_2$), Sigma Aldrich, St. Louis, MO, mixed with sterile water at various doses with or without a calcium sulfate carrier, were injected into the intramedullary canal prior to fracture. Doses of $ZnCl_2$ were not based on each animal's body weight, but on a lower theoretically tolerable dose for a 290-gram BB Wistar rat, which would not elicit heavy metal poisoning or behavioral changes. This weight is over 50 grams lower than the average weight of non-diabetic BB Wistar rats at an age of approximately 90 days (the age of investigation in this study). A 0.1 ml volume of the $ZnCl_2$ solution was administered locally via a single injection into the marrow space for each dose examined.

*Preparation of $ZnCl_2$/$CaSO_4$ formulation*

To prepare the $ZnCl_2$ / $CaSO_4$ mixture, $CaSO_4$ (2g) were placed in glass vials. The vials were placed in an autoclave and sterilized at for two hours in a dry cycle. $CaSO_4$ powder (0.8 g) was mixed with 400 $\mu$l of saline or 400 $\mu$l of $ZnCl_2$ solution (1.0 mg/kg) for one minute at room temperature. The mixture was packed into the barrel of a 1 cc sterile syringe and pushed down into the open orifice of the syringe barrel by insertion of the syringe plunger. After attaching an 18-gauge sterile needle to the syringe barrel, 0.1 ml volume of the mixture was directly injected into the rat femoral canal (non-diabetic BB Wistar rat) prior to Kirschner wire insertion and fracture.

*Microradiographic Evaluation*

Serial microradiographs were obtained from all animals every two weeks after surgery. Under the same anesthesia as described above, the rats were positioned prone and lateral and anteroposterior (AP) radiographs of their femurs were obtained. Radiographs were taken using a Packard Faxitron (MX 20 - Radiographic Inspection System) and Kodak MinR-2000 mammography film. Exposures were for 30 seconds at 55 kVp. Magnified radiographs were obtained of resected femurs. Qualitative analysis was performed on all radiographic sample at four weeks post-fracture. Two independent observers individually scored radiographs based on bridging of the lateral and AP femoral orientations. Treatment group averages were computed to estimate healing at 4 weeks post-fracture. The analysis was conducted in a blinded fashion using a validated, five-point radiographic scoring system, 0 = no evident bony bridging, 1 = bony bridging of one cortex, 2 = bony bridging of two cortices, 3 = bony bridging of three cortices, and 4 = bony bridging of all four cortices. *(See* Bergenstock, M.W., et al., J. Orthop. Trauma 2005, 19(10):717-723.)

*Torsional mechanical testing*

Torsional testing was conducted at four weeks using a servohydraulics machine (MTS Sys. Corp., Eden Prairie, MN) with a 20 Nm reaction torque cell (Interface, Scottsdale, AZ). Femurs were tested to failure at a rate of 2.0 deg/sec at four and six week time points. The peak torque, torsional rigidity, effective bulk modulus, and the effective maximum shear stress ($\sigma$) were determined with standard equations that model each femur as a hollow ellipse. (Ekeland, A., et al., Acta Orthop. Scand. 1981, 52(6):605-613; Engesaeter, L.B., et al., Acta Orthop. Scand. 1978, 49(6):512-518). In order to compare the biomechanical parameters between different groups, the data was normalized by dividing each fractured femur value by its corresponding intact, contralateral femur value. Torsional mechanical testing is limited by differences in gauge length during bone potting in Field's metal. Placement and dimension of fracture gap can contribute to standard deviations. Finally, this test is limited because it relies on a mathematical model that assumes the femur is a hollow ellipse, as opposed to the natural architecture of femoral bone. (Levenston, M.E., et al., J. Bone Miner. Res. 1994, 9(9): 1459-1465.)

*Early-stage healing analysis by histomorphometry*

The fractured femora were resected at seven days post-fracture, decalcified, dehydrated, embedded in paraffin, and sectioned using standard histological techniques. Sections were stained with Masson's Trichrome (Accustain™ Trichrome Staining kit, Sigma Diagnostics, St. Louis, MO) for histological observation using an Olympus BH2-RFCA microscope (Olympus Optical Co., Ltd., Shinjuku-ku, Tokyo, Japan). Digital images were collected using a Nikon DXM1200F digital camera (Nikon, Tokyo, Japan). Cartilage, new bone, and total callus area were measured from the digital images using Image-Pro Plus software (version 5, Media Cybernetics, Inc., Silver Spring, MD). Total cartilage and new bone area were normalized to total callus area and expressed as the percent area. Two independent reviewers were used to minimize inconsistencies.

*Late-stage healing analysis by histomorphometry*

To examine the effects of VAC at later stages of fracture healing, femora were resected from animals in the groups described above at day 21, embedded and sectioned using standard histological techniques. This includes dehydration, soaking in Xylenes, and finally pre-embedding in a layer of Polymethylmethacrylate (PMMA). After embedding in pure PMMA and allowed to solidify in a hot water bath, slides were sectioned from the PMMA blocks, polished, and stained with a combination of Stevenel's blue and Van Gieson picro-fuchsin (SVG). Histological images of fracture calluses were obtained using an Olympus SZX12 upright microscope (Olympus Optical Co, LTD, Japan) connected via a CCD camera (Optronics, Goleta, California) to a personal computer and analyzed with the Bioquant software package (Biometrics, Inc, Nashville, TN). Parameters that were compared include a) callus area, b) percent calcified tissue area, and c) percent cartilage area. Limitations of this procedure include production of slides with high thicknesses, due to the difficulties associated with sectioning PMMA. This limits the number of possible sections that may be cut for staining in addition to analysis of cellular morphology, due to overlapping layers of cells.

*General Health of Animals*

The age of the BB Wistar rats at the time of fracture surgery varied between 75 and 137 days. However, animals amongst treatment groups were age and sex matched for each experiment. The percent weight change following surgery to the day of sacrifice was similar amongst treatment groups.

RESULTS

**General Health**

In this experiment, the rats were 93-117 days old at time of fracture. No significant difference in percent weight gain was found between treatment groups from time of fracture until euthanization (Table 2). Blood glucose levels were higher in the zinc chloride treated rats, but the blood glucose values were within the normal range for all treatment groups (Table 2).

**Table 2.** General health of non-DM BB Wistar rats: local zinc ($ZnCl_2$) delivery without a carrier (Mechanical Testing)

| | Blood Glucose (mg/dl)* 12 Hours Post-Surgery | % Weight gain |
|---|---|---|
| Saline Control (n=6) | 81.7±4.3 [a] | 3.5±2.3 |
| 0.1 mg/kg $ZnCl_2$ (n=2) | 87.0±7.1 [a] | 15.3±11.5 |
| 1.0 mg/kg $ZnCl_2$ (n=3) | 99.3±3.1 [b] | 11.0±9.4 |
| 3.0 mg/kg $ZnCl_2$ (n=3) | 105.0±4.4 [b] | 6.9±11.7 |
| 6.0 mg/kg $ZnCl_2$ (n=4) | 88.0±4.3 [a] | 4.6±2.3 |
| 10.0 mg/kg $ZnCl_2$ (n=3) | 87. 7±8. 5 [a] | 4.2±2.0 |
| The data represents average values ± standard deviation<br>[a] represents values significantly less than the 3.0 mg/kg $ZnCl_2$ group; $p<0.05$<br>[b] represents values significantly less than the saline group; $p<0.05$ | | |

## Microradiographic Evaluation

At four weeks post-fracture, femurs from rats treated with $ZnCl_2$ had significantly higher radiograph scores than control femurs (Table 3).

## Mechanical Testing Results

*Local* $ZnCl_2$ *(no carrier)*

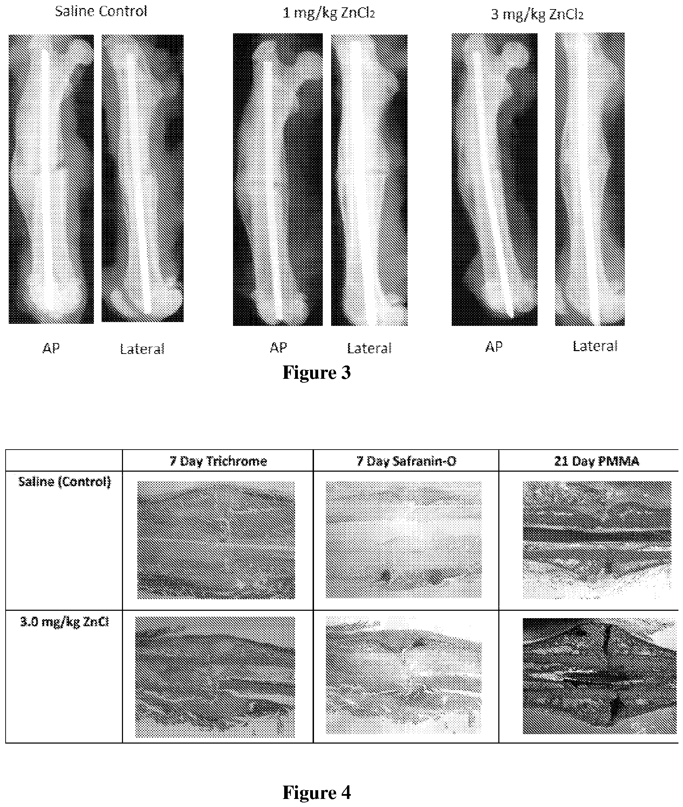

The effect of local zinc therapy on healing of femur fractures was measured by torsional mechanical testing. At four weeks post-fracture, rats treated with local $ZnCl_2$ displayed improved mechanical properties of the fractured femora compared to the untreated group. Radiographs taken at 4 weeks post-fracture support this finding (Figure 3). Table 3 represents the radiograph scoring values at two different dosages.

**Table 3.** Radiographic scoring evaluation

| | 4 Weeks Post-Fracture (# of cortices bridged) |
|---|---|
| Saline Control (n=6) | 1.2 ± 0.75 (n=6) |
| 1.0 mg/kg $ZnCl_2$ (n=3) | 3.0 ± 0.6* (n=3) |
| 3.0 mg/kg $ZnCl_2$ (n=3) | 3.3 ± 0.6* (n=3) |
| The data represents average values ± standard deviation<br>* Represent values statistically higher than control, $p<0.05$ | |

Table 4 summarizes the results of the mechanical testing of the bone for fractured bone, following four weeks of healing. The effective shear stress was 1.6x and 2.2x higher at four weeks post-fracture for the healing femurs from the $ZnCl_2$-treated animals, at dosages of 1.0 mg/kg and 3.0 mg/kg respectively. When normalized to their intact, contralateral femurs, the percent maximum torque to failure, percent torsional rigidity, and percent effective shear modulus, of the fractured femora were 2.0x, 3.8x, and 8.0x higher, respectively, at the dosage of 3 mg/kg $ZnCl_2$ compared to the control group ($p < 0.05$).

The effect of local zinc therapy on healing of femur fractures in normal (non-diabetic) rats was measured by torsional mechanical testing. At 4 weeks post-fracture, fractured femurs from the rats treated with zinc chloride had greater mechanical properties than the fractured femurs from the control group. For the 10 mg/kg $ZnCl_2$ group, the maximum torsional rigidity was significantly greater than the untreated group (Table 4). When the mechanical parameters of the fractured femora were normalized to the intact, contralateral femora, percent maximum torque to failure (saline group vs. 3 mg/kg $ZnCl_2$ group $p<0.05$), torsional rigidity (saline group vs. 3 mg/kg $ZnCl_2$ group $p<0.05$), and shear modulus (Saline group vs. 3 mg/kg $ZnCL_2$ group $p<0.05$, Saline group vs. 10 mg/kg $ZnCL_2$ group $p<0.05$) were significantly greater in the local zinc treated groups when compared to the saline group (Table 4).

Healing was assessed by radiographic examination and quantified by mechanical testing. Local $ZnCl_2$ treatment improved radiographic appearance and significantly increased the mechanical strength of fractured femurs.. At four weeks post-fracture, the average percent maximum torque to failure of the fractured femora for 3.0 mg/kg $ZnCl_2$ was significantly (2.04 times) greater (82.0% of contralateral vs. 27.0%), compared to the untreated saline group. Percent maximum torsional rigidity values for 3.0 mg/kg $ZnCl_2$ was significantly (3.85 times) greater (97.0% of contralateral vs. 20.0%), compared to the untreated saline group. Percent shear modulus values for both low (3.0 mg/kg $ZnCl_2$) and high (10.0 mg/kg $ZnCl_2$) doses were significantly greater, with high dose 8.8 times greater (36.0% of contralateral vs. 4.0%), and low dose 9.0 times greater (39.0% of contralateral vs. 4.0%) compared to the untreated saline group. The data indicate that local $ZnCl_2$ treatment enhanced bone regeneration during fracture healing and indicates that zinc and potentially similar metals can be used as therapeutically as osteogenic drugs.

**Table 4.** Four weeks post-fracture mechanical testing with local zinc ($ZnCl_2$)

| Fractured Femur Values | | | |
|---|---|---|---|
| | Maximum Torque to Failure (Nmm) | Maximum Torsional Rigidity (Nmm$^2$/rad) | Effective Shear Modulus (MPa) | Effective Shear Stress (MPa) |
| Saline Control (n=6) | $161 \pm 48$ | $9.9\times10^3 \pm 4.7\times10^3$ | $2.6\times10^2 \pm 1.1\times10^2$ | $17 \pm 4$ |
| 0.1 mg/kg $ZnCl_2$ (n=2) | $252 \pm 13$ | $2.1\times10^4 \pm 4.2\times10^3$ | $1.7\times10^3 \pm 3.3\times10^2$ | $61 \pm 14$ |
| 1.0 mg/kg $ZnCl_2$ (n=3) | $281 \pm 86$ | $2.2\times10^4 \pm 2.7\times10^3$ | $9.7\times10^2 \pm 3.6\times10^2$ | $44 \pm 15$ |
| 3.0 mg/kg $ZnCl_2$ (n=3) | $369 \pm 74$ | $3.1\times10^4 \pm 1.1\times10^4$ | $1.3\times10^3 \pm 6.4\times10^2$ | $55 \pm 21$* |
| 6.0 mg/kg $ZnCl_2$ (n=4) | $276 \pm 190$ | $2.9\times10^4 \pm 1.6\times10^4$ | $1.1\times10^3 \pm 7.5\times10^2$ | $32 \pm 25$* |
| 10.0 mg/kg $ZnCl_2$ (n=3) | $254 \pm 36$ | $3.6\times10^4 \pm 2.5\times10^4$ | $3.0\times10^3 \pm 1.9\times10^{3*}$ | $62 \pm 30$ |
| Fractured Femur Values Normalized to the Contralateral (Intact) Femur | | | |
| | Percent Maximum Torque to Failure | Percent Maximum Torsional Rigidity | Percent Effective Shear Modulus | Percent Effective Shear Stress |
| Saline Control (n=6) | $27 \pm 18$ | $20 \pm 10$ | $4 \pm 2$ | $10 \pm 5$ |
| 0.1 mg/kg $ZnCl_2$ (n=2) | $57 \pm 12$ | $87 \pm 14$ | $34 \pm 4$ | $33 \pm 14$ |
| 1.0 mg/kg $ZnCl_2$ (n=3) | $65 \pm 29$ | $55 \pm 14$ | $32 \pm 15$ | $18 \pm 8$ |

(continued)

| | Percent Maximum Torque to Failure | Percent Maximum Torsional Rigidity | Percent Effective Shear Modulus | Percent Effective Shear Stress |
|---|---|---|---|---|
| 3.0 mg/kg $ZnCl_2$ (n=3) | $82 \pm 25$* | $97 \pm 55$* | $36 \pm 10$* | $27 \pm 17$ |
| 6.0 mg/kg $ZnCl_2$ (n=4) | $38 \pm 20$ | $62 \pm 35$ | $18 \pm 12$ | $15 \pm 10$ |
| 10.0 mg/kg $ZnCl_2$ (n=3) | $41 \pm 8$ | $73 \pm 44$ | $39 \pm 23$* | $27 \pm 11$ |

The table is titled: **Fractured Femur Values Normalized to the Contralateral (Intact) Femur**

The data represents average values $\pm$ standard deviation* Represents values statistically higher than saline control, $p < 0.05$ versus saline control.
One way ANOVA between 6 groups (all pairwise) with a Holm-Sidak post-hoc analysis

### Histomorphometry of Zinc Chloride treated fractures

The results of histomorphometry of zinc chloride treated fractures after 7, 10, and 21 days are listed in Table 5 and illustrated in Figure 4.

**Table 5.** Histomorphometry of zinc chloride-treated fractures

| 7 Day | % Bone | % Cartilage |
|---|---|---|
| Saline Control (n=5) | $8.08 \pm 2.45$ | $3.00 \pm 1.7$ |
| 3.0 mg/kg (n=7) | $18.92 \pm 5.97$ * | $4.64 \pm 3.41$ |
| **10 Day** | | |
| Saline Control (n=5) | $17.90 \pm 5.20$ | $16.3 \pm 2.8$ |
| 3.0 mg/kg (n=7) | $21.31 \pm 5.40$ | $12.79 \pm 3.02$ |
| **21 Day** | | |
| Saline Control (n=6) | $25.00 \pm 6.10$ | $6.1 \pm 3.2$ |
| 3.0 mg/kg (n=7) | $24.47 \pm 3.53$ | $11.57 \pm 5.53$ |

*Local $ZnCl_2$ /$CaSO_4$ Formulations*

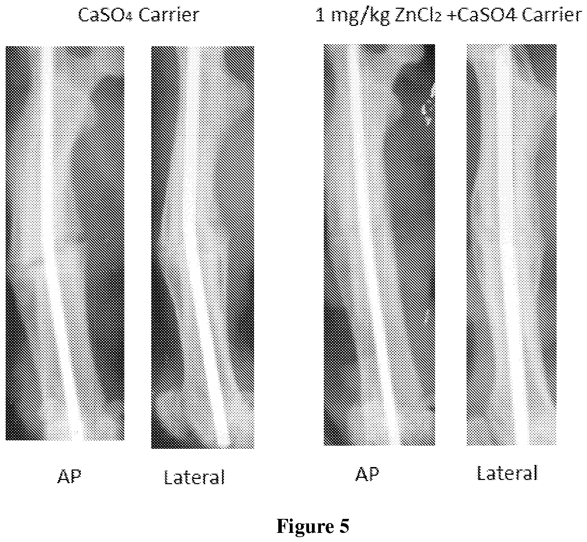

We repeated the above experiment with formulations of $ZnCl_2$/$CaSO_4$ applied to the fracture site. Radiographs taken at four weeks post-fracture support this finding (Figure 5) shows significant bone formation.

**Table 6**.  Four weeks post-fracture mechanical testing with formulation of zinc chloride ($ZnCl_2$) with $CaSO_4$ carrier applied to the fracture site.

| | Maximum Torque to Failure (Nmm) | Maximum Torsional Rigidity (Nmm²/rad) | Effective Shear Modulus (MPa) | Effective Shear Stress (MPa) |
|---|---|---|---|---|
| Fractured Femur Values | | | | |
| Saline Control (n=6) | 161 ± 48 | $9.9x10^3 \pm 4.7x10^3$ | $2.6x10^2 \pm 1.1x10^2$ | 17 ± 4 |
| CaSO4 Control (n=7) | 251 ± 78 | $2.1x10^4 \pm 1.3x10^4$ | $6.0x10^2 \pm 3.7x10^2$ | 26 ± 10 |
| 0.5 mg/kg ZnCl2 + CaSO4 (n=4) | 337 ± 175 | $3.0x10^4 \pm 7.9x10^3$ | $1.1x10^3 \pm 9.4x10^2$ | 36 ± 22 |
| 1.0 mg/kg ZnCl2 + CaSO4 (n=7) | 396 ± 112* | $3.9x10^4 \pm 1.4x10^4$*,# | $1.3x10^3 \pm 7.1x10^2$* | 46 ± 16* |
| 3.0 mg/kg ZnCl2 + CaSO4 (n=5) | 262 ± 126 | $2.1x10^4 \pm 7.8x10^3$ | $7.0x10^2 \pm 3.1x10^2$ | 33 ± 19 |
| | Percent Maximum Torque to Failure | Percent maximum Torsional Rigidity | Percent Effective Shear Modulus | Percent Effective Shear Stress |
| Fractured Femur Values Normalized to the Contralateral (Intact) Femur | | | | |
| Saline Control (n=6) | 27 ± 18 | 20 ± 10 | 4 ± 2 | 10 ± 5 |
| CaSO4 Control (n=7) | 48 ± 21 | 55 ± 35 | 11 ± 7 | 16 ± 7 |
| 0.5 mg/kg ZnCl2 + CaSO4 (n=4) | 56 ± 31 | 63 ± 20 | 17 ± 19 | 19 ± 12 |
| 1.0 mg/kg ZnCl2 + CaSO4 (n=7) | 75 ± 18* | 79 ± 32* | 18 ± 10 | 27 ± 8* |
| 3.0 mg/kg ZnCl2 + CaSO4 (n=5) | 45 ± 22 | 52 ± 22 | 14 ± 8 | 20 ± 14 |

The data represents average values ± standard deviation

\* Represents values statistically higher than saline control, $p < 0.05$ versus saline control.

\# Represents values statistically higher than CaSO4 control, $p < 0.05$ versus CaSO4 control.

One-way ANOVA between 5 groups with Holm-Sidak post-hoc analysis

Table 6 summarizes the results of the mechanical testing of the bone for fractured bone, following four weeks of healing using the formulation. The effective shear stress was 2.7x and 1.7x higher at four weeks post-fracture for the healing femurs from the $ZnCl_2$/ $CaSO_4$ treated animals, at dosages of 1.0 mg/kg compared to saline and $CaSO_4$ control, respectively. When normalized to their intact, contralateral femurs, the percent maximum torque to failure, percent torsional rigidity, and percent effective shear modulus, of the fractured femora were 2.8x, 4.0x, and 4.5x higher, respectively, at the dosage of 1 mg/kg $ZnCl_2$ $CaSO_4$ compared to the saline control group ($p < 0.05$).

*Comparison of use of $ZnCl_2$ with existing therapy (BMP2)*

As an insulin-mimetic adjunct, zinc compounds can be used to accelerate bone regeneration by stimulating insulin signaling at the fracture site. $ZnCl_2$ treatment applied directly to the fracture site significantly increased the mechanical parameters of the bone in treated animals after four weeks, compared to controls. It accelerated fracture-healing process (fracture healing resolved in four to five weeks, instead of average eight to ten weeks in standard rat femur fracture model).

Other healing adjuncts currently approved for FDA use in the United States include Bone Morphogenic Proteins (BMP's) and Exogen/ Pulsed Electromagnetic Fields (PEMF). However, BMPs may be associated with shortcomings such as causing ectopic bone growth and having high cost per application; and Exogen/PEMF therapy has shown only limited proven usefulness in fracture healing and needs for patient compliance for daily use.

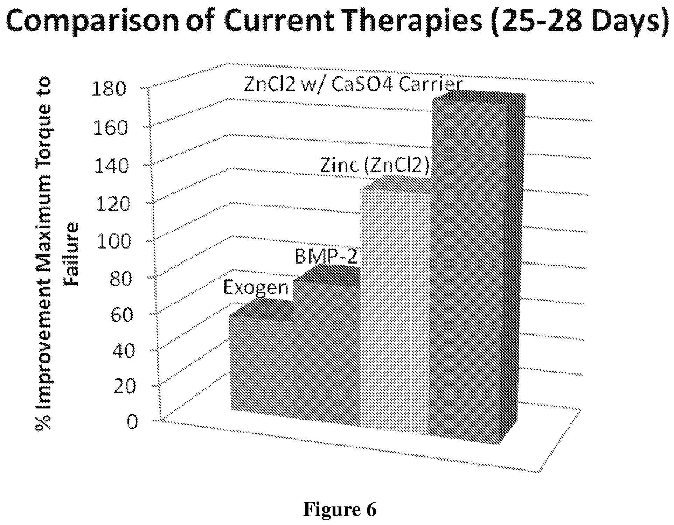

The chart in Figure 6 compares the use of $ZnCl_2$ (alone or in combination with $CaSO_4$) with the currently approved products (BMP-2 and Exogen) for fracture healing. Each of these studies examined the effectiveness of a therapeutic adjunct on femur fracture healing by measuring the maximum torque to failure at the four week time point. Specifically the following were compared to their repective untreated control group: (1) a single intramedullary dose (1 mg/kg) of ZnCl2 with the calcium sulfate ($CaSO_4$) vehicle (purple); (2) a single intramedullary dose (3 mg/kg) of ZnCl2 without a vehicle (green); (3) BMP-2 study used a single percutaneous dose of BMP-2 (80 mg) with buffer vehicle (red) (see Einhorn, T.A., et al., J. Bone Joint Surg. Am. 2003, 85-A(8):1425-1435); and (4) Exogen study used daily exposure periods of ultrasound treatment (20 min/day). The average value (duration of 25 days) is shown in blue (see Azuma, Y., et al., J. Bone Miner. Res. 2001,16(4):671-680.

As graphically shown, use of single application of insulin-mimetic like zinc chloride results in significantly increased improvement of torque to failure and other mechanical properties of the fracture callus, compared to the existing gold standard of LIPUS and BMP2, using torsional mechanical testing of rat femur fracture model of Bonnarrens and Einhorn.

In summary, we have found that acute, local $ZnCl_2$ treatment (either alone or as a formulation with a carrier), administered immediately prior to an induced fracture, promoted healing in non-diabetic rats. At the four week time point, mechanical parameters of the healed bone were substantially higher than that of the control group. This is consistent with our earlier findings of insulin's ability to promote bone growth when applied to the fracture site. This is also consistent with our finding that insulin mimetic compounds such as vanadyl acetylacetonate (VAC) accelerate fracture healing much like insulin. Though also an insulin mimetic, unlike VAC, $ZnCl_2$ is a compound commonly used in many commercial medical products and hence potential regulatory barriers are minimal. This suggests that insulin mimetics applied locally to the fracture may be used therapeutically as a fracture-healing adjunct, and local $ZnCl_2$ treatment is a cost-effective fracture-healing adjunct and has potential for other possible orthopedic applications.

The above preliminary data indicate that local treatment with an insulin-mimetic such as zinc is an effective method to enhance bone regeneration. Mechanical parameters and radiography revealed that bone bridged at four weeks after fracture in the zinc-treated rats as compared to saline treated controls. Spiral fractures that occurred during mechanical testing support the radiographic observations and suggest that local $ZnCl_2$ application at the dosages tested may accelerate fracture healing, compared to untreated controls. These data support additional testing of $ZnCl_2$ as a therapeutic agent to accelerate or enhance bone regeneration.

REFERENCE EXAMPLE 1

**Use of Manganese Compounds for Fracture Healing**

MATERIAL AND METHODS

**Rat Model**

The animal model used for this study is the Diabetes Resistant (DR) BB Wistar Rat. It will be obtained from a breeding colony at UMDNJ-New Jersey Medical School (NJMS) which is maintained under controlled environmental conditions and fed ad libitum.

The BB Wistar colony was established from diabetic-prone BB Wistar rats originally obtained from BioBreeding (Toronto, Canada). Similar to human type I diabetes, spontaneously diabetic BB Wistar rats display marked hyperglycemia, glycosuria and weight loss within a day of onset, associated with decreased plasma insulin after undergoing selective and complete destruction of pancreatic β-cells. If left untreated, diabetic BB Wistar rats would become ketoacidic within several days, resulting in death. Genetic analysis of the BB-Wistar rat shows the development of diabetes is strongly related to the presence of the *iddm4* diabetogenic susceptibility locus on chromosome 4 as well as at least four other loci related to further susceptibility and the development of lymphopenia (Martin, A.M., et al., Diabetes 1999, 48(11):2138-44).

The DR-BB Wistar rat colony was also originally purchased from BioBreeding and has been established as an effective control group for studies involving the diabetic BB Wistar rat. Under controlled environmental conditions, DR-BB Wistar rats would never develop spontaneous type I diabetes, are non-lymphopenic, and are immunocompetent. It has since been used in our lab as a model of a "normal" rat model. The choice was made to utilize the DR-BB Wistar rat, rather than purchase commercially available rats for our studies, because of the ability to expand the colony by breeding at any time as necessary for different protocols, as well our familiarity with the rat over years of its utilization in similar protocols. The consistent use of the BB Wistar and the DR-BB Wistar rat models allow for an increase in reliability when comparing data between our various protocols.

**General Health of Animals**

The age of the BB Wistar rats at the time of fracture surgery varied between 95 and 137 days. However, animals amongst treatment groups were age and sex matched for each experiment. The percent weight change following surgery to the day of sacrifice was similar amongst treatment groups.

**Surgical Technique**

Surgery will be performed to produce a closed mid-diaphyseal fracture model in the right femur. General anesthesia will be administered prior to surgery by intraperitoneal (IP) injection of ketamine (60mg/kg) and xylazine (8mg/kg). The right leg of each rat is shaved and the incision site is prepared with Betadine and 70% alcohol. A one centimeter medial, parapatellar skin incision is made, followed by a smaller longitudinal incision through the quadriceps muscle, just proximal to the quadriceps tendon. The patella is dislocated laterally and the intercondylar notch of the distal femur is exposed. An entry hole is made with an 18-guage needle and the femoral intramedullary canal is subsequently reamed. For experimental groups, 0.1mL of MnCl2 solution(of different dosage) is injected into the medullary canal of the femur. For control groups, 0.1mL of saline is injected. A Kirschner wire (316LVM stainless steel, 0.04 inch diameter, Small Parts, Inc., Miami Lakes, FL) is inserted into the intramedullary canal. The Kirschner wire is cut flush with the femoral condyles. After irrigation, the wound is closed with 4-0 vicryl resorbable sutures. A closed midshaft fracture is then created unilaterally with the use of a three-point bending fracture machine. X-rays are taken to determine whether the fracture is of acceptable configuration. Only transverse, mid-diaphyseal fractures are accepted. The rats are allowed to ambulate freely immediately post-fracture.

**Post Surgery Procedures**

X-rays are taken at two-week intervals to the day of euthanasia. After euthanasia x-rays are taken as well. To take x-rays, animals will be given a half dose of anesthesia. All groups will be monitored closely for four days after surgery for infection, and the ability to ambulate freely.

**Torsional Mechanical Testing**

Torsional testing was conducted at 4 weeks post-fracture, using a servohydraulics machine (MTS Sys. Corp., Eden Prairie, MN) with a 20 Nm reaction torque cell (Interface, Scottsdale, AZ). Femurs were tested to failure at a rate of 2.0 deg/sec at four weeks post-fracture. The peak torque, torsional rigidity, effective bulk modulus, and the effective maximum shear stress ($\sigma$) were determined with standard equations that model each femur as a hollow ellipse (Ekeland, A., et al., Acta Orthop. Scand. 1981, 52(6):605-613; Engesaeter, L.B., et al., Acta Orthop. Scand. 1978, 49(6):512-518). In order to compare the biomechanical parameters between different groups, the data was normalized by dividing each fractured femur value by its corresponding intact, contralateral femur value. Torsional mechanical testing is limited by differences in gauge length during bone potting in Field's metal. Placement and dimension of fracture gap can contribute to standard deviations. Finally, this test is limited because it relies on a mathematical model that assumes the femur is a hollow ellipse, as opposed to the natural architecture of femoral bone (Levenston, M.E., et al., J. Bone Miner. Res. 1994, 9(9):1459-1465).

**Early-Stage Healing Analysis by Histomorphometry**

The fractured femora were resected at seven and ten days post-fracture, decalcified, dehydrated, embedded in paraffin, and sectioned using standard histological techniques. Sections were stained with Masson's Trichrome (Accustain™ Trichrome Staining kit, Sigma Diagnostics, St. Louis, MO) for histological observation using an Olympus BH2-RFCA microscope (Olympus Optical Co., Ltd., Shinjuku-ku, Tokyo, Japan). Digital images were collected using a Nikon DXM1200F digital camera (Nikon, Tokyo, Japan). Cartilage, new bone, and total callus area were measured from the digital images using Image-Pro Plus software (version 5, Media Cybernetics, Inc., Silver Spring, MD). Total cartilage and new bone area were normalized to total callus area and expressed as the percent area. Two independent reviewers were used to minimize inconsistencies.

**Data and Statistical Analysis**

Analysis of variance (ANOVA) was performed followed by Holm-Sidak post-hoc tests to determine differences between the treated $MnCl_2$ groups with a group size larger than two. A Student's t-test was performed to identify differences between the two treated groups in the $MnCl_2$ study (SigmaStat 3.0, SPSS Inc., Chicago, Illinois). A p value less than 0.05 was considered statistically significant.

RESULTS

**Mechanical Testing**

*Local $MnCl_2$ no carrier*

The effect of local MnCl2 therapy on healing of femur fractures was measured by torsional mechanical testing. At four weeks post-fracture, rats treated with $NnCl_2$ displayed improved mechanical properties of the fractured femora compared to the saline control group. The maximum torque to failure was significantly increased compared to the saline control group ($p < 0.05$: 0.125 mg/kg $MnCl_2$, $p < 0.05$: 0.25 mg/kg $MnCl_2$, , $p < 0.05$: 0.3 mg/kg $MnCl_2$) (Table 7). When the mechanical parameters of the fractured femora were normalized to the intact, contralateral femora, percent torsional rigidity was significantly greater in the local $MnCl_2$ treated groups when compared to the saline control group ($p < 0.05$: 0.125 mg/kg $MnCl_2$, $p < 0.05$: 0.25 mg/kg $MnCl_2$) (Table 7).

**Table 7.** Four weeks post-fracture mechanical testing with local manganese chloride ($MnCl_2$)

| Fractured Femur Values | | | |
|---|---|---|---|
| | Maximum Torque to Failure (Nmm) | Maximum Torsional Rigidity ($Nmm^2$/rad) | Effective Shear Modulus (MPa) | Effective Shear Stress (MPa) |
| Saline Control (n=6) | $161 \pm 48$ | $9.9 \times 10^3 \pm 4.7 \times 10^3$ | $2.6 \times 10^2 \pm 1.1 \times 10^2$ | $17 \pm 4$ |
| 0.083 mg/kg $MnCl_2$ (n=5) | $272 \pm 39$ | $2.6 \times 10^4 \pm 1.2 \times 10^4$ | $8.7 \times 10^2 \pm 4.9 \times 10^2$ | $30 \pm 8$ |
| 0.125 mg/kg $MnCl_2$ (n=4) | $351 \pm 59$* | $4.2 \times 10^4 \pm 1.1 \times 10^4$ | $6.4 \times 10^2 \pm 8.8 \times 10^1$ | $21 \pm 6$ |
| 0.25 mg/kg $MnCl_2$ (n=4) | $344 \pm 84$* | $3.4 \times 10^4 \pm 1.6 \times 10^4$ | $8.1 \times 10^2 \pm 5.0 \times 10^2$ | $32 \pm 11$ |
| 0.30 mg/kg $MnCl_2$ (n=6) | $323 \pm 135$* | $3.0 \times 10^4 \pm 2.6 \times 10^4$ | $7.6 \times 10^2 \pm 9.2 \times 10^2$ | $27 \pm 23$ |
| 0.50 mg/kg $MnCl_2$ (n=6) | $230 \pm 83$ | $2.9 \times 10^4 \pm 1.2 \times 10^4$ | $6.2 \times 10^2 \pm 3.5 \times 10^2$ | $19 \pm 9$ |
| Fractured Femur Values Normalized to the Contralateral (Intact) Femur | | | |
| | Percent Maximum Torque to Failure | Percent maximum Torsional Rigidity | Percent Effective Shear Modulus | Percent Effective Shear Stress |
| Saline Control (n=6) | $27 \pm 18$ | $20 \pm 10$ | $4 \pm 2$ | $10 \pm 5$ |
| 0.083 mg/kg $MnCl_2$ (n=5) | $42 \pm 5$ | $56 \pm 30$ | $8 \pm 7$ | $8 \pm 4$ |
| 0.125 mg/kg $MnCl_2$ (n=4) | $54 \pm 5$ | $103 \pm 40$* | $16 \pm 11$ | $14 \pm 5$ |
| 0.25 mg/kg $MnCl_2$ (n=4) | $55 \pm 19$ | $80 \pm 34$* | $14 \pm 9$ | $16 \pm 6$ |
| 0.30 mg/kg $MnCl_2$ (n=6) | $50 \pm 22$ | $50 \pm 37$ | $10 \pm 12$ | $16 \pm 12$ |

(continued)

| Fractured Femur Values Normalized to the Contralateral (Intact) Femur | | | | |
|---|---|---|---|---|
| | Percent Maximum Torque to Failure | Percent maximum Torsional Rigidity | Percent Effective Shear Modulus | Percent Effective Shear Stress |
| 0.50 mg/kg $MnCl_2$ (n=6) | 38 ± 15 | 61 ± 16 | 17 ± 13 | 14 ± 7 |
| The data represents average values ± standard deviation * Represents values statistically higher than saline control, p < 0.05 versus saline control. | | | | |

### Radiographic analysis

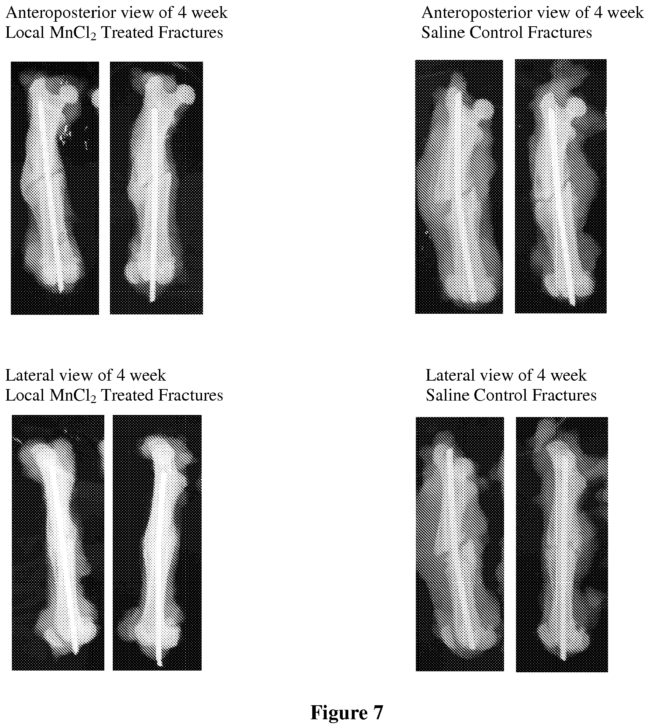

Radiographs taken at four weeks post-fracture support these mechanical testing results (Figure 7). At four weeks, the fractures treated with 0.25 mg/kg dosage of $MnCl_2$ displayed increased mineralized tissue than saline controls. Additionally, analysis of radiographs showed the $MnCl_2$ group demonstrated union at the subperiosteal bony area and at the callus, whereas saline control radiographs had no evidence of union.

### Histomorphometric Analysis

In animals treated with MnCl2, histomorphometric analysis revealed a statistically lower ($p < 0.05$) percent cartilage in 0.3 mg/kg Mn Cl2 treated femora, compared to controls at seven days (Table 8). At ten days, percent mineralized tissue in 0.3 mg/kg Mn Cl2 treated femora were significantly increased ($p < 0.05$: 0.3 mg/kg Mn Cl2) compared to saline controls (Table 8).

**Table 8.** Histology: comparison of manganese chloride with saline control

| Group | 7 days post fracture | | 10 days post fracture | |
|---|---|---|---|---|
| | % cartilage | % new bone | % cartilage | % new bone |
| Saline | 6.116 ± 2.51 n=5 | 15.668 ± 2.93 n=5 | 9.542 ± 1.02 n=5 | 14.011 ± 1.29 n=5 |
| 0.125 mg/kg | 4.843 ± 2.3 n=6 | 15.518 ± 2.69 n=6 | 5.9 ± 4.47 n=5 | 14.69 ± 11.74 n=5 |
| 0.3 mg/kg | 2.859 ± 1.09 n=7 | 15.604 ± 2.39 n=7 | 11.051 ± 3.05 n=9 | 18.866 ± 2.28*,# n=9 |
| 0.87 mg/kg | 4.355 ± 3.99 n=3 | 16.492 ± 3.59 n=3 | 8.227 ± 2.24 n=3 | 15.948 ± 3.04 n=3 |
| * Represents values statistically higher than saline control, p<0.001 # Represents values statistically lower than saline control, p<0.05 | | | | |

REFERENCE EXAMPLE 2

### Use of Vanadium Compounds for Bone Fracture Healing

### Method

*General Description of Animal Surgery*

A closed mid-diaphyseal fracture surgery was performed on the right femur of each rat as described previously. General anesthesia was administered by intraperitoneal injection of ketamine (60 mg/kg) and xylazine (8 mg/kg). A closed, midshaft fracture was then created using a three-point bending fracture instrument (BBC Specialty Automotive, Linden NJ) and confirmed with X-rays immediately post-fracture.

*Preparation of VAC solution*

Vanadyl acetylacetonate (VAC), Sigma Aldrich, St. Louis, MO, mixed with sterile water at various doses with or without a calcium sulfate carrier, were injected into the intramedullary canal prior to fracture. VAC was chosen over alternative organo-vanadium compounds such as BMOV and VS, due to its' observed superior potency at stimulating protein kinase B (PKB), Glycogen synthase kinase 3 beta (GSK-3$\beta$), and protein tyrosine phosphorylation (PTP). Additionally, Mehdi et al. noted more potent Insulin receptor beta subunit (IR$\beta$), and Insulin receptor substrate 1 (IRS-1) tyrosine phosphorylation, for VAC, compared to BMOV and VS. Doses of VAC were not based on each animal's body weight, but on a lower theoretically tolerable dose for a 290 gram BB Wistar rat, which would not elicit heavy metal poisoning or behavioral changes. This weight is over 50 grams lower than the average weight of non-diabetic BB Wistar rats at an age of approximately 90 days (the age of investigation in this study). The daily subcutaneous dose injected by Zhang et al. (3mg VAC/kg body weight) was multiplied by this average weight of 0.29 kg. A 0.1 mL volume of the VAC solution was administered locally via a single injection into the marrow space for each dose examined. This reduced the absolute concentration of VAC administered in the high dose to the same concentration as Zhang et al., while the 1.5 mg/kg dose was 50% of the dose administered by Zhang. Later the 0.5 mg/kg dose (33.3% of low dose) and 0.25 mg/kg dose (16.6% of low dose) were evaluated to determine the optimal dose of VAC, and examine the range of effectiveness of VAC.

*Preparation of VAC/CaSO$_4$ formulation*

To prepare the CaSO$_4$-VAC mixture, two grams of CaSO$_4$ were placed in glass vials. The vials were placed in an autoclave and sterilized at for two hours in a dry cycle. CaSO$_4$ powder (0.8 grams) was mixed with 400 $\mu$l of saline or 400 $\mu$l of VAC solution (0.25 mg/kg and 1.5 mg/kg) for one minute at room temperature. The mixture was packed into the barrel of a 1 cc sterile syringe and pushed down into the open orifice of the syringe barrel by insertion of the syringe plunger. After attaching an 18-gauge sterile needle to the syringe barrel, 0.1 ml volume of the mixture was directly injected into the rat femoral canal (non-diabetic BB Wistar rat) prior to Kirschner wire insertion and fracture.

*Pack Boriding (Vanadium-Boron and Boron Control) Stainless Steel Rod Manufacturing:*

During boriding of steel and other metallic and alloy surfaces, boron atoms diffuse into the material and form various types of metal borides.

A 1.6 mm Kirschner wire was annealed, cleaned and packed in a boriding powder mixture contained within a 5 mm thick, heat resistant steel box. This allows the surfaces to be borided with a layer that is 10-20 micrometers thick. A mixture was made consisting of boron carbide, VAC, silicon carbide, and a boriding activator. The parts conformed to the container which they were packed, and then covered with a lid, which rests inside the container. This container was then weighted with an iron slug to ensure even trickling of the boriding agent during the manufacturing. The container was then heated to the boriding temperature in an electrically heated box with covered heating coils. The coated rods were allowed to come to room temperature and wiped with 95% ethyl alcohol prior to surgery for sterilization.

*Vanadium quantification in animal models*

BB Wistar rats were anesthetized and confirmed to be non-responsive to external stimuli before beginning the surgical procedure. The anesthetized rat was be exsanguinated by cardiac puncture using a 10 ml syringe with a 22 gauge needle after shallow puncture just lateral to the sternum and through the intercostal space. After puncturing the dermis and cardiac wall slight backpressure was placed on the plunger to withdraw blood from the ventricle. The collected blood was transferred to an appropriate container used for collection of plasma (heparinized) or serum (non-heparinized). Following the cardiac puncture, the rats were euthanized via cervical dislocation.

The excised femora were be stripped of adhering muscle, tendon and other tissue after which the bones were rinsed three times with deionized water, and then placed on glassine paper and air dried. The pin was rinsed once and stored in a clean conical tube. The liver, kidneys, brain, and left humerous collected were rinsed 3x and air dried. The objective of "drying" was to remove adhering water droplets after the water rinse and to allow the true tissue weight to be recorded as precisely as possible. The tissue's location on the glassine paper was changed after a minute or two of air exposure. Air-drying does not last longer than 5 minutes. The dry bone is placed into a dry, previously acid-soaked/deionized-water rinsed, 7 ml pre-weighed sealable scintillation vial with plastic liner cap. Other organs were also stored in pre-rinsed, dry pre-weighed plastic vials of sizes appropriate for each. The vials were labeled with an indelible marker indicating the date of collection, right or left femur, Rat ID code, Investigator and Study ID. The organs were then placed into a low temperature (-80 degrees Celsius) freezer for storage until future analysis (quantification not currently planned).

The bone was carefully air-dried and any entrained fluid in the endosteal space drawn or shaken out to avoid bone weight anomaly. Beakers for subsequent collections were re-cleaned and femurs were handled carefully to avoid cross contamination of specimens. Bones were analyzed via atomic absorption spectrophotometry to determine levels of vanadium in bone, compared to standard levels in normal rat femoral bone. Analysis was based on a standard published technique14 for quantifying vanadium levels in tissues.

*Early-stage healing analysis by histomorphometry*

The fractured femora were resected at 2, 4, 7, and 10 days post-fracture, decalcified, dehydrated, embedded in paraffin, and sectioned using standard histological techniques. Sections were stained with Masson's Trichrome (Accustain™ Trichrome Staining kit, Sigma Diagnostics, St. Louis, MO) for histological observation using an Olympus BH2-RFCA microscope (Olympus Optical Co., Ltd., Shinjuku-ku, Tokyo, Japan). Digital images were collected using a Nikon DXM1200F digital camera (Nikon, Tokyo, Japan). Cartilage, new bone, and total callus area were measured from the digital images using Image-Pro Plus software (version 5, Media Cybernetics, Inc., Silver Spring, MD). Total cartilage and new bone area were normalized to total callus area and expressed as the percent area. Two independent reviewers were used to minimize inconsistencies.

*Late-stage healing analysis by histomorphometry*

To examine the effects of VAC at later stages of fracture healing, femora were resected from animals in the groups described above at days 10, 14, and 21, embedded and sectioned using standard histological techniques. This includes dehydration, soaking in Xylenes, and finally pre-embedding in a layer of Polymethylmethacrylate (PMMA). After embedding in pure PMMA and allowed to solidify in a hot water bath, slides were sectioned from the PMMA blocks, polished, and stained with a combination of Stevenel's blue and Van Gieson picro-fuchsin (SVG). Histological images of fracture calluses were obtained using an Olympus SZX12 upright microscope (Olympus Optical Co, LTD, Japan) connected via a CCD camera (Optronics, Goleta, California) to a personal computer and analyzed with the Bioquant software package (Biometrics, Inc, Nashville, TN). Parameters that were compared include a) callus area, b) percent calcified tissue area, and c) percent cartilage area. Limitations of this procedure include production of slides with high thicknesses, due to the difficulties associated with sectioning PMMA This limits the number of possible sections that may be cut for staining in addition to analysis of cellular morphology, due to overlapping layers of cells.

*Early Immuohistochemistry*

At days 2, 4, and 7, and 10, animals were injected intraperitoneally with 30 mg/kg of 5-bromo-2'deoxyuridine (BrdU, Sigma Chemical Co., St. Louis, MO) one hour prior to sacrifice to label replicating cells as a measure of cellular proliferation. The fractured femurs were resected and fixed in formalin, decalcified (Immunocal, Decal Corp., Tallman, NY), embedded in paraffin, and sectioned longitudinally (5 $\mu$m thick). Cells positive for BrdU incorporation were detected by immunohistochemistry using commercially available reagents (DAKO Corp., Carpentaria, California). Digital images of each fracture were collected with an Olympus BH2-RFCA microscope equipped with a Nikon DXM1200f camera. For each specimen, callus area was measured and the BrdU positive cells in the periosteal callus region were counted using Image Pro Plus software. All BrdU positive cells in the external callus to a maximum of 1 cm proximal and distal of the fracture site and 3 mm from the external surface of the femur were counted. The number of BrdU positive cells was normalized per unit area of callus and only one datum per rat (BrdU positive cells per mm2) was used for the statistical analysis.

*Torsional mechanical testing*

Torsional testing was conducted at weeks four and five using a servohydraulics machine (MTS Sys. Corp., Eden Prairie, MN) with a 20 Nm reaction torque cell (Interface, Scottsdale, AZ). Femurs were tested to failure at a rate of 2.0 deg/sec at four and six week time points. The peak torque, torsional rigidity, effective bulk modulus, and the effective maximum shear stress ($\sigma$) were determined with standard equations that model each femur as a hollow ellipse. In order to compare the biomechanical parameters between different groups, the data was normalized by dividing each fractured femur value by its corresponding intact, contralateral femur value. Torsional mechanical testing is limited by differences in gauge length during bone potting in Field's metal. Placement and dimension of fracture gap can contribute to standard deviations. Finally, this test is limited because it relies on a mathematical model that assumes the femur is a hollow ellipse, as opposed to the natural architecture of femoral bone.

*Data and Statistical Analysis*

Analysis of variance (ANOVA) was performed followed by Holm-Sidak post-hoc tests to determine differences between the treated VAC groups with a group size larger than two. A Student's t-test was performed to identify differences between the two treated groups in the VAC study (SigmaStat 3.0, SPSS Inc., Chicago, Illinois). A p value less than 0.05 was considered statistically significant.

*General Health of Animals*

The age of the BB Wistar rats at the time of fracture surgery varied between 75 and 137 days. However, animals amongst treatment groups were age and sex matched for each experiment. The percent weight change following surgery to the day of sacrifice was similar amongst treatment groups.

**Results**

*Vanadium Quantification in Animal Models*

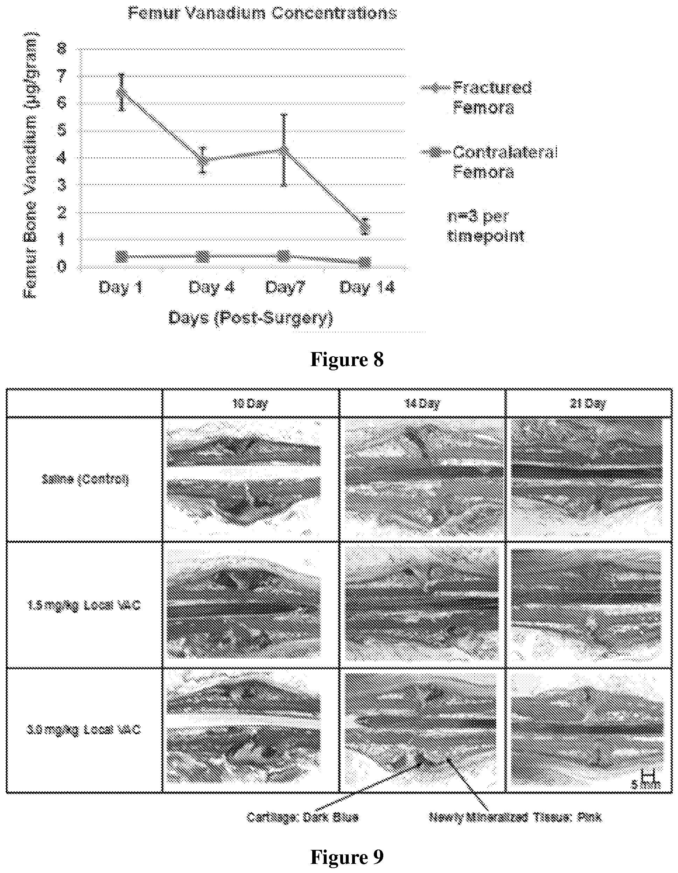

Locally injected VAC remains bound within the fractured femora approximately two weeks after local injection. These results were determined from the following experiment. Immediately prior to fracture, the femoral canal of each rat was injected with 0.1 mL of either saline or 4.35 mg/mL of VAC solution (4.35 mg/ml VAC solution; approximately 1.5 mg VAC/kg weight of the rat; approximately 435 $\mu$g of VAC powder; approximately 84 $\mu$g of vanadium). To assess how rapidly the vanadium disperses from the fracture site, rats were sacrificed at one, four, seven, and 14 days after surgery to measure vanadium levels in the fracture callus. Atomic absorption spectrophotometry was used to quantify local vanadium levels and normalized compared to levels in normal rat femur bone. Significant differences ($p < 0.05$) in local vanadium levels were detected between the right, fractured femora and left, non-fractured femora of the rats treated with local vanadium at all time points examined (Figure 8). The half-life of VAC is relatively short (6 days) according to Zhang et al and the quantity within the fractured femora significantly decreased at four, seven, and 14 days compared to the contralateral femora. At 14 days, the local level of vanadium was significantly decreased ($p < 0.05$) compared to days one, four, and seven.

*Histomorphometric Analysis*

In animals treated with VAC, histomorphometric analysis revealed a statistically higher ($p < 0.05$) percent cartilage in 1.5 mg/kg VAC treated femora, compared to controls at both 7 and 10 days (Table 9). At 14 days, percent mineralized tissue in both 1.5 mg/kg and 3 mg/kg VAC treated femora were significantly increased ($p < 0.05$: 1.5mg/kg VAC, $p < 0.05$: 3 mg/kg VAC) compared to saline controls (Table 9). After 21 days, percent mineralized tissue was significantly increased ($p < 0.05$) in 1.5 mg/kg VAC treated femora. This VAC-mediated acceleration of healing may be seen via histological sections at days 10-21 (Table 9; Figure 9).

**Table 9.** Late histology local VAC delivery without a carrier in normal rats

| | 10 Days Post-Fracture | | | 14 Days Post-Fracture | | |
|---|---|---|---|---|---|---|
| | Callus Area (mm$^2$) | % Mineralized Tissue | % Cartilage | Callus Area (mm$^2$) | % Mineralized Tissue | % Cartilage |
| Saline Control | 15.8 $\pm$ 2.3 (n=5) | 17.9 $\pm$ 5.2 (n=5) | 16.3 $\pm$ 2.8 (n=5) | 19.5 $\pm$ 4.8 (n=6) | 15.6 $\pm$ 4.7 (n=6) | 12.4 $\pm$ 4.9 (n=6) |
| 1.5 mg/kg VAC | 18.4 $\pm$ 3.6 (n=7) | 15.8 $\pm$ 5.1 (n=7) | 30.6 $\pm$ 12.4* (n=7) | 21.7 $\pm$ 5.0 (n=5) | 21.3 $\pm$ 2.1* (n=5) | 14.2 $\pm$ 4.9 (n=5) |
| 3.0 mg/kg VAC | 18.6 $\pm$ 2.9 (n=5) | 15.2 $\pm$ 3.4 (n=5) | 17.8$\pm$ 5.5 (n=5) | 19.3 $\pm$ 3.1 (n=5) | 21.9 $\pm$ 3.2* (n=5) | 13.0 $\pm$ 6.4 (n=5) |

(continued)

|  | 10 Days Post-Fracture | | | 14 Days Post-Fracture | | |
|---|---|---|---|---|---|---|
|  | Callus Area (mm$^2$) | % Mineralized Tissue | % Cartilage | Callus Area (mm$^2$) | % Mineralized Tissue | % Cartilage |
|  | 21 Days Post-Fracture | | | | | |
|  | Callus Area (mm$^2$) | % Mineralized Tissue | % Cartilage | | | |
| Saline Control | 20.0± 6.8 (n=6) | 25.0 ± 6.1 (n=6) | 6.1 ± 3.2 (n=6) | | | |
| 1.5 mg/kg VAC | 20.1 ± 4.5 (n=5) | 32.7 ± 2.9* (n=5) | 11.0± 4.7 (n=5) | | | |
| 3.0 mg/kg VAC | 20.3± 5.1 (n=4) | 33.4 ± 5.2 (n=4) | 9.4± 5.7 (n=4) | | | |
| The data represent mean values (± S.D.).<br>* Represents values statistically higher than saline control, p < 0.05 versus saline control. | | | | | | |

*Early Immunohistochemistry*

In animals treated with VAC, no significant differences in cell proliferation existed at two or four days post-fracture, but significantly more proliferating cells per unit area (p < 0.05) was observed in the periosteum at seven and ten days post-fracture.

**Mechanical Testing**

*Local VAC without a Carrier*

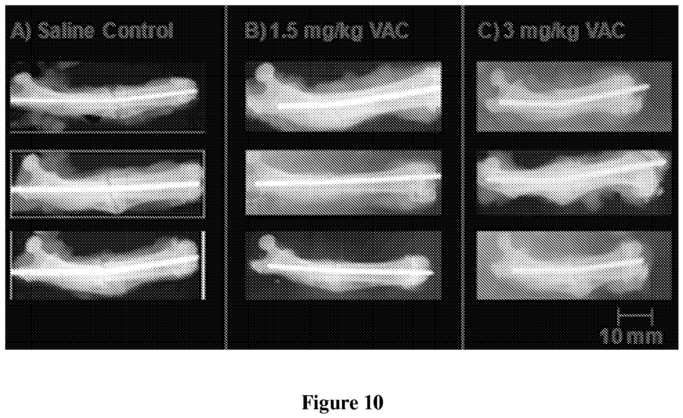

The effect of local vanadium therapy on healing of femur fractures was measured by torsional mechanical testing. At four weeks post-fracture, rats treated with vanadium displayed improved mechanical properties of the fractured femora compared to the untreated group. The maximum torque to failure, torsional rigidity, maximum effective shear stress, and effective shear modulus were all significantly increased compared to the untreated group (p < 0.05: 1.5mg/kg VAC, p < 0.05: 3 mg/kg VAC) (Table 10). Radiographs taken at four weeks post-fracture support these mechanical testing results (Figure 10). When the mechanical parameters of the fractured femora were normalized to the intact, contralateral femora, percent maximum torque to failure, percent torsional rigidity, and percent effective shear modulus were still significantly greater in the local vanadium treated groups when compared to the saline control group (p < 0.05: 1.5 mg/kg VAC, p < 0.05: 3 mg/kg VAC). By five weeks post-fracture the maximum torque to failure and torsional rigidity were significantly greater in the 1.5 mg/kg VAC treated group compared to both control and 3 mg/kg VAC groups respectively (p < 0.05) (Table 11).

**Table 10.** Post-fracture mechanical testing of vanadium (VAC) in normal rats at 4-weeks

|  | Maximum Torque to failure (Nmm) | Maximum Torsional Rigidity (Nmm$^2$/rad) | Effective Shear Modulus (MPa) | Effective Shear Stress (MPa) |
|---|---|---|---|---|
| Control (n=6) | 161 ± 48 | 9,889 ± 4,719 | 258 ± 108 | 17 ± 4 |
| 0.25 mg/kg VAC (n=6) | 227 ± 64 | 28,218 ± 9,107 | 878 ± 416 | 25 ± 9 |
| 0.5 mg/kg VAC (n=6) | 362 ± 49 *,# | 45,877 ± 13,079* | 1,107 ± 441 | 32 ± 13 |

(continued)

| | Maximum Torque to failure (Nmm) | Maximum Torsional Rigidity (Nmm$^2$/rad) | Effective Shear Modulus (MPa) | Effective Shear Stress (MPa) |
|---|---|---|---|---|
| 1.5 mg/kg VAC (n=6) | 329 ± 117 * | 34,526 ± 16,851 * | 2,454 ± 2,370 * | 69 ± 59 * |
| 3.0 mg/kg VAC (n=5) | 409 ± 43 *,# | 41,007 ± 11,236 * | 2,948 ± 1,218 * | 101 ± 18 *,#,& |
| Fractured femur values normalized to the contralateral (intact) femur | | | | |
| | Percent maximum torque to failure | Percent maximum torsional rigidity | Percent Effective Shear Modulus | Percent Effective Shear Stress |
| Control (n=6) | 27 ± 18 | 20 ± 10 | 4 ± 2 | 10 ± 5 |
| 0.25 mg/kg VAC (n=6) | 49 ± 14 | 67 ± 21 * | 14 ± 4 | 10 ± 3 |
| 0.5 mg/kg VAC (n=6) | 72 ± 19 * | 103 ± 23 *,# | 16 ± 7 | 20 ± 11 |
| 1.5 mg/kg VAC (n=6) | 59 ± 28 | 76 ± 28 * | 23 ± 12 * | 26 ± 16 |
| 3.0 mg/kg (n=5) | 79 ± 12 * | 78 ± 10 * | 20 ± 11 * | 30 ± 12 *,# |
| The data represents average values ± standard deviation<br>* Represent values statistically higher than control, p < 0.05 versus control.<br># Represent values statistically higher than Extra Low Dose, p < 0.05 versus Extra Low Dose.<br>& Represent values statistically higher than Lowered Low Dose, p < 0.05 versus Lowered Low Dose. | | | | |

**Table 11.** Post-fracture mechanical testing of VAC in normal rats at 5 weeks

| Fractured femur values | | | | |
|---|---|---|---|---|
| | Maximum Torque to failure (Nmm) | Maximum Torsional Rigidity (Nmm$^2$/rad) | Effective Shear Modulus (MPa) | Effective Shear Stress (MPa) |
| Control (n=6) | 295 ± 164 | 20,111 ± 10,944 | 1,060 ± 693 | 45 ± 28 |
| 1.5 mg/kg VAC (n=9) | 471 ± 91 *,# | 34,522 ± 8,347 * | 2,026 ± 924 | 75 ± 26 |
| 3.0 mg/kg VAC (n=8) | 335 ± 89 | 37,496 ± 12,846* | 1,453 ± 683 | 43 ± 25 |
| Fractured femur values normalized to the contralateral (intact) femur | | | | |
| | Percent maximum torque to failure | Percent maximum torsional rigidity | Percent Effective Shear Modulus | Percent Effective Shear Stress |
| Control (n=6) | 74 ± 42 | 80 ± 57 | 28 ± 29 | 31 ± 21 |

(continued)

| Fractured femur values normalized to the contralateral (intact) femur | | | | |
|---|---|---|---|---|
| | Percent maximum torque to failure | Percent maximum torsional rigidity | Percent Effective Shear Modulus | Percent Effective Shear Stress |
| 1.5 mg/kg VAC (n=9) | 99 ± 17 | 103 ± 33 | 39 ± 26 | 47 ± 25 |
| 3.0 mg/kg VAC (n=8) | 64 ± 26 | 98 ± 28 | 23 ± 9 | 22 ± 11 |
| The data represents average values ± standard deviation<br>* Represent values statistically higher than control, p < 0.05 versus control.<br># Represent values statistically higher than high dose, p < 0.05 versus high dose. | | | | |

*Local VAC without a Carrier in Diabetic Model*

The effect of local vanadium therapy on healing of diabetic femur fractures was measured by torsional mechanical testing. Blood glucose was monitored biweekly for type I diabetic BB Wistar rats and subcutaneous Linplants TM (Linshin, Canada) were administered to all diabetic animals, roughly every two weeks to maintain systemic glucose levels. At six weeks post-fracture, diabetic rats treated with vanadium displayed significantly improved mechanical properties of the fractured femora compared to the untreated diabetic group. The maximum torque to failure, torsional rigidity, maximum effective shear stress, and effective shear modulus were all significantly increased compared to the untreated diabetic group (p < 0.05: 1.5mg/kg VAC) (Table 12). When the mechanical parameters of the fractured femora were normalized to the intact, contralateral femora, percent maximum torque to failure, percent torsional rigidity, percent effective shear stress, and percent effective shear modulus were still significantly greater in the local vanadium treated diabetic groups when compared to the untreated diabetic group (p < 0.05: 1.5mg/kg VAC). Torsional mechanical testing parameters for the VAC treated diabetic animals were comparable to the non-diabetic animals at six weeks.

**Table 12.** Post-fracture mechanical testing of non-diabetic, diabetic and diabetic rats treated with VAC at 6-weeks

| | Maximum Torque to failure (Nmm) | Maximum Torsional Rigidity (Nmm$^2$/rad) | Effective Shear Stress (MPa) |
|---|---|---|---|
| diabetic control (n=23) | 154 ± 69 | 425 ± 259 | 3 ± 2 |
| 1.5mg/kg VAC in diabetic (n=3) | 410 ± 71 * | 43,089 ± 19,720 * | 98 ± 53 *,# |
| normal (n=12) | 456 ± 66 * | 33,784 ± 11,849 * | 48 ± 16 * |
| Normalized to contralateral femur | Percent maximum torque to failure | Percent maximum torsional rigidity | Percent Effective Shear Stress |
| diabetic control (n=23) | 27 ± 10 | 27 ± 15 | 8 ± 4 |
| 1.5mg/kg VAC in diabetic (n=3) | 85 ± 23 * | 136 ± 111 * | 33 ± 20 * |
| normal (n=12) | 78 ± 15 * | 86 ± 29 | 28 ± 13 * |
| The data represents average values ± standard deviation<br>* Represent values statistically higher than control, p < 0.05 versus control.<br># Represent values statistically higher than normal, *p* < 0.05 versus normal.<br>The value of the numbers for diabetic control is obtained from two papers of Gandhi (Insulin: Bone **2005;** PRP:Bone **2006** and Beam et al **2002** JOR). The value of 6-week normal group is an average of Gandhi's paper and the investigator's 6 week mechanical test saline animals. | | | |

*Local VAC /CaSO4 Formulations*

When local vanadium with a calcium sulfate carrier was torsionally tested, results revealed significantly higher effective shear stress (p < 0.05) for the 0.25 mg/kg VAC with calcium sulfate carrier group, compared to both the calcium sulfate buffer and 1.5 mg/kg VAC with calcium sulfate carrier groups. When the mechanical parameters of the fractured femora were normalized to the intact, contralateral femora, maximum torque to failure, and percent effective shear modulus were significantly greater in the 0.25 mg/kg VAC with calcium sulfate carrier group, compared to the calcium sulfate buffer group (p < 0.05) (Table 13).

**Table 13.** Post-fracture mechanical testing of VAC/ $CaSO_4$ in normal rats at 4-weeks

| | Maximum Torque to failure (Nmm) | Maximum Torsional Rigidity (Nmm$^2$/rad) | Effective Shear Modulus (MPa) | Effective Shear Stress (MPa) |
|---|---|---|---|---|
| Control (n=6) | 161 $\pm$ 48 | 9,889 $\pm$ 4,719 | 258 $\pm$ 108 | 17 $\pm$ 4 |
| $CaSO_4$ Buffer (n=9) | 241 $\pm$ 172 | 25,684 $\pm$ 20,795 | 680 $\pm$ 623 | 23 $\pm$ 16 |
| **0.25 mg/kg VAC and $CaSO_4$ Carrier (n=6)** | **430 $\pm$ 133 *** | **31,138 $\pm$ 11,518** | **1,178 $\pm$ 484 *** | **55 $\pm$ 21 *,#,&** |
| 1.5 mg/kg VAC and $CaSO_4$ Carrier (n=5) | 322 $\pm$ 157 | 26,302 $\pm$ 17,974 | 637 $\pm$ 395 | 29 $\pm$ 15 |
| **Fractured femur values normalized to the contralateral (intact) femur** | | | | |
| | Percent maximum torque to failure | Percent maximum torsional rigidity | Percent Effective Shear Modulus | Percent Effective Shear Stress |
| Control (n=6) | 27 $\pm$ 18 | 20 $\pm$ 10 | 4 $\pm$ 2 | 10 $\pm$ 5 |
| $CaSO_4$ Buffer (n=9) | 37 $\pm$ 30 | 47 $\pm$ 47 | 7 $\pm$ 7 | 9 $\pm$ 6 |
| **0.25 mg/kg VAC and $CaSO_4$ Carrier (n=6)** | **85 $\pm$ 24 *,#** | **100 $\pm$ 49 *** | **24 $\pm$ 10 *,#** | **18 $\pm$ 9** |
| 1.5 mg/kg VAC and $CaSO_4$ Carrier (n=5) | 64 $\pm$ 30 | 69 $\pm$ 47 | 15 $\pm$ 8 | 10 $\pm$ 7 |
| The data represents average values $\pm$ standard deviation<br>* Represent values statistically higher than control, p < 0.05 versus control.<br># Represent values statistically higher than $CaSO_4$ Buffer , p < 0.05 versus $CaSO_4$ Buffer.<br>& Represent values statistically higher than Low Dose and $CaSO_4$ Carrier, p < 0.05 versus Low Dose and $CaSO_4$ Carrier. | | | | |

*Surface Modified VAC Coated Implants*

Torsional mechanical testing of surface modified rods four weeks post-fracture demonstrated significantly greater maximum torque to failure for the animals with vanadium-boron surface modified rods compared to the group with untreated 316L stainless steel (SS) control rods (p < 0.05). When the mechanical parameters of the fractured femora were normalized to the intact, contralateral femora, the percent maximum torque to failure was significantly greater for the animals with vanadium-boron surface modified rods compared to the group with untreated 316L stainless steel control rods (p < 0.05). Although torsional mechanical parameters were higher for the vanadium-boron surface modified rods, compared to the boron surface modified control rods, no significant differences were found between these groups (Tables 14 and 15).

**Table 14.** Post-fracture mechanical testing on surface modified vanadium-borided rods in normal rats (Pilot Data in Female Rats) at 4 weeks

| Fractured femur values | | | | |
|---|---|---|---|---|
| | **Maximum Torque to failure (Nmm)** | **Maximum Torsional Rigidity (Nmm$^2$/rad)** | **Shear Modulus (MPa)** | **Maximum Shear Stress (MPa)** |
| 316L Stainless Steel Control Rod (n=5) | 178 ± 38 | 9,363 ± 5,032 | 235 ± 102 | 19 ± 3 |
| Boron Coated Control Rod (n=3) | 251 ± 93 | 19,683 ± 9,207 | 1,909 ± 1,582 | 70 ± 46 |
| **0.6 mg/kg Vanadium-Boron Coated Rod (n=4)** | **305 ± 30 \*** | **31,078 ± 6,917 \*** | **2,347 ± 1,649** | **60 ± 33** |
| Fractured femur values Normalized to the contralateral (intact) femur | | | | |
| | **Percent maximum torque to failure** | **Percent maximum torsional rigidity** | **Percent shear modulus** | **Percent maximum shear stress** |
| 316L Stainless Steel Control Rod (n=5) | 30 ± 18 | 19 ± 11 | 4 ± 2 | 11 ± 5 |
| Boron Coated Control Rod (n=3) | 68 ± 22 \* | 73 ± 36 | 23 ± 18 | 33 ± 16 |
| **0.6 mg/kg Vanadium-Boron Coated Rod (n=4)** | **76 ± 9 \*** | **107 ± 36 \*** | **38 ± 19 \*** | **40 ± 20 \*** |
| The data represents average values ± standard deviation<br>\* Represent values statistically higher than control, p < 0.05 versus 316L Stainless Steel control group. | | | | |

**Table 15.** Post-fracture mechanical testing with surface modified vanadium-borided rods in normal rats (Study in Male Rats) at 4 weeks

| | **Maximum Torque to failure (Nmm)** | **Maximum Torsional Rigidity (Nmm$^2$/rad)** | **Effective Shear Modulus (MPa)** | **Effective Shear Stress (MPa)** |
|---|---|---|---|---|
| 316L Stainless Steel Control Rod (n=6) | 161 ± 48 | 9,889 ± 4,719 | 258 ± 108 | 17 ± 4 |
| Boron Coated Control Rod (n=5) | 269 ± 102 | 22,340 ± 12,323 | 400 ± 304 | 17 ± 8 |
| **0.6 mg/kg Vanadium-Boron Coated Rod (n=5)** | **366 ± 150 \*** | **23,650 ± 11,718** | **609 ± 422** | **32 ± 15** |
| Fractured femur values normalized to the contralateral (intact) femur | | | | |
| | **Percent maximum torque to failure** | **Percent maximum torsional rigidity** | **Percent Effective Shear Modulus** | **Percent Effective Shear Stress** |
| 316L Stainless Steel Control Rod (n=6) | 27 ± 18 | 20 ± 10 | 4 ± 2 | 10 ± 5 |
| Boron Coated Control Rod (n=5) | 45 ± 20 | 51 ± 34 | 7 ± 6 | 10 ± 6 |

(continued)

| Fractured femur values normalized to the contralateral (intact) femur | | | | |
|---|---|---|---|---|
| | **Percent maximum torque to failure** | **Percent maximum torsional rigidity** | **Percent Effective Shear Modulus** | **Percent Effective Shear Stress** |
| **0.6 mg/kg Vanadium-Boron Coated Rod (n=5)** | **65 ± 25 \*** | **52 ± 25** | **9 ± 5** | **19 ± 9** |

The data represents average values ± standard deviation
\* Represent values statistically higher than control, p < 0.05 versus 316L Stainless Steel control group.

*Effect of VAC on Rats of Advanced Age*

The effect of local vanadium therapy on healing of femur fractures in normal (non-diabetic) rats was measured by torsional mechanical testing. At four weeks post-fracture, fractured femurs from the rats of advanced age (190-195 days of age) treated with VAC had greater mechanical properties than the fractured femurs from the control group. When the mechanical parameters of the fractured femora were normalized to the intact, contralateral femora, percent maximum torque to failure (saline group vs. 1.5 mg/kg VAC group $p$<0.05 was significantly greater in the local vanadium treated groups when compared to the saline group (Table 16).

Healing was assessed by radiographic examination and quantified by mechanical testing. Local VAC treatment improved radiographic appearance and significantly increased the mechanical strength of fractured femurs. At four weeks post-fracture, the average percent maximum torque to failure of the fractured femora for 1.5 mg/kg VAC was significantly 76 percent greater (44.0% of contralateral vs. 25.0%), compared to the untreated saline group (Table 16). The data indicate that local VAC treatment enhanced bone regeneration during fracture healing even in a population of advanced age.

**Table 16.** Four weeks post-fracture mechanical testing with local vanadium (VAC) in rats of advanced age (Age: 190-195 days)

| Fractured Femur Values | | | | | |
|---|---|---|---|---|---|
| | Maximum Torque to Failure (Nmm) | Maximum Torsional Rigidity (Nmm$^2$/rad) | Effective Shear Modulus (MPa) | Effective Shear Stress (MPa) | Mean Angle at Failure (degrees) |
| Saline Control (n=3) | 220 ± 76 | $4.2\times10^4 \pm 1.710^4$ | $2.2\times10^3 \pm 1.5\times10^3$ | 36 ± 5 | 6 ± 3 |
| 1.5 mg/kg VAC (n=4) | 324 ± 83 | $3.0\times10^4 \pm 1.8\times10^4$ | $1.3\times10^3 \pm 1.3\times10^3$ | 43 ± 17 | 12 ± 4 |
| Fractured Femur Values Normalized to the Contralateral (Intact) Femur | | | | | |
| | Percent Maximum Torque to Failure | Percent maximum Torsional Rigidity | Percent Effective Shear Modulus | Percent Effective Shear Stress | ---------- |
| Saline Control (n=3) | 25 ± 7 | 62 ± 19 | 24 ± 10 | 15 ± 7 | NA |

(continued)

| Fractured Femur Values Normalized to the Contralateral (Intact) Femur | | | | | |
|---|---|---|---|---|---|
| | Percent Maximum Torque to Failure | Percent maximum Torsional Rigidity | Percent Effective Shear Modulus | Percent Effective Shear Stress | ---------- |
| 1.5 mg/kg VAC (n=4) | 44 ± 10* | 56 ± 37 | 16 ± 18 | 16 ± 4 | NA |
| The data represents average values ± standard deviation<br>* Represents values statistically higher than saline control, p < 0.05 versus saline control. Student t-test between 2 groups | | | | | |

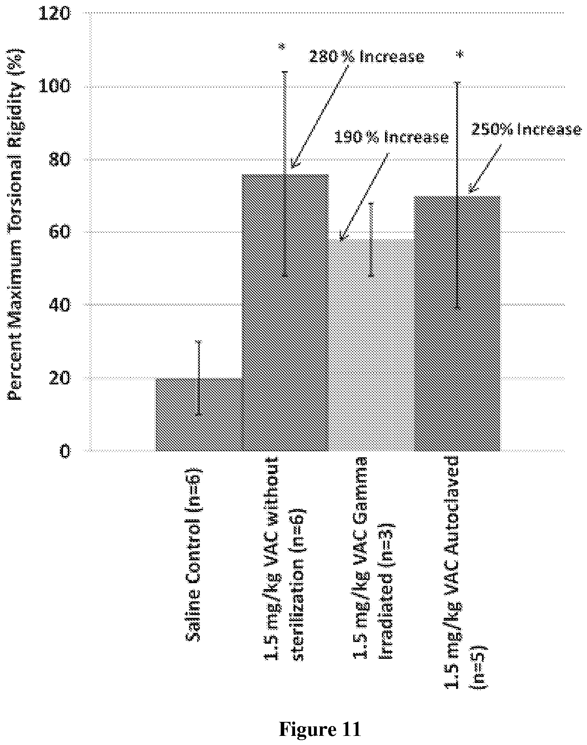

The effect of local vanadium therapy on healing of femur fractures in normal (non-diabetic) rats was measured by torsional mechanical testing. At four weeks post-fracture, fractured femurs from the rats treated with VAC had greater mechanical properties than the fractured femurs from the control group, even if the VAC solution was autoclaved or gamma irradiated prior to administration (Figure 11, Table 17). For the 1.5 mg/kg VAC group without sterilization, the maximum torque to failure (saline group vs. 1.5 mg/kg VAC group without sterilization $p<0.05$) and torsional rigidity (saline group vs. 1.5 mg/kg VAC group without sterilization $p<0.05$) were significantly greater than the saline control group. For the 1.5 mg/kg VAC autoclaved VAC group, the torsional rigidity (saline group vs. 1.5 mg/kg autoclaved VAC group $p<0.05$) was significantly greater than the saline control group (Table 17). When the mechanical parameters of the fractured femora were normalized to the intact, contralateral femora, percent torsional rigidity (saline group vs. 1.5 mg/kg VAC group without sterilization $p<0.05$, saline group vs. 1.5 mg/kg autoclaved VAC group $p<0.05$), and shear modulus (saline group vs. 1.5 mg/kg VAC group without sterilization $p<0.05$) were significantly greater in the local vanadium treated groups when compared to the saline group (Table 17).

**Table 17** Four weeks post-fracture mechanical testing with local vanadium (VAC) without a Carrier

| | Maximum Torque to failure (Nmm) | Maximum Torsional Rigidity (Nmm$^2$/rad) | Effective Shear Modulus (MPa) | Effective Shear Stress (MPa) |
|---|---|---|---|---|
| Saline Control (n=6) | 161 ± 48 | $9.9\times10^3 \pm 4.7\times10^3$ | $2.6\times10^2 \pm 1.1\times10^2$ | 17 ± 4 |
| 1.5 mg/kg VAC without sterilization (n=6) | 329 ± 117 * | $3.5\times10^4 \pm 1.7\times10^{4*}$ | $2.5\times10^3 \pm 2.4\times10^3$ | 69 ± 59 |
| 1.5 mg/kg VAC Gamma Irradiated (n=3) | 276 ± 79 | $2.6\times10^4 \pm 3.7\times10^3$ | $7.9\times10^2 \pm 2.4\times10^2$ | 28 ± 4 |
| 1.5 mg/kg VAC Autoclaved (n=5) | 292 ± 83 | $3.4\times10^4 \pm 1.5\times10^{4*}$ | $9.6\times10^2 \pm 7.8\times10^2$ | 26 ± 16 |
| Fractured Femur Values Normalized to the Contralateral (Intact) Femur | | | | |
| | Percent maximum torque to failure | Percent maximum torsional rigidity | Percent Effective Shear Modulus | Percent Effective Shear Stress |
| Saline Control (n=6) | 27 ± 18 | 20 ± 10 | 4 ± 2 | 10 ± 5 |
| 1.5 mg/kg VAC without sterilization (n=6) | 59 ± 28 | 76 ± 28 * | 23 ± 12 * | 26 ± 16 |
| 1.5 mg/kg VAC Gamma Irradiated (n=3) | 50 ± 18 | 58 ± 10 | 9 ± 2 | 14 ± 5 |
| 1.5 mg/kg VAC Autoclaved (n=5) | 47 ± 12 | 70 ± 31 * | 15 ± 10 | 14 ± 7 |
| The data represents average values ± standard deviation<br>* Represents values statistically higher than saline control, p < 0.05 versus saline control. One-way ANOVA between 4 groups with Holm-Sidak post-hoc analysis. | | | | |

Healing was assessed by radiographic examination and quantified by mechanical testing. Local VAC treatment improved radiographic appearance and significantly increased the mechanical strength of fractured femurs. At four weeks post-fracture, the average percent maximum torsional rigidity values for 1.5 mg/kg VAC without sterilization and following the autoclave process were significantly greater, with non-sterile VAC; 2.8 times greater (76.0% of contralateral vs. 20.0%), and autoclaved VAC; 2.5 times greater (70.0% of contralateral vs. 20.0%) compared to the saline control group. Percent shear modulus values for 1.5 mg/kg VAC without sterilization was significantly greater; 4.8 times greater (23.0% of contralateral vs. 4.0%) compared to the saline control group. The data indicate that local VAC treatment enhanced bone regeneration during fracture healing and indicates that effective sterilization techniques that may affect the stability and bioactivity of proteins, do not significantly alter the bioactivity of VAC.

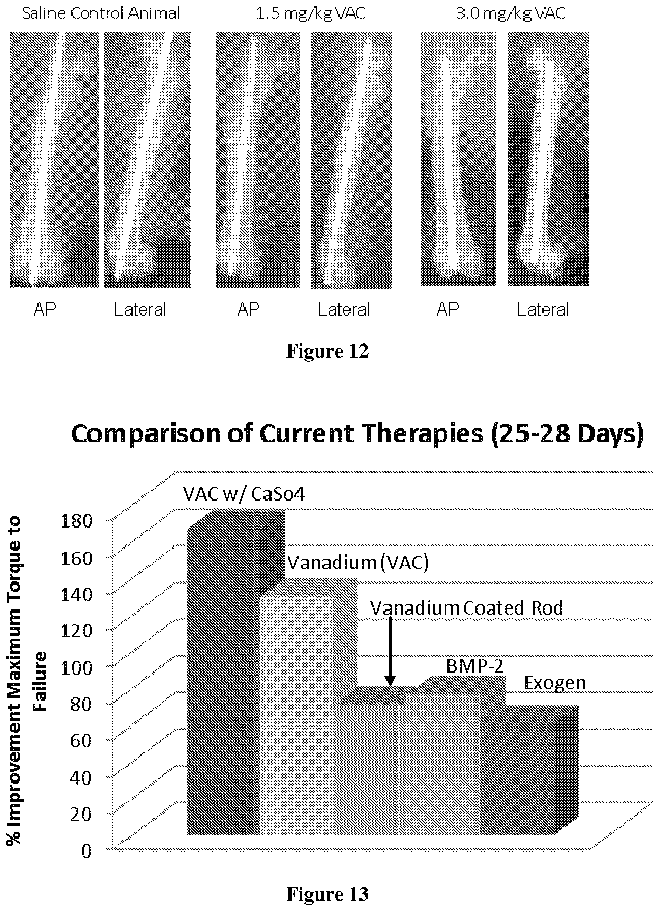

The effect of local vanadium therapy on healing of femur fractures in normal (non-diabetic) rats was measured by radiographic analysis. At twelve weeks post-fracture, fractured femurs from the rats treated with both low (1.5 mg/kg) and high (3.0 mg/kg) VAC had no evidence of ectopic bone formation, following resolution of the induced fracture (Figure 12). Femora treated with local VAC demonstrated normal remodeling suggesting no evidence of a toxic/carcinogenic effect of VAC throughout fracture healing. The above data have demonstrated an effective VAC therapeutic dosage range 0.5 to 3.0 mg/Kg, which resulted in two to three-fold increase in mechanical parameter of torsion.

*Comparison with Existing Therapies*

The chart in Figure 13 compares the vanadium technologies with the currently approved products (BMP-2 and Exogen) for fracture healing. Each of these studies examined the effectiveness of a therapeutic adjunct on femur fracture healing by measuring the maximum torque to failure at the same timepoint (four weeks). Specifically the following were compared: (1) a single percutaneous dose (0.25 mg/kg) of VAC with the calcium sulfate ($CaSO_4$)vehicle (red); (2) a single percutaneous dose (1.5 mg/kg) of VAC without a vehicle (blue); (3) a 316L stainless steel k-wire surface modified with vanadium (a process called vanadium pack-boriding), implanted into the intramedullary canal of the femur (green); (4) BMP-2 study used a single percutaneous dose of BMP-2 (80 $\mu$g) with buffer vehicle (orange); and (5) Exogen study used varying exposure periods of ultrasound treatment (20 mins /day). The most effective duration (25 days) is shown in dark blue.

Thus, the results have demonstrated, among others, (a) that the use of vanadium compounds (such as VAC) alone or as part of a formulation with an orthopedic carrier (CaSO4 for example) that is applied directly to the site of fracture; and (b) that the use of orthopedic implants (pedicle screws, plates, rods, wires, etc..) where the surface has been modified with vanadium via known thermal processing techniques. As an insulin-mimetic adjunct, vanadium compounds can be used to accelerate bone regeneration by stimulating insulin signaling at the fracture site. Local VAC targets the beta-subunit of the insulin signaling receptor. The presence of the insulin mimetic also enhances cartilage and mineralized tissue formation. Our laboratory data demonstrated that VAC treatment significantly increases cell proliferation within the subperiosteal region of the fracture callus (seven and ten days post-fracture). This translates into significantly higher percent cartilage within the fracture callus, (seven and ten days post-fracture). The percent mineralized tissue for local VAC treated rat animal models was significantly higher than controls after 21 days. This accelerated progression of the bone healing process results in significantly enhanced mechanical testing parameters for VAC treated animals after four and five weeks, compared to controls.

EXAMPLE 2

**Insulin Mimetics Enhancing Spinal Fusion**

Increased fusion rates were observed in a rat posterolateral lumbar spinal fusion model when treated with a time-released insulin implant in comparison with controls. The effects of insulin-mimetic agents were analyzed as an adjunct to spinal fusion in the rat posterolateral lumbar fusion model. Vanadyl acetylacetonate (VAC) or Zinc was made into a pellet with Calcium Sulfate, and applied to the fusion bed with autograft in a rat posterolataeral lumbar fusion. These results were compared with a control group treated with autograft and a palmitic acid pellet.

*Study Design*

The protocol was approved by the animal Institutional Care and Use Committee at UMDNJ-New Jersey Medical School. Fifty skeletally mature Sprague-Dawley rats weighing approximately 500 grams each underwent posterolateral intertransverse lumbar fusions with iliac crest autograft from L4-L5 utilizing a Wiltse-type approach. After exposure of the transverse processes and high-speed burr decortication, one of five pellets were added to the fusion site: a low dose Vanadium Calcium Sulfate pellet (0.75 mg/kg), a high dose Vanadium Calcium Sulfate pellet (1.5 mg/kg), a low dose Zinc Calcium Sulfate pellet (0.5 mg/kg), a high dose Zinc Calcium Sulfate pellet (1.0 mg/kg), and a control of micro-recrystalized palmitic acid pellet. An equal amount of iliac crest autograft (approximately 0.3g per side) was harvested and implanted with each pellet. The rats were sacrificed at eight weeks, and spines were harvested, removed of soft tissue, and tested by manual palpation, radiographs and MicroCT. All outcome parameters were independently reviewed by two separate individuals in a blinded manner and the lower grade of fusion was accepted when there was a discrepancy.

*Surgical Procedure*

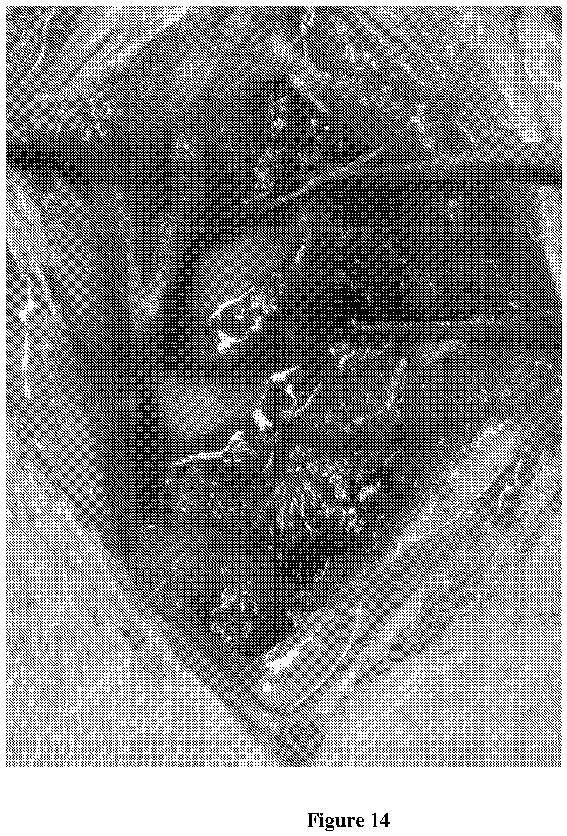

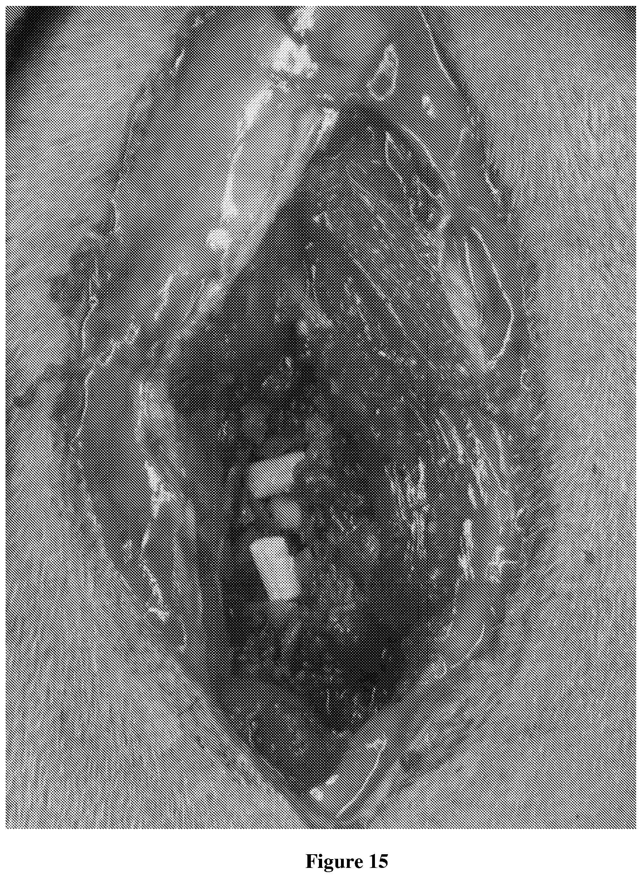

After obtaining general anaesthesia with intraperitoneal Ketamine (40mg/kg) and Xylazine (5 mg/kg), the lumbar region of the rat was shaved and cleansed with povidone iodine soaked gauze. A dorsal midline incision was made from L3 to the sacrum. Two paramedian incisions were made through the lumbar fascia 5mm from the midline. Dissection was taken to the iliac crest, and approximately 0.3g of bone was harvested with small rongeurs. The harvested autograft was measured on a sterile scale in order to obtain 0.3g per side. Blunt dissection was carried down posterolaterally, reflecting the paraspinal muscles lateral to the facet joints on each side. The reflected paraspinal muscles were held in place with retractors. The transverse processes of L4-L5 were cleaned of soft tissue, and decorticated with a high-speed burr (Figure 14). The crushed autograft was then spread over and between the transverse processes at the appropriate level (L4-L5). An equivalent amount of implant, or blank was incorporated into the autograft bed (Figure 15). Retractors were removed and the paraspinal muscles were allowed to cover the fusion bed. The dorsal lumbar fascia was closed using a running 4-0 resorbable suture and the skin was closed with interrupted 4-0 resorbable sutures. The surgical site was treated with antibiotic ointment, and the rats were given a dose of Enrofloxacin antibiotic (10 mg/kg). Radiographs were taken immediately after surgery. Blood glucose levels were taken before surgery, and 12 and 24 hours after surgery. See Table 18.

**Table 18.** Systemic blood glucose levels (mg/dL)

| Group | Before surgery | 12 hours | 24 hours |
|---|---|---|---|
| Controls | | | 91.4 |
| VAC-low | 103.5 | 213.4 | 117.7 |
| VAC-high | 102.9 | 153.2 | 90.7 |
| Zn-low | 106.0 | 122.8 | 101.8 |
| Zn-high | 109.3 | 120.0 | 89.0 |

*Pellet Preparation*

In order to prepare the pellets, 0.2 mL of each stock solution will be mixed with 0.4 g of $CaSO_4$ to obtain the appropriate consistency of the carrier in a 1 mL syringe. It will then be injected into 2mm diameter clear Tygon laboratory tubing and allowed to harden overnight.

Once set, pellets will be sectioned into 7mm pieces and autoclaved (to sterilize), prior to implantation.

Assumption: Weight of SD rat = 0.45 kg

| | Vn (0.75mg/kg) | Vn (1.5 mg/kg) | Zn (0.5 mg/kg) | Zn (1.0 mg/kg) |
|---|---|---|---|---|
| Mass of treatment for each rat | 0.338 mg | 0.675 mg | 0.225 mg | 0.45 mg |

In order to prepare the stock solution, the volume of solution in each pellet will be calculated by using the volume ratio of solution to mixture.

Volume of $CaSO_4$ in each mixture

- 
$$D\ CaSO_4 = 2.96\ g/cm^3$$

- 
$$(0.4g\_CaSO_4)/(2.96\frac{g}{cm^3}) = 0.135 cm^3 = 0.135 mL$$

Volume of mixture and ratio

- $$0.135 \text{ mL CaSO}_4 + 0.2 \text{ mL solution} = 0.335 \text{ mL mixture}$$

- $$0.2 \text{ mL solution} / 0.335 \text{ mL mixture x } 100\% = 59.7\% \text{ solution per mixture}$$

Volume of each pellet, 1 mm radius, 7 mm height

- $$V = \pi r^2 h \; , \; = \pi (1mm)^2 (7mm) \; = \; 22mm^3 = 0.022mL$$

Volume of solution in each pellet

- $$0.022 \text{ mL x } 59.7\% = 0.0131 \text{ mL solution per pellet}$$

Stock Solution (10 mL)
- Because bilateral surgery is performed, mass of treatment (X) must be halved for each pellet.
-

$$\left( \left( \frac{X}{2} \right) / 0.0131mL \right) \times 10$$

|  | Vn (0.75mg/kg) | Vn (1.5 mg/kg) | Zn (0.5 mg/kg) | Zn (1.0 mg/kg) |
|---|---|---|---|---|
| Mass of treatment in each stock solution (10 ml) | 129.0mg | 258.0 mg | 85.9 mg | 171.8 mg |

*Radiographic Analysis*

Posteroanterior radiographs at 35 kV for 90 seconds were taken at eight weeks after sacrifice and harvest. All soft tissue was removed prior to radiographic exam. Two blinded independent observers graded the radiographs as solid fusion mass bilaterally (A), unilateral fusion mass (B), small fusion mass bilaterally (C), and graft resorption (D), based on previously published radiographic scales. See Table 19.

**Table 19.** Radiographs

| Group | A | B | C | D | Kappa | P Value |
|---|---|---|---|---|---|---|
| **Controls (n=9)** | 2 | 3 | 1 | 3 | 0.297 | |
| **VAC-low (n=10)** | 3 | 3 | 0 | 4 | 0.583 | 0.807 |
| **VAC-high (n=10)** | 5 | 3 | 1 | 1 | 0.667 | 0.270 |
| **Zn-low (n=10)** | 7 | 1 | 2 | 0 | 0.512 | 0.066 |
| **Zn-high (n=10)** | 7 | 3 | 0 | 0 | 1.0 | 0.050 |
| A= solid fusion mass bilaterally<br>B=unilateral fusion mass<br>C=small fusion mass bilaterally<br>D= Graft resorption | | | | | | |

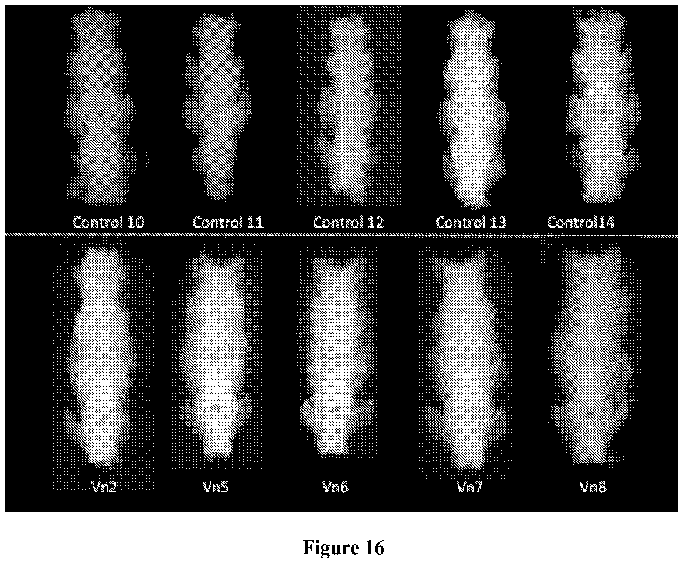

Based on radiographs (Figure 16), in the high dose vanadium group 5/10 had solid fusion mass bilaterally, 3/10 had unilateral fusion, 1/10 had small fusion mass bilaterally, and 1/10 had graft resorption. The low dose vanadium group had 3/10 solid fusion mass bilaterally, 3/10 had unilateral fusion, 0/10 had small fusion mass bilaterally, and 4/10 had graft resorption. The high dose zinc group had 7/10 solid fusion mass bilaterally, 3/10 had unilateral fusion, 0/10 had small fusion mass bilaterally, and 0/10 had graft resorption. The low dose zinc group had 7/10 solid fusion mass bilaterally, 1/10 had unilateral fusion, 2/10 had small fusion mass bilaterally, and 0/10 had graft resorption. The control group had 2/9 solid fusion mass bilaterally, 3/9 unilateral fusion, 1 small fusion mass bilaterally, and 3/9 had graft resorption.

*Manual Palpation*

After removal of all soft tissue, two blinded independent observers manually palpated and stressed across the fusion site (L4-L5). Specimens were graded as fused (A), partially fused (B), and not fused (C). See Table 20.

**Table 20.** Manual palpation

| Group | A | B | C | Kappa | P Value |
|---|---|---|---|---|---|
| **Controls (n=9)** | 0 | 1 | 8 | 0.156 | |
| **VAC-low (n=10)** | 1 | 4 | 5 | 0.130 | 0.072 |
| **VAC-high (n=10)** | 6 | 2 | 2 | 0.412 | 0.002 |
| **Zn-low (n=10)** | 3 | 4 | 3 | 0.565 | 0.055 |
| **Zn-high (n=10)** | 4 | 1 | 5 | 0.306 | 0.008 |
| A=fused<br>B=partially fused<br>C=not fused | | | | | |

Based on manual palpation in the high dose Vanadium group 6/10 had solid fusion, 2/10 were partially fused, and 2/10 were not fused. In the low dose vanadium group, 1/10 had solid fusion, 4/10 were partially fused, and 5/10 were not fused. In the high dose Zinc group, 4/10 had solid fusion, 1/10 had partially fused, and 5/10 were not fused. In the low dose Zinc group, 3/10 had solid fusion, 4/10 had partially fused, and 3/10 were not fused. In the control group, 0/9 had solid fusion, 1/9 had partially fused, and 8/9 were not fused.

*Micro CTAnalysis*

**Table 21.** MicroCT

| Group | Mean Bone Volume mm$^3$ | Std Dev | P value |
|---|---|---|---|
| Table 21a | | | ANOVA p=0.006 |
| Vn high dose (n=10) | 170.8 | 37.1 | <0.01 vs control |
| Vn low dose (n=10) | 167.4 | 23.5 | <0.05 vs control |
| Controls (n=9) | 126.7 | 26.3 | |
| | | | |
| Table 21b | | | ANOVA p=0.002 |
| Zn high dose (n=10) | 172.7 | 26.4 | <0.01 vs control |
| Zn low dose (n=10) | 172.9 | 31.6 | <0.01 vs control |
| Controls (n=9) | 126.7 | 26.3 | |

Based on MicroCT analysis, the mean bone volume of the L4/L5 transverse processes and fusion mass for controls was 126.7mm$^3$. In the high dose Vanadium group there was 170.8mm$^3$, and in the low dose Vanadium group there was 167.4mm$^3$. The high dose Zinc group had a mean of 172.7mm$^3$, and the low dose Zinc group had a mean of 172.9mm$^3$. Differences between each experimental group versus controls were significant (see Table 21).

*Statistical Analysis*

A Mann-Whitney Rank Test was performed for analysis of radiographs and manual palpation. Kappa values were calculated for inter-rater agreement. ANOVA was performed for amt of new bone formation as per micro CT with secondary test using Holm Sidak test. Statistical analysis was performed using SigmaStat.

Of the 50 animals, one of the control rats died on postoperative day one, likely due to anaesthesia. The remaining 49 rats had no complications and were sacrificed as planned (0.02% perioperative mortality rate).

significantly increased the mechanical strength of fractured femurs. At four weeks post-fracture, the average percent maximum torque to failure of the fractured femora for 1.5 mg/kg VAC was significantly 76 percent greater (44.0% of contralateral vs. 25.0%), compared to the untreated saline group (Table 16). The data indicate that local VAC treatment enhanced bone regeneration during fracture healing even in a population of advanced age.

**Table 16.** Four weeks post-fracture mechanical testing with local vanadium (VAC) in <u>rats of advanced age (Age: 190-195 days)</u>

| Fractured Femur Values | | | | | |
|---|---|---|---|---|---|
| | Maximum Torque to Failure (Nmm) | Maximum Torsional Rigidity (Nmm$^2$/rad) | Effective Shear Modulus (MPa) | Effective Shear Stress (MPa) | Mean Angle at Failure (degrees) |
| Saline Control (n=3) | $220 \pm 76$ | $4.2 \times 10^4 \pm 1.7 \times 10^4$ | $2.2 \times 10^3 \pm 1.5 \times 10^3$ | $36 \pm 5$ | $6 \pm 3$ |
| 1.5 mg/kg VAC (n=4) | $324 \pm 83$ | $3.0 \times 10^4 \pm 1.8 \times 10^4$ | $1.3 \times 10^3 \pm 1.3 \times 10^3$ | $43 \pm 17$ | $12 \pm 4$ |
| Fractured Femur Values Normalized to the Contralateral (Intact) Femur | | | | | |
| | Percent Maximum Torque to Failure | Percent maximum Torsional Rigidity | Percent Effective Shear Modulus | Percent Effective Shear Stress | -------- |
| Saline Control (n=3) | $25 \pm 7$ | $62 \pm 19$ | $24 \pm 10$ | $15 \pm 7$ | NA |
| 1.5 mg/kg VAC (n=4) | $44 \pm 10$* | $56 \pm 37$ | $16 \pm 18$ | $16 \pm 4$ | NA |
| The data represents average values $\pm$ standard deviation<br>* Represents values statistically higher than saline control, p < 0.05 versus saline control.<br>Student t-test between 2 groups | | | | | |

The effect of local vanadium therapy on healing of femur fractures in normal (non-diabetic) rats was measured by torsional mechanical testing. At four weeks post-fracture, fractured femurs from the rats treated with VAC had greater mechanical properties than the fractured femurs from the control group, even if the VAC solution was autoclaved or gamma irradiated prior to administration (Figure 11, Table 17) . For the 1.5 mg/kg VAC group without sterilization, the maximum torque to failure (saline group vs. 1.5 mg/kg VAC group without sterilization $p<0.05$) and torsional rigidity (saline group vs. 1.5 mg/kg VAC group without sterilization $p<0.05$) were significantly greater than the saline control group. For the 1.5 mg/kg VAC autoclaved VAC group, the torsional rigidity (saline group vs. 1.5 mg/kg autoclaved VAC group $p<0.05$) was significantly greater than the saline control group (Table 17). When the mechanical parameters of the fractured femora were normalized to the intact, contralateral femora, percent torsional rigidity (saline group vs. 1.5 mg/kg VAC group without sterilization p<0.05, saline group vs. 1.5 mg/kg autoclaved VAC group $p<0.05$), and shear modulus (saline group vs. 1.5 mg/kg VAC group without sterilization $p<0.05$) were significantly greater in the local vanadium treated groups when compared to the saline group (Table 17).

**Table 17** Four weeks post-fracture mechanical testing with local vanadium (VAC) without a Carrier

| | Maximum Torque to failure (Nmm) | Maximum Torsional Rigidity (Nmm$^2$/rad) | Effective Shear Modulus (MPa) | Effective Shear Stress (MPa) |
|---|---|---|---|---|
| Saline Control (n=6) | 161 ± 48 | $9.9\times10^3 \pm 4.7\times10^3$ | $2.6\times10^2 \pm 1.1\times10^2$ | 17 ± 4 |
| 1.5 mg/kg VAC without sterilization (n=6) | 329 ± 117 * | $3.5\times10^4 \pm 1.7\times10^{4*}$ | $2.5\times10^3 \pm 2.4\times10^3$ | 69 ± 59 |
| 1.5 mg/kg VAC Gamma Irradiated (n=3) | 276 ± 79 | $2.6\times10^4 \pm 3.7\times10^3$ | $7.9\times10^2 \pm 2.4\times10^2$ | 28 ± 4 |
| 1.5 mg/kg VAC Autoclaved (n=5) | 292 ± 83 | $3.4\times10^4 \pm 1.5\times10^{4*}$ | $9.6\times10^2 \pm 7.8\times10^2$ | 26 ± 16 |
| Fractured Femur Values Normalized to the Contralateral (Intact) Femur | | | | |
| | Percent maximum torque to failure | Percent maximum torsional rigidity | Percent Effective Shear Modulus | Percent Effective Shear Stress |
| Saline Control (n=6) | 27 ± 18 | 20 ± 10 | 4 ± 2 | 10 ± 5 |
| 1.5 mg/kg VAC without sterilization (n=6) | 59 ± 28 | 76 ± 28* | 23 ± 12* | 26 ± 16 |
| 1.5 mg/kg VAC Gamma Irradiated (n=3) | 50 ± 18 | 58 ± 10 | 9 ± 2 | 14 ± 5 |
| 1.5 mg/kg VAC Autoclaved (n=5) | 47 ± 12 | 70 ± 31* | 15 ± 10 | 14 ± 7 |
| The data represents average values ± standard deviation<br>* Represents values statistically higher than saline control, p < 0.05 versus saline control.<br>One-way ANOVA between 4 groups with Holm-Sidak post-hoc analysis. | | | | |

Healing was assessed by radiographic examination and quantified by mechanical testing. Local VAC treatment improved radiographic appearance and significantly increased the mechanical strength of fractured femurs. At four weeks post-fracture, the average percent maximum torsional rigidity values for 1.5 mg/kg VAC without sterilization and following the autoclave process were significantly greater, with non-sterile VAC; 2.8 times greater (76.0% of contralateral vs. 20.0%), and autoclaved VAC; 2.5 times greater (70.0% of contralateral vs. 20.0%) compared to the saline control group. Percent shear modulus values for 1.5 mg/kg VAC without sterilization was significantly greater; 4.8 times greater (23.0% of contralateral vs. 4.0%) compared to the saline control group. The data indicate that local VAC treatment enhanced bone regeneration during fracture healing and indicates that effective sterilization techniques that may affect the stability and bioactivity of proteins, do not significantly alter the bioactivity of VAC.

The effect of local vanadium therapy on healing of femur fractures in normal (non-diabetic) rats was measured by radiographic analysis. At twelve weeks post-fracture, fractured femurs from the rats treated with both low (1.5 mg/kg) and high (3.0 mg/kg) VAC had no evidence of ectopic bone formation, following resolution of the induced fracture (Figure 12). Femora treated with local VAC demonstrated normal remodeling suggesting no evidence of a toxic/carcinogenic effect of VAC throughout fracture healing. The above data have demonstrated an effective VAC therapeutic dosage range 0.5 to 3.0 mg/Kg, which resulted in two to three-fold increase in mechanical parameter of torsion.

*Comparison with Existing Therapies*

The chart in Figure 13 compares the vanadium technologies with the currently approved products (BMP-2 and Exogen) for fracture healing. Each of these studies examined the effectiveness of a therapeutic adjunct on femur fracture healing by measuring the maximum torque to failure at the same timepoint (four weeks). Specifically the following were compared: (1) a single percutaneous dose (0.25 mg/kg) of VAC with the calcium sulfate (CaSO$_4$) vehicle (red); (2) a single percutaneous dose (1.5 mg/kg) of VAC without a vehicle (blue); (3) a 316L stainless steel k-wire surface modified with vanadium (a process called vanadium pack-boriding), implanted into the intramedullary canal of the femur (green); (4) BMP-2 study used a single percutaneous dose of BMP-2 (80 μg) with buffer vehicle (orange); and (5) Exogen study used varying exposure periods of ultrasound treatment (20 mins /day). The most effective duration (25 days) is shown in dark blue.

Thus, the results have demonstrated, among others, (a) that the use of vanadium compounds (such as VAC) alone or as part of a formulation with an orthopedic carrier (CaSO4 for example) that is applied directly to the site of fracture; and (b) that the use of orthopedic implants (pedicle screws, plates, rods, wires, etc..) where the surface has been modified with vanadium via known thermal processing techniques. As an insulin-mimetic adjunct, vanadium compounds can be used to accelerate bone regeneration by stimulating insulin signaling at the fracture site. Local VAC targets the beta-subunit of the insulin signaling receptor. The presence of the insulin mimetic also enhances cartilage and mineralized tissue formation. Our laboratory data demonstrated that VAC treatment significantly increases cell proliferation within the subperiosteal region of the fracture callus (seven and ten days post-fracture). This translates into significantly higher percent cartilage within the fracture callus, (seven and ten days post-fracture). The percent mineralized tissue for local VAC treated rat animal models was significantly higher than controls after 21 days. This accelerated progression of the bone healing process results in significantly enhanced mechanical testing parameters for VAC treated animals after four and five weeks, compared to controls.

EXAMPLE 4

**Insulin Mimetics Enhancing Spinal Fusion**

Increased fusion rates were observed in a rat posterolateral lumbar spinal fusion model when treated with a time-released insulin implant in comparison with controls. The effects of insulin-mimetic agents were analyzed as an adjunct to spinal fusion in the rat posterolateral lumbar fusion model. Vanadyl acetylacetonate (VAC) or Zinc was made into a pellet with Calcium Sulfate, and applied to the fusion bed with autograft in a rat posterolataeral lumbar fusion. These results were compared with a control group treated with autograft and a palmitic acid pellet.

*Study Design*

The protocol was approved by the animal Institutional Care and Use Committee at UMDNJ-New Jersey Medical School. Fifty skeletally mature Sprague-Dawley rats weighing approximately 500 grams each underwent posterolateral intertransverse lumbar fusions with iliac crest autograft from L4-L5 utilizing a Wiltse-type approach. After exposure of the transverse processes and high-speed burr decortication, one of five pellets were added to the fusion site: a low dose Vanadium Calcium Sulfate pellet (0.75 mg/kg), a high dose Vanadium Calcium Sulfate pellet (1.5 mg/kg), a low dose Zinc Calcium Sulfate pellet (0.5 mg/kg), a high dose Zinc Calcium Sulfate pellet (1.0 mg/kg), and a control of micro-recrystalized palmitic acid pellet. An equal amount of iliac crest autograft (approximately 0.3g per side) was harvested and implanted with each pellet. The rats were sacrificed at eight weeks, and spines were harvested, removed of soft tissue, and tested by manual palpation, radiographs and MicroCT. All outcome parameters were independently reviewed by two separate individuals in a blinded manner and the lower grade of fusion was accepted when there was a discrepancy.

*Surgical Procedure*

After obtaining general anaesthesia with intraperitoneal Ketamine (40mg/kg) and Xylazine (5 mg/kg), the lumbar region of the rat was shaved and cleansed with povidone iodine soaked gauze. A dorsal midline incision was made from L3 to the sacrum. Two paramedian incisions were made through the lumbar fascia 5mm from the midline. Dissection was taken to the iliac crest, and approximately 0.3g of bone was harvested with small rongeurs. The harvested autograft was measured on a sterile scale in order to obtain 0.3g per side. Blunt dissection was carried down posterolaterally, reflecting the paraspinal muscles lateral to the facet joints on each side. The reflected paraspinal muscles were held in place with retractors. The transverse processes of L4-L5 were cleaned of soft tissue, and decorticated with a high-speed burr (Figure 14). The crushed autograft was then spread over and between the transverse processes at the appropriate level (L4-L5). An equivalent amount of implant, or blank was incorporated into the autograft bed (Figure 15). Retractors were removed and the paraspinal muscles were allowed to cover the fusion bed. The dorsal lumbar fascia was closed using a running 4-0 resorbable suture and the skin was closed with interrupted 4-0 resorbable sutures. The surgical site was treated with antibiotic ointment, and the rats were given a dose of Enrofloxacin antibiotic (10 mg/kg). Radiographs were taken immediately after surgery. Blood glucose levels were taken before surgery, and 12 and 24 hours after surgery. See Table 18.

**Table 18.** Systemic blood glucose levels (mg/dL)

| Group | Before surgery | 12 hours | 24 hours |
|---|---|---|---|
| Controls | | | 91.4 |
| VAC-low | 103.5 | 213.4 | 117.7 |
| VAC-high | 102.9 | 153.2 | 90.7 |
| Zn-low | 106.0 | 122.8 | 101.8 |
| Zn-high | 109.3 | 120.0 | 89.0 |

*Pellet Preparation*

In order to prepare the pellets, 0.2 mL of each stock solution will be mixed with 0.4 g of $CaSO_4$ to obtain the appropriate consistency of the carrier in a 1 mL syringe. It will then be injected into 2mm diameter clear Tygon laboratory tubing and allowed to harden overnight.

Once set, pellets will be sectioned into 7mm pieces and autoclaved (to sterilize), prior to implantation. Assumption: Weight of SD rat = 0.45 kg

| | Vn (0.75mg/kg) | Vn (1.5 mg/kg) | Zn (0.5 mg/kg) | Zn (1.0 mg/kg) |
|---|---|---|---|---|
| Mass of treatment for each rat | 0.338 mg | 0.675 mg | 0.225 mg | 0.45 mg |

In order to prepare the stock solution, the volume of solution in each pellet will be calculated by using the volume ratio of solution to mixture.

Volume of $CaSO_4$ in each mixture

- $$D\ CaSO_4 = 2.96\ g/cm^3$$

- $$(0.4g\_CaSO_4)/(2.96\frac{g}{cm^3}) = 0.135 cm^3 = 0.135 mL$$

Volume of mixture and ratio

- $$0.135\ mL\ CaSO_4 + 0.2\ mL\ solution = 0.335\ mL\ mixture$$

- $$0.2\ mL\ solution\ /\ 0.335\ mL\ mixture \times 100\% = 59.7\%\ solution\ per\ mixture$$

Volume of each pellet, 1 mm radius, 7 mm height

- $$V = \pi r^2 h\ , = \pi(1mm)^2(7mm) = 22mm^3 = 0.022 mL$$

Volume of solution in each pellet

- 

$$0.022\ mL \times 59.7\% = 0.0131\ mL\ solution\ per\ pellet$$

Stock Solution (10 mL)

• Because bilateral surgery is performed, mass of treatment (X) must be halved for each pellet.

•

$$\left(\left(\frac{X}{2}\right)/0.0131mL\right)\times10$$

| | Vn (0.75mg/kg) | Vn (1.5 mg/kg) | Zn (0.5 mg/kg) | Zn (1.0 mg/kg) |
|---|---|---|---|---|
| Mass of treatment in each stock solution (10 ml) | 129.0mg | 258.0 mg | 85.9 mg | 171.8 mg |

*Radiographic Analysis*

Posteroanterior radiographs at 35 kV for 90 seconds were taken at eight weeks after sacrifice and harvest. All soft tissue was removed prior to radiographic exam. Two blinded independent observers graded the radiographs as solid fusion mass bilaterally (A), unilateral fusion mass (B), small fusion mass bilaterally (C), and graft resorption (D), based on previously published radiographic scales. See Table 19.

**Table 19.** Radiographs

| Group | A | B | C | D | Kappa | P Value |
|---|---|---|---|---|---|---|
| **Controls (n=9)** | 2 | 3 | 1 | 3 | 0.297 | |
| **VAC-low (n=10)** | 3 | 3 | 0 | 4 | 0.583 | 0.807 |
| **VAC-high (n=10)** | 5 | 3 | 1 | 1 | 0.667 | 0.270 |
| **Zn-low (n=10)** | 7 | 1 | 2 | 0 | 0.512 | 0.066 |
| **Zn-high (n=10)** | 7 | 3 | 0 | 0 | 1.0 | 0.050 |
| A= solid fusion mass bilaterally<br>B= unilateral fusion mass<br>C= small fusion mass bilaterally<br>D= Graft resorption | | | | | | |

Based on radiographs (Figure 16), in the high dose vanadium group 5/10 had solid fusion mass bilaterally, 3/10 had unilateral fusion, 1/10 had small fusion mass bilaterally, and 1/10 had graft resorption. The low dose vanadium group had 3/10 solid fusion mass bilaterally, 3/10 had unilateral fusion, 0/10 had small fusion mass bilaterally, and 4/10 had graft resorption. The high dose zinc group had 7/10 solid fusion mass bilaterally, 3/10 had unilateral fusion, 0/10 had small fusion mass bilaterally, and 0/10 had graft resorption. The low dose zinc group had 7/10 solid fusion mass bilaterally, 1/10 had unilateral fusion, 2/10 had small fusion mass bilaterally, and 0/10 had graft resorption. The control group had 2/9 solid fusion mass bilaterally, 3/9 unilateral fusion, 1 small fusion mass bilaterally, and 3/9 had graft resorption.

*Manual Palpation*

After removal of all soft tissue, two blinded independent observers manually palpated and stressed across the fusion site (L4-L5). Specimens were graded as fused (A), partially fused (B), and not fused (C). See Table 20.

**Table 20.** Manual palpation

| Group | A | B | C | Kappa | P Value |
|---|---|---|---|---|---|
| **Controls (n=9)** | 0 | 1 | 8 | 0.156 | |
| **VAC-low (n=10)** | 1 | 4 | 5 | 0.130 | 0.072 |
| **VAC-high (n=10)** | 6 | 2 | 2 | 0.412 | 0.002 |
| **Zn-low (n=10)** | 3 | 4 | 3 | 0.565 | 0.055 |

(continued)

| Group | A | B | C | Kappa | P Value |
|---|---|---|---|---|---|
| Zn-high (n=10) | 4 | 1 | 5 | 0.306 | 0.008 |
| A= fused<br>B= partially fused<br>C= not fused | | | | | |

Based on manual palpation in the high dose Vanadium group 6/10 had solid fusion, 2/10 were partially fused, and 2/10 were not fused. In the low dose vanadium group, 1/10 had solid fusion, 4/10 were partially fused, and 5/10 were not fused. In the high dose Zinc group, 4/10 had solid fusion, 1/10 had partially fused, and 5/10 were not fused. In the low dose Zinc group, 3/10 had solid fusion, 4/10 had partially fused, and 3/10 were not fused. In the control group, 0/9 had solid fusion, 1/9 had partially fused, and 8/9 were not fused.

*Micro CT Analysis*

**Table 21.** MictroCT

| Group | Mean Bone Volume mm$^3$ | Std Dev | P value |
|---|---|---|---|
| Table 21a | | | ANOVA p=0.006 |
| Vn high dose (n=10) | 170.8 | 37.1 | <0.01 vs control |
| Vn low dose (n=10) | 167.4 | 23.5 | <0.05 vs control |
| Controls (n=9) | 126.7 | 26.3 | |
| | | | |
| Table 21b | | | ANOVA p=0.002 |
| Zn high dose (n=10) | 172.7 | 26.4 | <0.01 vs control |
| Zn low dose (n=10) | 172.9 | 31.6 | <0.01 vs control |
| Controls (n=9) | 126.7 | 26.3 | |

Based on MicroCT analysis, the mean bone volume of the L4/L5 transverse processes and fusion mass for controls was 126.7mm$^3$. In the high dose Vanadium group there was 170.8mm$^3$, and in the low dose Vanadium group there was 167.4mm$^3$. The high dose Zinc group had a mean of 172.7mm$^3$, and the low dose Zinc group had a mean of 172.9mm$^3$. Differences between each experimental group versus controls were significant (see Table 21).

*Statistical Analysis*

A Mann-Whitney Rank Test was performed for analysis of radiographs and manual palpation. Kappa values were calculated for inter-rater agreement. ANOVA was performed for amt of new bone formation as per micro CT with secondary test using Holm Sidak test. Statistical analysis was performed using SigmaStat.
Of the 50 animals, one of the control rats died on postoperative day one, likely due to anaesthesia. The remaining 49 rats had no complications and were sacrificed as planned (0.02% perioperative mortality rate).
The foregoing examples and description of the preferred embodiments should be taken as illustrating, rather than as limiting the present invention as defined by the claims. As will be readily appreciated, numerous variations and combinations of the features set forth above can be utilized without departing from the present invention as set forth in the claims.

**Claims**

1. A bone regeneration material for bone fusion or void filling consisting of an osteoconductive carrier and an insulin-mimetic zinc compound, wherein said osteoconductive carrier is selected from the group consisting of autograft materials, allograft materials, and xenograft materials, and wherein the insulin-mimetic zinc compound is an inorganic zinc compound selected from the group consisting of zinc chloride, zinc sulfate, zinc phosphate, and zinc carbonate, or an organic acid zinc salt selected from the group consisting of zinc acetate, zinc formate, zinc propionate, zinc gluconate, bis(maltolato)zinc, zinc acexamate, zinc aspartate, bis(maltolato)zinc(II) [Zn(ma)2], bis(2-hydroxypyridine-N-oxido)zinc(II) [Zn(hpo)2], bis(allixinato)Zn(II) [Zn(alx)2], bis(6-methylpicolinato)Zn(II) [Zn(6mpa)2], bis(aspirinato)zinc(II), bis(pyrrole-2-carboxylato)zinc [Zn(pc)2], bis(alpha-furonic acidato)zinc [Zn(fa)2], bis(thiophene-2-carboxylato)zinc [Zn(tc)2], bis(thiophene-2-acetato)zinc [Zn(ta)2], (N-acetyl-L-cysteinato)Zn(II) [Zn(nac)], zinc(II)/poly(y-glutamic acid) [Zn(y-pga)], bis(pyrrolidine-N-dithiocarbamate)zinc(II) [Zn(pdc)2], zinc(II) L-lactate [Zn(lac)2], zinc(II) D-(2)-quinic acid [Zn(qui)2], bis(1,6-dimethyl-3-hydroxy-5-methoxy-2-pentyl-1,4-dihydropyridine-4-thionato)zinc(II) [Zn(tanm)2], and β-alanyl-L-histidinato zinc(II) (AHZ).

2. An orthopedic or spinal implant, comprising at least one bone-contacting surface coated with a composition consisting of an insulin-mimetic zinc compound and an osteoconductive carrier selected from the group consisting of autograft materials, allograft materials, and xenograft materials, wherein the orthopedic implant is selected from the group consisting of screws, plates, rods, k-wires, pins, hooks, anchors, intramedullary devices, pedicle screws, pedicle hooks, spinal fusion cages, spinal fusion plates, prostheses, and said implant is made from a metal selected from the group consisting of titanium, alloys thereof, tantalum, alloys thereof, cobalt chrome alloys, steel alloys, and combinations thereof or from a polymeric material comprising a polymer selected from polyglycolic acid (PGA), poly(lactic-co-glycolic acid) (PLGA), polylactic acid (PLA), polycaprolactone (PCL), polyether ether ketone (PEEK), polyethylene terephthalate (PET), polypropylene (PP), polycarbonates (PC), poly(ortho esters) (POEs), and combinations thereof, and wherein the insulin-mimetic zinc compound is an inorganic zinc compound selected from the group consisting of zinc chloride, zinc sulfate, zinc phosphate, and zinc carbonate, or an organic acid zinc salt selected from the group consisting of zinc acetate, zinc formate, zinc propionate, zinc gluconate, bis(maltolato)zinc, zinc acexamate, zinc aspartate, bis(maltolato)zinc(II) [Zn(ma)2], bis(2-hydroxypyridine-N-oxido)zinc(II) [Zn(hpo)2], bis(allixinato)Zn(II) [Zn(alx)2], bis(6-methylpicolinato)Zn(II) [Zn(6mpa)2], bis(aspirinato)zinc(II), bis(pyrrole-2-carboxylato)zinc [Zn(pc)2], bis(alpha-furonic acidato)zinc [Zn(fa)2], bis(thiophene-2-carboxylato)zinc [Zn(tc)2], bis(thiophene-2-acetato)zinc [Zn(ta)2], (N-acetyl-L-cysteinato)Zn(II) [Zn(nac)], zinc(II)/poly(y-glutamic acid) [Zn(y-pga)], bis(pyrrolidine-N-dithiocarbamate)zinc(II) [Zn(pdc)2], zinc(II) L-lactate [Zn(lac)2], zinc(II) D-(2)-quinic acid [Zn(qui)2], bis(1,6-dimethyl-3-hydroxy-5-methoxy-2-pentyl-1,4-dihydropyridine-4-thionato)zinc(II) [Zn(tanm)2], and β-alanyl-L-histidinato zinc(II) (AHZ).

3. A bone tissue kit for use in a spinal fusion surgical procedure to facilitate fusion of vertebrae comprising a composition formulated for facile application in said spinal fusion procedure consisting of an insulin-mimetic zinc compound, an allograft bone tissue material and/or ceramic bone-graft substitute and a pharmaceutically acceptable carrier, and wherein the insulin-mimetic zinc compound is an inorganic zinc compound selected from the group consisting of zinc chloride, zinc sulfate, zinc phosphate, and zinc carbonate, or an organic acid zinc salt selected from the group consisting of zinc acetate, zinc formate, zinc propionate, zinc gluconate, bis(maltolato)zinc, zinc acexamate, zinc aspartate, bis(maltolato)zinc(II) [Zn(ma)2], bis(2-hydroxypyridine-N-oxido)zinc(II) [Zn(hpo)2], bis(allixinato)Zn(II) [Zn(alx)2], bis(6-methylpicolinato)Zn(II) [Zn(6mpa)2], bis(aspirinato)zinc(II), bis(pyrrole-2-carboxylato)zinc [Zn(pc)2], bis(alpha-furonic acidato)zinc [Zn(fa)2], bis(thiophene-2-carboxylato)zinc [Zn(tc)2], bis(thiophene-2-acetato)zinc [Zn(ta)2], (N-acetyl-L-cysteinato)Zn(II) [Zn(nac)], zinc(II)/poly(y-glutamic acid) [Zn(y-pga)], bis(pyrrolidine-N-dithiocarbamate)zinc(II) [Zn(pdc)2], zinc(II) L-lactate [Zn(lac)2], zinc(II) D-(2)-quinic acid [Zn(qui)2], bis(1,6-dimethyl-3-hydroxy-5-methoxy-2-pentyl-1,4-dihydropyridine-4-thionato)zinc(II) [Zn(tanm)2], and β-alanyl-L-histidinato zinc(II) (AHZ).

4. A composition for use in a spinal fusion surgical procedure consisting of an insulin-mimetic zinc compound and an osteoconductive carrier selected from the group consisting of autograft materials, allograft materials, and xenograft materials, wherein said composition enhances spinal fusion, and wherein the insulin-mimetic zinc compound is an inorganic zinc compound selected from the group consisting of zinc chloride, zinc sulfate, zinc phosphate, and zinc carbonate, or an organic acid zinc salt selected from the group consisting of zinc acetate, zinc formate, zinc propionate, zinc gluconate, bis(maltolato)zinc, zinc acexamate, zinc aspartate, bis(maltolato)zinc(II) [Zn(ma)2], bis(2-hydroxypyridine-N-oxido)zinc(II) [Zn(hpo)2], bis(allixinato)Zn(II) [Zn(alx)2], bis(6-methylpicolinato)Zn(II) [Zn(6mpa)2], bis(aspirinato)zinc(II), bis(pyrrole-2-carboxylato)zinc [Zn(pc)2], bis(alpha-furonic acidato)zinc [Zn(fa)2], bis(thiophene-2-carboxylato)zinc [Zn(tc)2], bis(thiophene-2-acetato)zinc [Zn(ta)2], (N-acetyl-L-cysteinato)Zn(II) [Zn(nac)], zinc(II)/poly(y-glutamic acid) [Zn(y-pga)], bis(pyrrolidine-N-dithiocarbamate)zinc(II) [Zn(pdc)2], zinc(II) L-lactate [Zn(lac)2], zinc(II) D-(2)-quinic acid [Zn(qui)2], bis(1,6-dimethyl-3-hydroxy-5-methoxy-2-pentyl-1,4-dihydropyridine-4-thionato)zinc(II)[Zn(tanm)2], and β-alanyl-L-histidinato zinc(II) (AHZ).

5. An implantable device for enhancing spinal fusion, comprising a prosthetic implant configured to stabilize and promote the fusion of two adjacent vertebrae, wherein the bone tissue contacting surfaces of the prosthetic implant are coated with a composition consisting of an insulin-mimetic zinc compound and a pharmaceutically acceptable carrier, and wherein the insulin-mimetic zinc compound is an inorganic zinc compound selected from the group consisting of zinc chloride, zinc sulfate, zinc phosphate, and zinc carbonate, or an organic acid zinc salt selected from the group consisting of zinc acetate, zinc formate, zinc propionate, zinc gluconate, bis(maltolato)zinc, zinc acexamate, zinc aspartate, bis(maltolato)zinc(II) [Zn(ma)2], bis(2-hydroxypyridine-N-oxido)zinc(II) [Zn(hpo)2], bis(allixinato)Zn(II) [Zn(alx)2], bis(6-methylpicolinato)Zn(II) [Zn(6mpa)2], bis(aspirinato)zinc(II), bis(pyrrole-2-carboxylato)zinc [Zn(pc)2], bis(alpha-furonic acidato)zinc [Zn(fa)2], bis(thiophene-2-carboxylato)zinc [Zn(tc)2], bis(thiophene-2-acetato)zinc [Zn(ta)2], (N-acetyl-L-cysteinato)Zn(II) [Zn(nac)], zinc(II)/poly(y-glutamic acid) [Zn(y-pga)], bis(pyrrolidine-N-dithiocarbamate)zinc(II) [Zn(pdc)2], zinc(II) L-lactate [Zn(lac)2], zinc(II) D-(2)-quinic acid [Zn(qui)2], bis(1,6-dimethyl-3-hydroxy-5-methoxy-2-pentyl-1,4-dihydropyridine-4-thionato)zinc(II) [Zn(tanm)2], and β-alanyl-L-histidinato zinc(II) (AHZ).

6. A bone regeneration material for bone fusion or void filling consisting of an insulin-mimetic zinc compound and an osteoconductive carrier, wherein the osteoconductive carrier is calcium sulfate, and wherein the insulin-mimetic zinc compound is an inorganic zinc compound selected from the group consisting of zinc chloride, zinc sulfate, zinc phosphate, and zinc carbonate, or an organic acid zinc salt selected from the group consisting of zinc acetate, zinc formate, zinc propionate, zinc gluconate, bis(maltolato)zinc, zinc acexamate, zinc aspartate, bis(maltolato)zinc(II) [Zn(ma)2], bis(2-hydroxypyridine-N-oxido)zinc(II) [Zn(hpo)2], bis(allixinato)Zn(II) [Zn(alx)2], bis(6-methylpicolinato)Zn(II) [Zn(6mpa)2], bis(aspirinato)zinc(II), bis(pyrrole-2-carboxylato)zinc [Zn(pc)2], bis(alpha-furonic acidato)zinc [Zn(fa)2], bis(thiophene-2-carboxylato)zinc [Zn(tc)2], bis(thiophene-2-acetato)zinc [Zn(ta)2], (N-acetyl-L-cysteinato)Zn(II) [Zn(nac)], zinc(II)/poly(y-glutamic acid) [Zn(y-pga)], bis(pyrrolidine-N-dithiocarbamate)zinc(II) [Zn(pdc)2], zinc(II) L-lactate [Zn(lac)2], zinc(II) D-(2)-quinic acid [Zn(qui)2], bis(1,6-dimethyl-3-hydroxy-5-methoxy-2-pentyl-1,4-dihydropyridine-4-thionato)zinc(II) [Zn(tanm)2], and β-alanyl-L-histidinato zinc(II) (AHZ).

**Patentansprüche**

1. Knochenregenerationsmaterial zur Knochenfusion oder Hohlraumfüllung, bestehend aus einem osteokonduktiven Träger und einer insulinmimetischen Zinkverbindung, wobei der osteokonduktive Träger ausgewählt ist aus der Gruppe bestehend aus Autotransplantatmaterialien, Allotransplantatmaterialien und Xenotransplantatmaterialien, und wobei die insulinmimetische Zinkverbindung eine anorganische Zinkverbindung ist, die aus der Gruppe ausgewählt ist, die aus Zinkchlorid, Zinksulfat, Zinkphosphat und Zinkcarbonat besteht, oder ein Zinksalz einer organischen Säure ist, das aus der Gruppe ausgewählt ist, die aus Zinkacetat, Zinkformiat, Zinkpropionat, Zinkgluconat, Bis(maltolato)zink, Zinkacexamat, Zinkaspartat, Bis(maltolato)zink(II) [Zn(ma)2], Bis(2-hydroxypyridin-N-oxido)Zink(II) [Zn(hpo)2], Bis(allixinato)Zn(II) [Zn(alx)2], Bis(6-methylpicolinato)Zn(II) [Zn(6mpa)2], Bis(aspirinato)Zink(II), Bis(pyrrol-2-carboxylato)Zink [Zn(pc)2], Bis(alpha-furonsäureato)Zink [Zn(fa)2], Bis(thiophen-2-carboxylato)Zink [Zn(tc)2], Bis(thiophen-2-acetato)Zink [Zn(ta)2], (N-Acetyl-L-cysteinato)Zn(II) [Zn(nac)], Zink(II)/Poly(y-glutaminsäure) [Zn(y-pga)], Bis(pyrrolidin-N-dithiocarbamat)Zink(II) [Zn(pdc)2], Zink(II) L-Lactat [Zn(lac)2], Zink(II) D-(2)-Chinasäure [Zn(qui)2], Bis(l,6-dimethyl-3-hydroxy-5-methoxy-2-pentyl-l,4-dihydropyridin-4-thionato)Zink(II) [Zn(tanm)2] und β-Alanyl-L-histidinato-Zink(II) (AHZ) besteht.

2. Orthopädisches oder Wirbelsäulenimplantat, das mindestens eine knochenberührende Oberfläche umfasst, die mit einer Zusammensetzung beschichtet ist, die aus einer insulinmimetischen Zinkverbindung und einem osteokonduktiven Träger besteht, der aus der Gruppe ausgewählt ist, die aus Autotransplantatmaterialien, Allotransplantatmaterialien und Xenotransplantatmaterialien besteht, wobei das orthopädische Implantat aus der Gruppe ausgewählt ist, die aus Schrauben, Platten, Stäben, K-Drähten, Stiften, Haken, Ankern, intramedullären Vorrichtungen, Pedikelschrauben, Pedikelhaken, Wirbelsäulenfusionskäfigen, Wirbelsäulenfusionsplatten und Prothesen besteht, und das Implantat aus einem Metall hergestellt ist, das aus der Gruppe ausgewählt ist, die aus Titan, Legierungen davon, Tantal, Legierungen davon, Kobalt-Chrom-Legierungen, Stahllegierungen und Kombinationen davon besteht, oder aus einem polymeren Material hergestellt ist, das ein Polymer umfasst, das ausgewählt ist aus Polyglykolsäure (PGA) Poly(milch-co-glykolsäure) (PLGA), Polymilchsäure (PLA), Polycaprolacton (PCL), Polyetheretherketon (PEEK), Polyethylenterephthalat (PET), Polypropylen (PP), Polycarbonate (PC), Poly(orthoester) (POEs) und Kombinationen davon, und wobei die insulinmimetische Zinkverbindung eine anorganische Zinkverbindung ist, die aus der Gruppe ausgewählt ist, die aus Zinkchlorid, Zinksulfat, Zinkphosphat und Zinkcarbonat besteht, oder ein Zinksalz einer organischen Säure ist, das aus der Gruppe ausgewählt ist, die aus Zinkacetat, Zinkformiat Zinkpropionat, Zinkgluconat, Bis(maltolato)zink, Zinkacexamat, Zinkaspartat, Bis(maltolato)zink(II) [Zn(ma)2], Bis(2-hydroxypyridin-N-oxido)Zink(II) [Zn(hpo)2], Bis(allixinato)Zn(II) [Zn(alx)2], Bis(6-methylpicolinato)Zink(II) [Zn(6mpa)2], Bis(aspirinato)Zink(II), Bis(pyrrol-2-carboxylato)Zink [Zn(pc)2], Bis(alpha-furonsäureato)Zink [Zn(fa)2], Bis(thiophen-2-carboxylato)Zink [Zn(tc)2], Bis(thiophen-2-acetato)Zink [Zn(ta)2], (N-Acetyl-L-cysteinato)Zn(II) [Zn(nac)], Zink(II)/Poly(y-glutaminsäure) [Zn(y-pga)], Bis(pyrrolidin-N-dithiocarbamat)Zink(II) [Zn(pdc)2], Zink(II)L-Lactat [Zn(lac)2], Zink(II)D-(2)-Chinasäure [Zn(qui)2], Bis(1,6-Dimethyl-3-hydroxy-5-methoxy-2-pentyl-1,4-dihydropyridin-4-thionato)Zink(II) [Zn(tanm)2] und β-Alanyl-L-histidinato-Zink(II) (AHZ) besteht.

3. Knochengewebe-Kit zur Verwendung in einem chirurgischen Verfahren zur Wirbelsäulenfusion zur Erleichterung der Fusion von Wirbeln, umfassend eine Zusammensetzung, die zur einfachen Anwendung in dem Wirbelsäulenfusionsverfahren formuliert ist, bestehend aus einer insulinmimetischen Zinkverbindung, einem Allotransplantat-Knochengewebematerial und/oder einem keramisches Knochentransplantatersatzmaterial und einem pharmazeutisch annehmbaren Träger, und wobei die insulinmimetische Zinkverbindung eine anorganische Zinkverbindung ist, die aus der Gruppe ausgewählt ist, die aus Zinkchlorid, Zinksulfat, Zinkphosphat und Zinkcarbonat besteht, oder ein Zinksalz einer organischen Säure ist, das aus der Gruppe ausgewählt ist, die aus Zinkacetat, Zinkformiat, Zinkpropionat, Zinkgluconat, Bis(maltolato)zink, Zinkacexamat, Zinkaspartat, Bis(maltolato)Zink(II) [Zn(ma)2], Bis(2-hydroxypyridin-N-oxido)Zink(II) [Zn(hpo)2], Bis(allixinato)Zn(II) [Zn(alx)2], Bis(6-methylpicolinato)Zn(II) [Zn(6mpa)2], Bis(aspirinato)Zink(II), Bis(pyrrol-2-carboxylato)Zink [Zn(pc)2], Bis(alpha-furonsäureato)Zink [Zn(fa)2], Bis(thiophen-2-carboxylato)Zink [Zn(tc)2], Bis(thiophen-2-acetato)Zink [Zn(ta)2], (N-Acetyl-L-cysteinato)Zn(II) [Zn(nac)], Zink(II)/Poly(y-glutaminsäure) [Zn(y-pga)], Bis(pyrrolidin-N-dithiocarbamat)Zink(II) [Zn(pdc)2], Zink(II) L-Lactat [Zn(lac)2], Zink(II) D-(2)-Chinasäure [Zn(qui)2], Bis(1,6-dimethyl-3-hydroxy-5-methoxy-2-pentyl-1,4-dihydropyridin-4-thionato)Zink(II) [Zn(tanm)2] und β-Alanyl-L-histidinato-Zink(11) (AHZ) besteht.

4. Zusammensetzung zur Verwendung in einem chirurgischen Verfahren zur Wirbelsäulenfusion, bestehend aus einer insulinmimetischen Zinkverbindung und einem osteokonduktiven Träger, ausgewählt aus der Gruppe bestehend aus Autotransplantatmaterialien, Allotransplantatmaterialien und Xenotransplantatmaterialien, wobei die Zusammensetzung die Wirbelsäulenfusion verbessert, und wobei die insulinmimetische Zinkverbindung eine anorganische Zinkverbindung ist, die aus der Gruppe ausgewählt ist, die aus Zinkchlorid, Zinksulfat, Zinkphosphat und Zinkcarbonat besteht, oder ein Zinksalz einer organischen Säure ist, das aus der Gruppe ausgewählt ist, die aus Zinkacetat, Zinkformiat, Zinkpropionat, Zinkgluconat, Bis(maltolato)zink, Zinkacexamat, Zinkaspartat, Bis(maltolato)zink(II) [Zn(ma)2], Bis(2-hydroxypyridin-N-oxido)Zink(II) [Zn(hpo)2], Bis(allixinato)Zn(II) [Zn(alx)2], Bis(6-methylpicolinato)Zn(II) [Zn(6mpa)2], Bis(aspirinato)Zink(II), Bis(pyrrol-2-carboxylato)Zink [Zn(pc)2], Bis(alpha-furonsäureato)Zink [Zn(fa)2], Bis(thiophen-2-carboxylato)Zink [Zn(tc)2], Bis(thiophen-2-acetato)Zink [Zn(ta)2], (N-Acetyl-L-cysteinato)Zn(II) [Zn(nac)], Zink(II)/Poly(y-glutaminsäure) [Zn(y-pga)], Bis(pyrrolidin-N-dithiocarbamat)Zink(II) [Zn(pdc)2], Zink(II) L-Lactat [Zn(lac)2], Zink(II) D-(2)-Chinasäure [Zn(qui)2], Bis(1,6-dimethyl-3-hydroxy-5-methoxy-2-pentyl-1,4-dihydropyridin-4-thionato)Zink(II) [Zn(tanm)2] und β-Alanyl-L-histidinato-Zink(11) (AHZ) besteht.

5. Implantierbare Vorrichtung zur Verbesserung der Wirbelsäulenfusion, die ein prothetisches Implantat umfasst, das so konfiguriert ist, dass es die Fusion zweier benachbarter Wirbel stabilisiert und fördert, wobei die das Knochengewebe berührenden Oberflächen des prothetischen Implantats mit einer Zusammensetzung beschichtet sind, die aus einer insulinmimetischen Zinkverbindung und einem pharmazeutisch akzeptablen Träger besteht, und wobei die insulinmimetische Zinkverbindung eine anorganische Zinkverbindung ist, die aus der Gruppe ausgewählt ist, die aus Zinkchlorid, Zinksulfat, Zinkphosphat und Zinkcarbonat besteht, oder ein Zinksalz einer organischen Säure ist, das aus der Gruppe ausgewählt ist, die aus Zinkacetat, Zinkformiat, Zinkpropionat, Zinkgluconat, Bis(maltolato)zink, Zinkacexamat, Zinkaspartat, Bis(maltolato)Zink(II) [Zn(ma)2], Bis(2-hydroxypyridin-N-oxido)Zink(II) [Zn(hpo)2], Bis(allixinato)Zn(II) [Zn(alx)2], Bis(6-methylpicolinato)Zn(II) [Zn(6mpa)2], Bis(aspirinato)Zink(II), Bis(pyrrol-2-carboxylato)Zink [Zn(pc)2], Bis(alpha-furonsäureato)Zink [Zn(fa)2], Bis(thiophen-2-carboxylato)Zink [Zn(tc)2], Bis(thiophen-2-acetato)Zink [Zn(ta)2], (N-Acetyl-L-cysteinato)Zn(II) [Zn(nac)], Zink(II)/Poly(y-glutaminsäure) [Zn(y-pga)], Bis(pyrrolidin-N-dithiocarbamat)Zink(II) [Zn(pdc)2], Zink(II) L-Lactat [Zn(lac)2], Zink(II) D-(2)-Chinasäure [Zn(qui)2], Bis(1,6-dimethyl-3-hydroxy-5-methoxy-2-pentyl-1,4-dlhydropyridin-4-thionato)Zink(II) [Zn(tanm)2] und β-Alanyl-L-histidinato-Zink(11) (AHZ) besteht.

6. Knochenregenerationsmaterial zur Knochenfusion oder Hohlraumfüllung, bestehend aus einer insulinmimetische Zinkverbindung und einem osteokonduktiven Träger, wobei der osteokonduktive Träger Calciumsulfat ist und die insulinmimetische Zinkverbindung eine anorganische Zinkverbindung ist, die aus der Gruppe ausgewählt ist, die aus Zinkchlorid, Zinksulfat, Zinkphosphat und Zinkcarbonat besteht, oder ein Zinksalz einer organischen Säure ist, das aus der Gruppe ausgewählt ist, die aus Zinkacetat, Zinkformiat, Zinkpropionat, Zinkgluconat, Bis(maltolato)Zink, Zinkacexamat, Zinkaspartat, Bis(maltolato)Zink(II) [Zn(ma)2], Bis(2-hydroxypyridin-N-oxido)Zink(II) [Zn(hpo)2], Bis(allixinato)Zn(II) [Zn(alx)2], Bis(6-methylpicolinato)Zn(II) [Zn(6mpa)2], Bis(aspirinato)Zink(II), Bis(pyrrol-2-carboxylato)Zink [Zn(pc)2], Bis(alpha-furonsäureato)zink [Zn(fa)2], Bis(thiophen-2-carboxylato)zink [Zn(tc)2], Bis(thiophen-2-acetato)zink [Zn(ta)2], (N-Acetyl-L-cysteinato)zink(II) [Zn(nac)], Zink(II)/Poly(y-glutaminsäure) [Zn(y-pga)], Bis(pyrrolidin-N-dithiocarbamat)Zink(II) [Zn(pdc)2], Zink(II) L-Lactat [Zn(lac)2], Zink(II) D-(2)-Chinasäure [Zn(qui)2], Bis(1,6-dimethyl-3-hydroxy-5-methoxy-2-pentyl-1,4-dihydropyridin-4-thionato)zink(II) [Zn(tanm)2], und β-Alanyl-L-histidinato-Zink(11) (AHZ) besteht.

**Revendications**

1. Matériau de régénération osseuse pour la fusion osseuse ou le remplissage de vides, consistant en un support ostéoconducteur et un composé de zinc mimétique de l'insuline, dans lequel le support ostéoconducteur est choisi dans le groupe constitué par des matériaux d'autogreffe, des matériaux d'allogreffe et des matériaux de xénogreffe, et dans lequel le composé de zinc mimétique de l'insuline est un composé de zinc inorganique choisi dans le groupe constitué par le chlorure de zinc, le sulfate de zinc, le phosphate de zinc et le carbonate de zinc, ou un sel de zinc d'un acide organique choisi dans le groupe constitué par l'acétate de zinc, le formiate de zinc, le propionate de zinc, le gluconate de zinc, le bis(maltolato)zinc, l'acéxamate de zinc, l'aspartate de zinc, bis(maltolato)zinc(II) [Zn(ma)2], bis(2-hydroxypyridine-N-oxido)zinc(II) [Zn(hpo)2], bis(allixinato)Zn(II) [Zn(alx)2], bis(6-méthylpicolinato)Zn(II) [Zn(6mpa)2], Bis(aspirinato)Zinc(II), Bis(pyrrole-2-carboxylato)Zinc [Zn(pc)2], Bis(alpha-furonic acidato)Zinc [Zn(fa)2], Bis(thiophene-2-carboxylato)Zinc [Zn(tc)2], Bis(thiophene-2-acetato)Zinc [Zn(ta)2], (N-acétyl-L-cystéinato)Zn(II) [Zn(nac)], Zinc(II)/poly(acide y-glutamique) [Zn(y-pga)], Bis(pyrrolidine-N-dithiocarbamate)Zinc(II) [Zn(pdc)2], Zinc(II) L-lactate [Zn(lac)2], Acide zinc(II) D-(2)-quinique [Zn(qui)2], bis(1,6-diméthyl-3-hydroxy-5-méthoxy-2-pentyl-1,4-dihydropyridine-4-thionato)Zinc(II) [Zn(tanm)2] et β-alanyl-L-histidinato zinc(II) (AHZ).

2. Implant orthopédique ou spinal comprenant au moins une surface de contact avec l'os revêtue d'une composition constituée d'un composé de zinc mimétique de l'insuline et d'un support ostéoconducteur choisi dans le groupe constitué par les matériaux d'autogreffe, les matériaux d'allogreffe et les matériaux de xénogreffe, dans lequel l'implant orthopédique est choisi dans le groupe constitué par les vis, les plaques, les tiges, les fils en K, les broches, les crochets, les ancres, les dispositifs intramédullaires, les vis pédiculaires, crochets pédiculaires, cages de fusion vertébrale, plaques de fusion vertébrale, et prothèses, et l'implant est fait d'un métal choisi dans le groupe constitué par le titane, ses alliages, le tantale, ses alliages, les alliages cobalt-chrome, les alliages d'acier et leurs combinaisons, ou d'un matériau polymère comprenant un polymère choisi parmi l'acide polyglycolique (PGA), le poly(acide lactique-co-glycolique) (PLGA), l'acide polylactique (PLA), la polycaprolactone (PCL), le polyétheréthercétone (PEEK), le polyéthylène téréphtalate (PET), le polypropylène (PP), polycarbonates (PC), poly(ortho esters) (POE) et leurs combinaisons, et dans lequel le composé de zinc mimétique de l'insuline est un composé de zinc inorganique choisi dans le groupe constitué par le chlorure de zinc, le sulfate de zinc, le phosphate de zinc et le carbonate de zinc, ou un sel de zinc d'un acide organique, choisi dans le groupe constitué par l'acétate de zinc, le formiate de zinc, le propionate de zinc, le gluconate de zinc, le bis(maltolato)zinc, l'acéxamate de zinc, l'aspartate de zinc, le bis(maltolato)zinc(II) [Zn(ma)2], le bis(2-hydroxypyridine-N-oxido)zinc(II) [Zn(hpo)2], le bis(allixinato)Zn(II) [Zn(alx)2], Bis(6-méthylpicolinato)Zn(II) [Zn(6mpa)2], Bis(aspirinato)Zinc(II), Bis(pyrrole-2-carboxylato)Zinc [Zn(pc)2], Bis(alpha-furonic acidato)Zinc [Zn(fa)2], Bis(thiophène-2-carboxylato)zinc [Zn(tc)2], Bis(thiophène-2-acétato)zinc [Zn(ta)2], (N-acétyl-L-cysteinato)Zn(II) [Zn(nac)], Zinc(II)/poly(acide y-glutamique) [Zn(y-pga)], Bis(pyrrolidine-N-dithiocarbamate)Zinc(II) [Zn(pdc)2], Zinc(II)L-lactate [Zn(lac)2], Zinc(II)D-(2)-acide quinique [Zn(qui)2], Bis(1,6-diméthyl-3-hydroxy-5-méthoxy-2-pentyl-1,4-dihydropyridine-4-thionato)zinc(II) [Zn(tanm)2] et β-alanyl-L-histidinato zinc(II) (AHZ).

3. Kit de tissu osseux destiné à être utilisé dans une procédure de fusion spinale chirurgicale pour faciliter la fusion des vertèbres, comprenant une composition formulée pour faciliter l'utilisation dans la procédure de fusion spinale, consistant en un composé de zinc mimétique de l'insuline, un matériau de tissu osseux d'allogreffe et/ou un substitut de greffe osseuse en céramique et un support pharmaceutiquement acceptable, et dans lequel le composé de zinc mimétique de l'insuline est un composé de zinc inorganique choisi dans le groupe constitué par le chlorure de zinc, le sulfate de zinc, le phosphate de zinc et le carbonate de zinc, ou un sel de zinc d'un acide organique choisi dans le groupe constitué par l'acétate de zinc, le formiate de zinc, le propionate de zinc, gluconate de zinc, bis(maltolato)zinc, acéxamate de zinc, aspartate de zinc, bis(maltolato)zinc(II) [Zn(ma)2], bis(2-hydroxypyridine-N-oxido)zinc(II) [Zn(hpo)2], bis(allixinato)Zn(II) [Zn(alx)2], Bis(6-méthylpicolinato)Zn(II) [Zn(6mpa)2], Bis(aspirinato)Zinc(II), Bis(pyrrole-2-carboxylato)Zinc [Zn(pc)2], Bis(alpha-furonic acidato)Zinc [Zn(fa)2], Bis(thiophene-2-carboxylato)Zinc [Zn(tc)2], Bis(thiophène-2-acétato)zinc [Zn(ta)2], (N-acétyl-L-cystéinato)Zn(II) [Zn(nac)], Zinc(II)/poly(acide y-glutamique) [Zn(y-pga)], Bis(pyrrolidine-N-dithiocarbamate)Zinc(II) [Zn(pdc)2], L-lactate de zinc(II) [Zn(lac)2], Acide zinc(II) D-(2)-quinique [Zn(qui)2], bis(1,6-diméthyl-3-hydroxy-5-méthoxy-2-pentyl-1,4-dihydropyridine-4-thionato)Zinc(II) [Zn(tanm)2] et β-alanyl-L-histidinato zinc(II) (AHZ).

**4.** Composition destinée à être utilisée dans une procédure chirurgicale de fusion spinale consistant en un composé de zinc mimétique de l'insuline et un support ostéoconducteur choisi dans le groupe constitué par les matériaux d'autogreffe, les matériaux d'allogreffe et les matériaux de xénogreffe, dans laquelle la composition améliore la fusion spinale, et dans lequel le composé de zinc mimétique de l'insuline est un composé de zinc inorganique choisi dans le groupe constitué par le chlorure de zinc, le sulfate de zinc, le phosphate de zinc et le carbonate de zinc, ou un sel de zinc acide organique choisi dans le groupe constitué par l'acétate de zinc, le formiate de zinc, le propionate de zinc, le gluconate de zinc, le bis(maltolato)zinc, acéxamate de zinc, aspartate de zinc, bis(maltolato)zinc(II) [Zn(ma)2], bis(2-hydroxypyridine-N-oxido)zinc(II) [Zn(hpo)2], bis(allixinato)Zn(II) [Zn(alx)2], bis(6-méthylpicolinato)Zn(II) [Zn(6mpa)2], Bis(aspirinato)Zinc(II), Bis(pyrrole-2-carboxylato)Zinc [Zn(pc)2], Bis(alpha-furonic acidato)Zinc [Zn(fa)2], Bis(thiophene-2-carboxylato)Zinc [Zn(tc)2], Bis(thiophene-2-acetato)Zinc [Zn(ta)2], (N-acétyl-L-cystéinato)Zn(II) [Zn(nac)], Zinc(II)/poly(acide y-glutamique) [Zn(y-pga)], Bis(pyrrolidine-N-dithiocarbamate)Zinc(II) [Zn(pdc)2], Zinc(II) L-lactate [Zn(lac)2], Acide zinc(II) D-(2)-quinique [Zn(qui)2], bis(1,6-diméthyl-3-hydroxy-5-méthoxy-2-pentyl-1,4-dihydropyridine-4-thionato)Zinc(II) [Zn(tanm)2] et β-alanyl-L-histidinato zinc(II) (AHZ).

**5.** Dispositif implantable pour améliorer la fusion spinale comprenant un implant prothétique configuré pour stabiliser et favoriser la fusion de deux vertèbres adjacentes, dans lequel les surfaces de contact avec le tissu osseux de l'implant prothétique sont revêtues d'une composition, constitué d'un composé de zinc mimétique de l'insuline et d'un support pharmaceutiquement acceptable, et dans lequel le composé de zinc mimétique de l'insuline est un composé de zinc inorganique choisi dans le groupe constitué par le chlorure de zinc, le sulfate de zinc, le phosphate de zinc et le carbonate de zinc, ou un sel de zinc d'un acide organique choisi dans le groupe constitué par l'acétate de zinc, le formiate de zinc, le propionate de zinc, gluconate de zinc, bis(maltolato)zinc, acéxamate de zinc, aspartate de zinc, bis(maltolato)zinc(II) [Zn(ma)2], bis(2-hydroxypyridine-N-oxido)zinc(II) [Zn(hpo)2], bis(allixinato)Zn(II) [Zn(alx)2], Bis(6-méthylpicolinato)Zn(II) [Zn(6mpa)2], Bis(aspirinato)Zinc(II), Bis(pyrrole-2-carboxylato)Zinc [Zn(pc)2], Bis(alpha-furonic acidato)Zinc [Zn(fa)2], Bis(thiophene-2-carboxylato)Zinc [Zn(tc)2], Bis(thiophène-2-acétato)zinc [Zn(ta)2], (N-acétyl-L-cystéinato)Zn(II) [Zn(nac)], Zinc(II)/poly(acide y-glutamique) [Zn(y-pga)], Bis(pyrrolidine-N-dithiocarbamate)Zinc(II) [Zn(pdc)2], L-lactate de zinc(II) [Zn(lac)2], Acide zinc(II) D-(2)-quinique [Zn(qui)2], bis(1,6-diméthyl-3-hydroxy-5-méthoxy-2-pentyl-1,4-dihydropyridine-4-thionato)Zinc(II) [Zn(tanm)2] et β-alanyl-L-histidinato zinc(II) (AHZ).

**6.** Matériau de régénération osseuse pour la fusion osseuse ou le remplissage de vides, consistant en un composé de zinc mimétique de l'insuline et un support ostéoconducteur, dans lequel le support ostéoconducteur est le sulfate de calcium et le composé de zinc mimétique de l'insuline est un composé de zinc inorganique choisi dans le groupe constitué par le chlorure de zinc, le sulfate de zinc, le phosphate de zinc et le carbonate de zinc, ou un sel de zinc d'un acide organique choisi dans le groupe constitué par l'acétate de zinc, le formate de zinc, le propionate de zinc, le gluconate de zinc, le bis(III) et le carbonate de zinc, le sulfate de zinc, le phosphate de zinc et le carbonate de zinc, ou un sel de zinc d'un acide organique choisi dans le groupe constitué par l'acétate de zinc, le formate de zinc, le propionate de zinc, le gluconate de zinc, bis(maltolato)zinc, acéxamate de zinc, aspartate de zinc, bis(maltolato)zinc(II) [Zn(ma)2], bis(2-hydroxypyridine-N-oxido)zinc(II) [Zn(hpo)2], bis(allixinato)Zn(II) [Zn(alx)2], Bis(6-méthylpicolinato)Zn(II) [Zn(6mpa)2], Bis(aspirinato)Zinc(II), Bis(pyrrole-2-carboxylato)Zinc [Zn(pc)2], Bis(alpha-furonic acidato)zinc [Zn(fa)2], Bis(thiophene-2-carboxylato)zinc [Zn(tc)2], Bis(thiophène-2-acétato)zinc [Zn(ta)2], (N-acétyl-L-cystéinato)zinc(II) [Zn(nac)], Zinc(II)/poly(acide y-glutamique) [Zn(y-pga)], Bis(pyrrolidine-N-dithiocarbamate)zinc(II) [Zn(pdc)2], L-lactate de zinc(II) [Zn(lac)2], Acide zinc(II) D-(2)-quinique [Zn(qui)2], bis(1,6-diméthyl-3-hydroxy-5-méthoxy-2-pentyl-1,4-dihydropyridine-4-thionato)zinc(II) [Zn(tanm)2], et β-alanyl-L-histidinato zinc(II) (AHZ).

**Figure 1**

**Figures 2 A-C**

Saline Control                    1 mg/kg ZnCl$_2$                    3 mg/kg ZnCl$_2$

AP          Lateral          AP          Lateral          AP          Lateral

**Figure 3**

| | 7 Day Trichrome | 7 Day Safranin-O | 21 Day PMMA |
|---|---|---|---|
| Saline (Control) | | | |
| 3.0 mg/kg ZnCl | | | |

**Figure 4**

CaSO₄ Carrier  |  1 mg/kg ZnCl₂ +CaSO4 Carrier

AP    Lateral         AP    Lateral

**Figure 5**

Figure 6

Anteroposterior view of 4 week
Local MnCl$_2$ Treated Fractures

Anteroposterior view of 4 week
Saline Control Fractures

Lateral view of 4 week
Local MnCl$_2$ Treated Fractures

Lateral view of 4 week
Saline Control Fractures

**Figure 7**

**Femur Vanadium Concentrations**

Figure 8

Figure 9

**Figure 10**

**Figure 11**

Saline Control Animal      1.5 mg/kg VAC      3.0 mg/kg VAC

AP    Lateral     AP    Lateral     AP    Lateral

**Figure 12**

## Comparison of Current Therapies (25-28 Days)

**Figure 13**

**Figure 14**

**Figure 15**

**Figure 16**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LIEBERMAN, J.R. et al.** *J. Bone Joint Surg. Am.,* 2002, vol. 84, 1032-1044
- **TRIPPEL, S.B. et al.** *J. Bone Joint Surg. Am.,* 1996, vol. 78, 1272-86
- **PETROVA, R. ; SUWATTANANONT, N.** *J. Electr. Mat.,* 2005, vol. 34 (5), 8
- **FISCHER, R.C.** *Met. Progr.,* 1986
- **HABIG, K.H.** *Tribol. Int.,* 1989, vol. 22, 65
- **Z. ZAKHARIEV, Z. et al.** *Surf. Coating Technol.,* 1987, vol. 31, 265
- **EKELAND, A. et al.** *Acta Orthop. Scand.,* 1981, vol. 52 (6), 605-13
- **ENGESAETER, L.B. et al.** *Acta Orthop. Scand.,* 1978, vol. 49 (6), 512-8
- **BEAM, H.A. et al.** *J. Orthop. Res.,* 2002, vol. 20 (6), 1210-1216
- **GANDHI, A. et al.** *Bone,* 2006, vol. 38 (4), 540-546
- **BERGENSTOCK, M.W. et al.** *J. Orthop. Trauma,* 2005, vol. 19 (10), 717-723
- **EKELAND, A. et al.** *Acta Orthop. Scand.,* 1981, vol. 52 (6), 605-613
- **ENGESAETER, L.B. et al.** *Acta Orthop. Scand.,* 1978, vol. 49 (6), 512-518
- **LEVENSTON, M.E. et al.** *J. Bone Miner. Res.,* 1994, vol. 9 (9), 1459-1465
- **EINHORN, T.A. et al.** *J. Bone Joint Surg. Am.,* 2003, vol. 85 (8), 1425-1435
- **AZUMA, Y. et al.** *J. Bone Miner. Res.,* 2001, vol. 16 (4), 671-680
- **MARTIN, A.M. et al.** *Diabetes,* 1999, vol. 48 (11), 2138-44